# EUROPÄISCHE PATENTANMELDUNG

(11) **EP 0 493 321 A1**
(43) Veröffentlichungstag der Anmeldung: **01.07.1992**
(21) Anmeldenummer: 91810970.3
(22) Anmeldetag: 12.12.1991
(51) Int. Cl.: C07D 239/60, C07D 401/12, C07D 239/52, C07D 239/56, C07F 9/28, C07D 251/30, A01N 43/54

(54) **Pyrimidinyl- und Triazinyl-salicylamide sowie deren Verwendung und Herstellung**

(30) Priorität: 21.12.1990 GB 9027864
(71) Anmelder: CIBA-GEIGY AG, CH-4002 Basel (CH)
(72) Erfinder: Lüthy, Christoph, Dr., CH-8603 Schwerzenbach (CH); Fisher, Raymond, Dr., Hadfield, Hyde, Cheshire SK14 6DW (GB)

(57) **Zusammenfassung**

Pyrimidinyl- und Triazinyl-salicylamide der Formel I 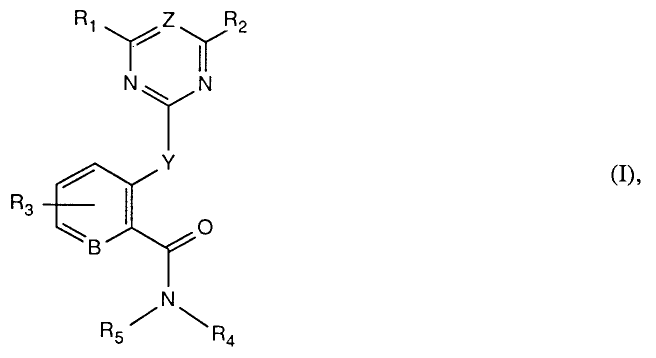
wobei die Bedeutungen der Reste in Anspruch 1 umschrieben sind, sowie die Salze von Verbindungen der Formel I, welche eine freie Hydroxycarbonylgruppe aufweisen, besitzen herbizide und pflanzenwachstumsregulierende Wirkung . Sie eignen sich als Wirkstoffe in Unkrautbekämpfungsmitteln und in Mitteln zur positiven Beeinflussung des Wachstums von Nutzpflanzen.

## Beschreibung

Die vorliegende Erfindung betrifft neue, herbizid wirksame und pflanzenwuchsregulierende Pyrimidinyl- und Triazinyl- salicylamide, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums.

Es wurde gefunden, daß Verbindungen der Formel I herbizide und pflanzenwachstumsregulierende Wirkung aufweisen. Sie eignen sich daher als Wirkstoffe in Unkrautbekämpfungsmitteln und in Mitteln zur positiven Beeinflussung des Wachstums von Nutzpflanzen.

Die erfindungsgemäßen Pyrimidinyl- und Triazinyl-salicylamide entsprechen der Formel I 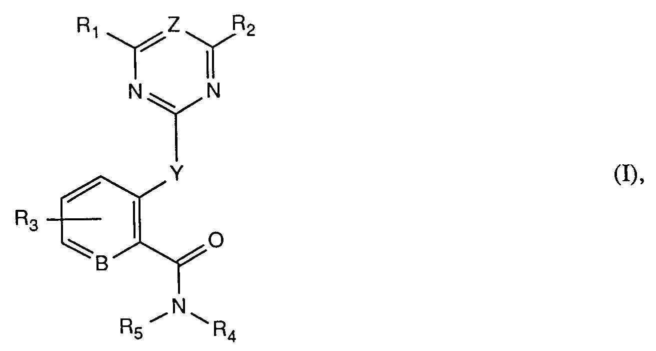
worin
B für Stickstoff, Methin, oder mit Fluor, Chlor, Methyl, Methoxy, 2-Propenyloxy,
2-Propinyloxy, Difluormethoxy, Phenoxy, Methylthio, Phenylthio, Phenyl oder Benzyloxy substituiertes Methin;
Y für Sauerstoff oder Schwefel;
Z für Methin oder Stickstoff;
R₁ für Chlor, Methyl, Methoxy, Ethoxy, Difluormethoxy, Methylamino, Ethylamino oder Dimethylamino;
R₂ für Methyl, Methoxy oder Difluormethoxy;
R₃ für Wasserstoff, Fluor, Chlor, Methyl oder Methoxy;
R₄ für eine der Gruppen (a), (b) oder (c) 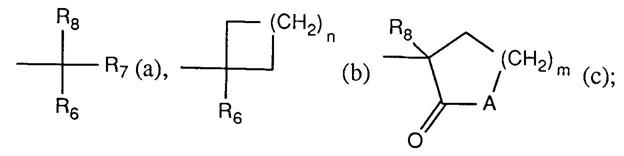
R₅ für Wasserstoff, Methyl oder zusammen mit R₇ -(CH₂)ₚ-, -CH₂SCH₂- oder -CH₂CHOHCH₂-;
R₆ für Hydroxymethyl, Formyl, Cyano, Hydroxyimino, C₁₋₄-Alkoxyimino, Phosphono, Phosphino, Methylphosphino oder eine Gruppe COX;
R₇ für Wasserstoff; C₁₋₄-Alkyl oder durch Hydroxy, C₁₋₄-Alkoxy, Mercapto, Acylmercapto, C₁₋₄-Alkylthio, Vinyl, Phenyl, 4-Hydroxyphenyl, 4-Imidazolyl, 3-Indolyl, Hydroxycarbonyl, C₁₋₄-Alkoxycarbonyl, 2-Propenyloxycarbonyl, Cyano, Carbamoyl, Methylphosphino oder Methylsulfoximino substituiertes C₁₋₄-Alkyl; Trifluormethyl; Ethinyl; Vinyl oder durch Chlor oder Methoxy substituiertes Vinyl; Phenyl oder durch Fluor, Chlor, Methyl, Trifluormethyl oder Methoxy substituiertes Phenyl; oder Cyano oder C₁₋₄-Alkoxycarbonyl;
R₈ für Wasserstoff oder Methyl;
A für Sauerstoff, Schwefel oder -NH-;
m für 1, 2 oder 3;
n für 0, 1, 2 oder 3;
p für 2 oder 3;
X für Hydroxy, C₁₋₄-Alkoxy, C₃₋₄-Alkenyloxy, Mercapto, C₁₋₄-Alkylthio, Amino, C₁₋₄-Alkylamino, C₂₋₄Dialkylamino, C₁₋₄-Alkoxyamino, oder durch Phenyl, Benzyloxy oder C₁₋₂-Alkoxy substituiertes C₁₋₄-Alkoxy, C₃₋₄-Alkenyloxy, Mercapto, C₁₋₄-Alkylthio, Amino, C₁₋₄-Alkylamino, C₂₋₄Dialkylamino, C₁₋₄-Alkoxyamino, oder eine der Gruppen (d), (e) oder f) 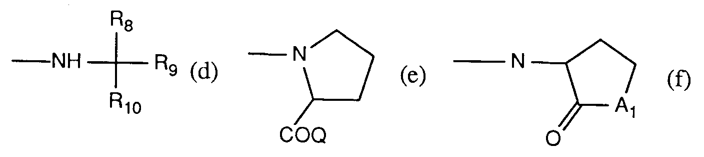
A₁ für Sauerstoff, Schwefel oder -NH-;
R₉ für Wasserstoff, C₁₋₄-Alkyl oder Benzyl;
R₁₀ für Hydroxymethyl, Cyano oder eine Gruppe COQ′;
Q für Hydroxy, C₁₋₄-Alkoxy, 2-Propenyloxy, Benzyloxy, Amino oder die Gruppe d); und
Q′ für Hydroxy, C₁₋₄-Alkoxy, 2-Propenyloxy, Benzyloxy, Amino oder die Gruppe d) stehen; sowie die Salze derjenigen Verbindungen der Formel I, welche eine freie Hydroxycarbonylgruppe aufweisen.

In der Formel I können die Alkyl- und Alkenylreste geradkettig oder verzweigt sein. Dasselbe gilt auch für den bzw. jeden Alkylteil von Alkoxy-, Alkylthio-, Alkoxycarbonyl-und von weiteren Alkyl-enthaltenden Gruppen.

Ist in den Verbindungen der Formel I ein asymmetrisches Kohlenstoffatom vorhanden, so hat dies zur Folge, daß die Verbindungen in optisch isomeren Formen auftreten können. Liegt eine aliphatische C=C-Doppelbindung vor, so kann auch geometrische Isomerie auftreten. Die Formel I umfaßt daher auch alle möglichen Stereoisomeren, die in Form von Enantiomeren, Diastereomeren oder deren Gemische vorliegen. Einige Verbindungen der Formel I können in tautomeren Formen (z.B. Keto-Enol- oder Imin-Enamin-Tautomerie) vorkommen. Die Formel I umfaßt daher auch alle Tautomeren.

Unter den Verbindungen der Formel I sind diejenigen bevorzugt, in denen
a) B Methin oder Stickstoff bedeutet, wobei das Methin vorzugsweise durch Fluor oder Chlor substituiert ist;
b) Y Sauerstoff bedeutet;
c) Z Methin bedeutet;
d) R₁ und R₂ Methoxy bedeuten;
e) R₃ Wasserstoff bedeutet;
f) R₄ eine Gruppe (a) bedeutet;
g) R₅ Wasserstoff bedeutet;
h) R₆ Cyano oder eine COX-Gruppe bedeutet;
i) R₇ Wasserstoff, C₁₋₄-Alkyl, 2-Propenyl oder zusammen mit R₅ -CH₂CH₂CH₂- oder -CH₂SCH₂- bedeutet;
j) R₈ Wasserstoff bedeutet; und
k) R₄ eine chirale Gruppe der Formel IA

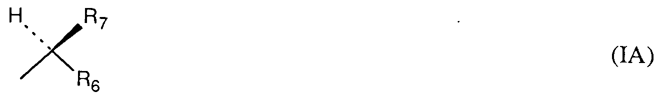

mit der Chiralität <S > bedeutet.

Von den Verbindungen der Untergruppe h) sind diejenigen bevorzugt, bei denen X C₁₋₄-Alkoxy oder Amino bedeutet.

Eine weitere bevorzugte Untergruppe bilden diejenigen Verbindungen der Formel I, in denen R₄ eine Gruppe (c), A Sauerstoff oder Schwefel und m = 1 bedeuten, wobei Verbindungen, in denen R₄ eine Gruppe der Formel IF 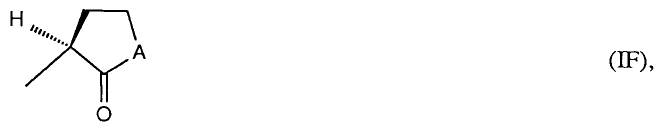
mit der Chiralität <S>, ganz besonders bevorzugt sind.

Eine durch ihre gute biologische Wirksamkeit besonders herausragende Gruppe von Verbindungen der Formel I ist jene, bei welcher:
R¹ für Methoxy, Ethoxy, oder Difluormethoxy;
R² für Methoxy;
R³ für Wasserstoff;
B für Stickstoff, Methin, oder durch Chlor oder Fluror substituiertes Methin;
Y für Sauerstoff oder Schwefel;
Z für Methin; und in der
NR⁴R⁵ eine der in der Tabelle 1 angegebenen Bedeutungen hat: 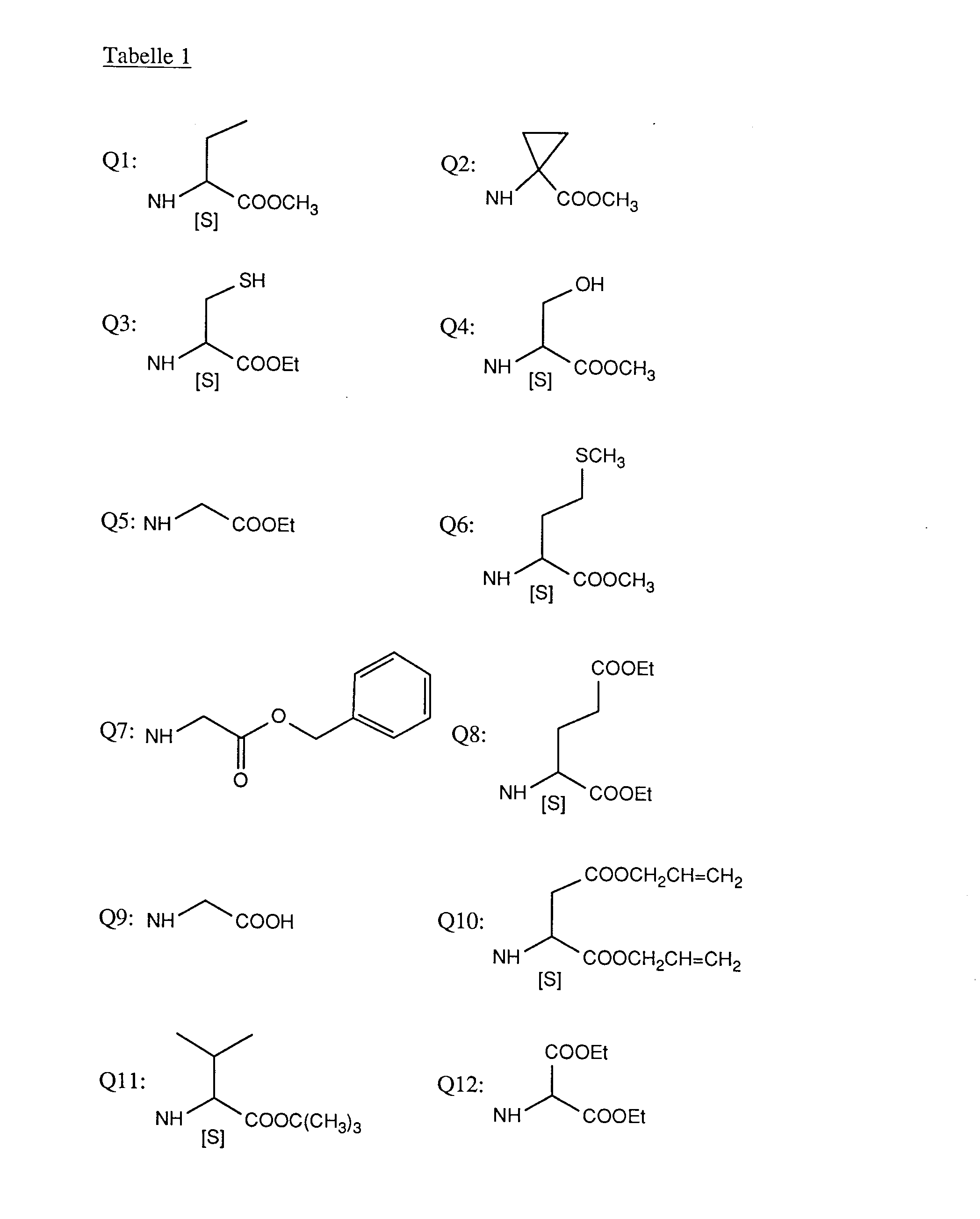 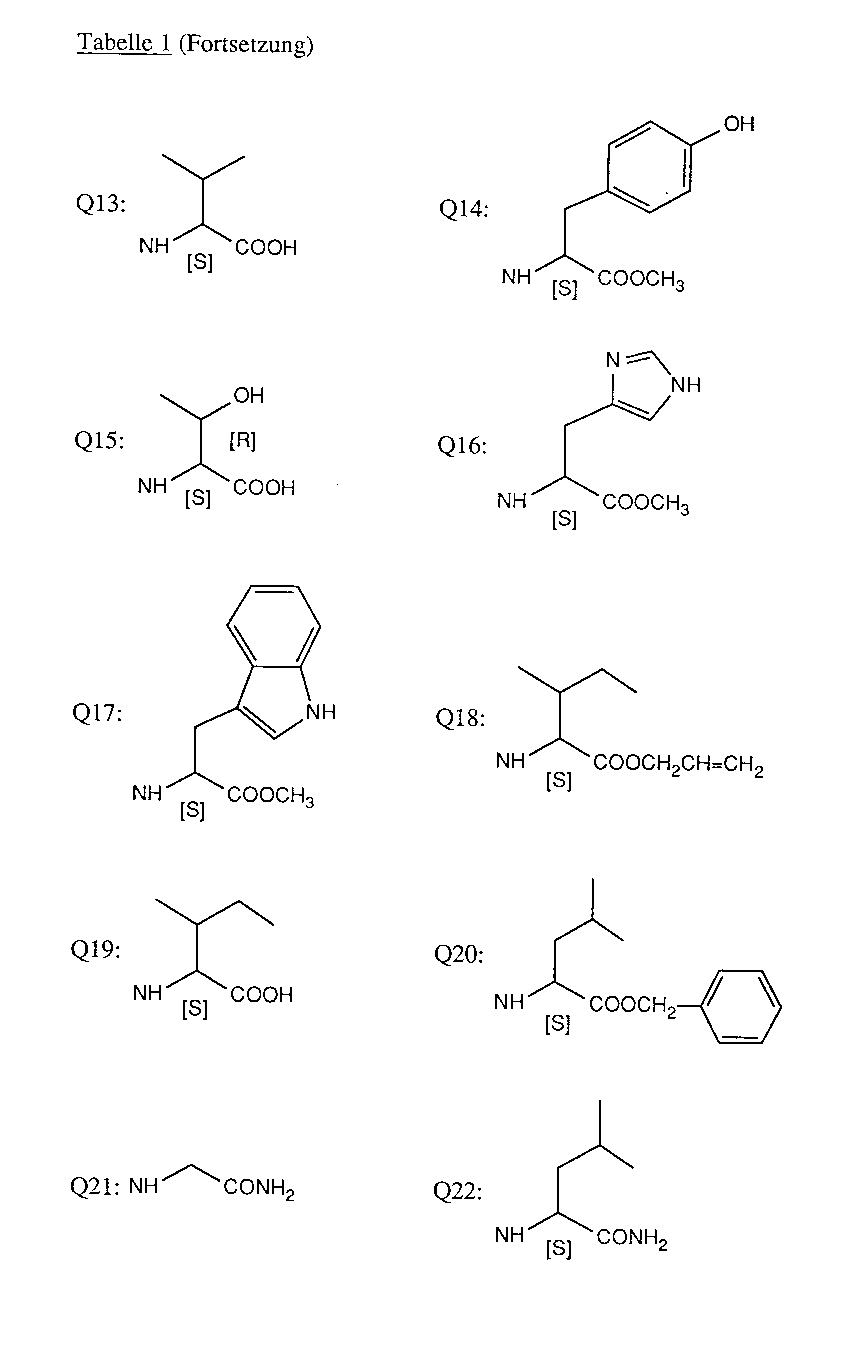 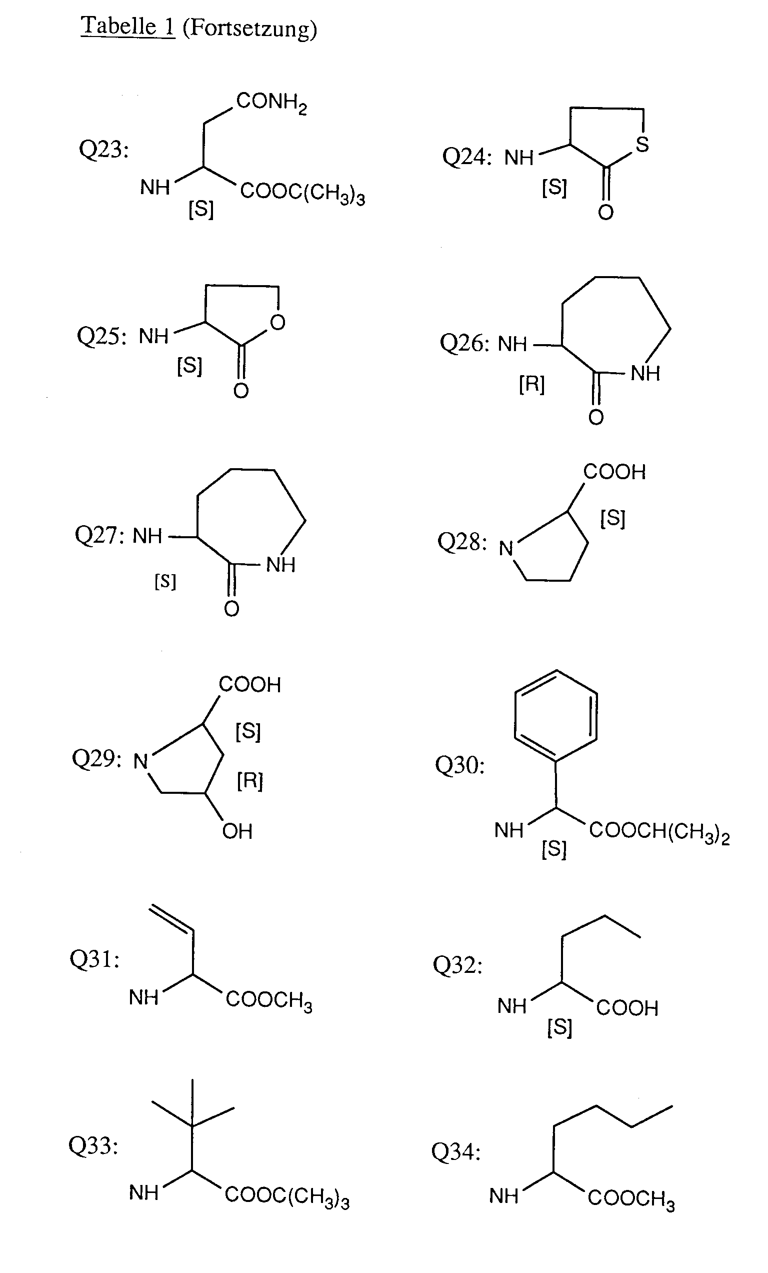 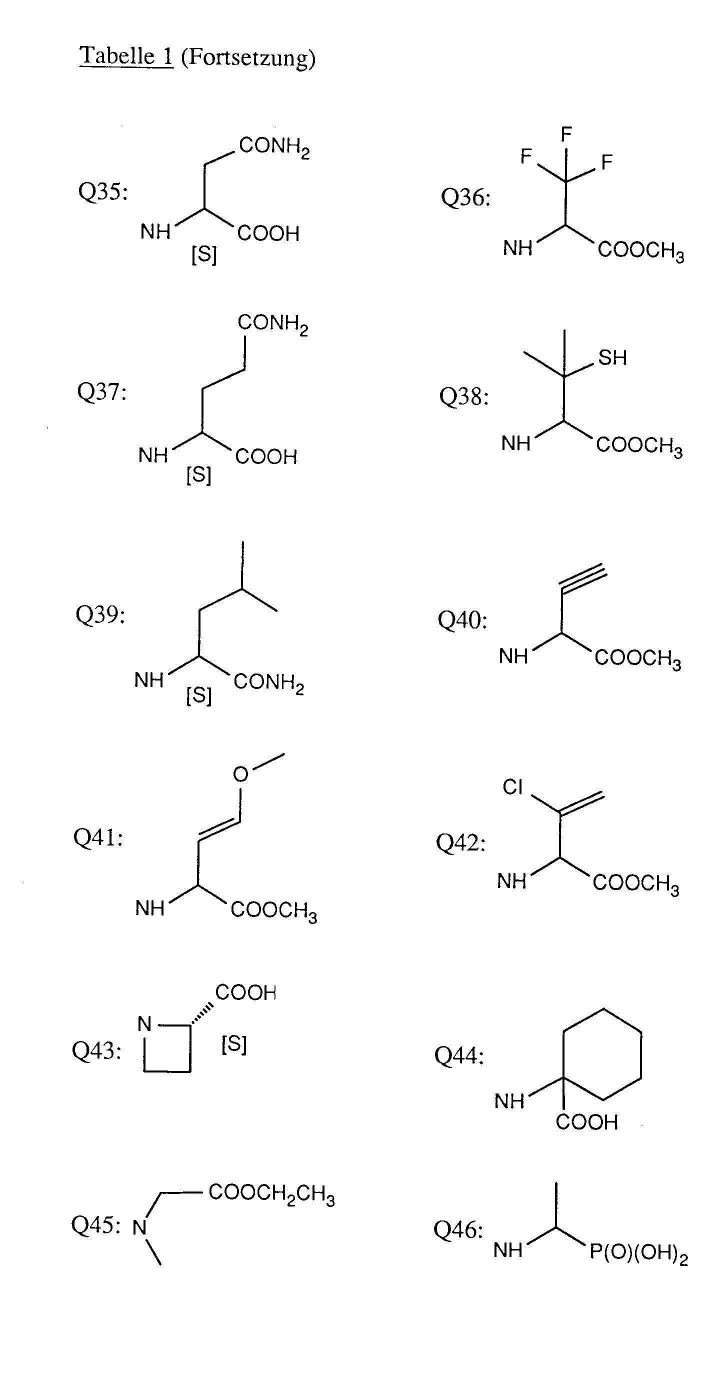 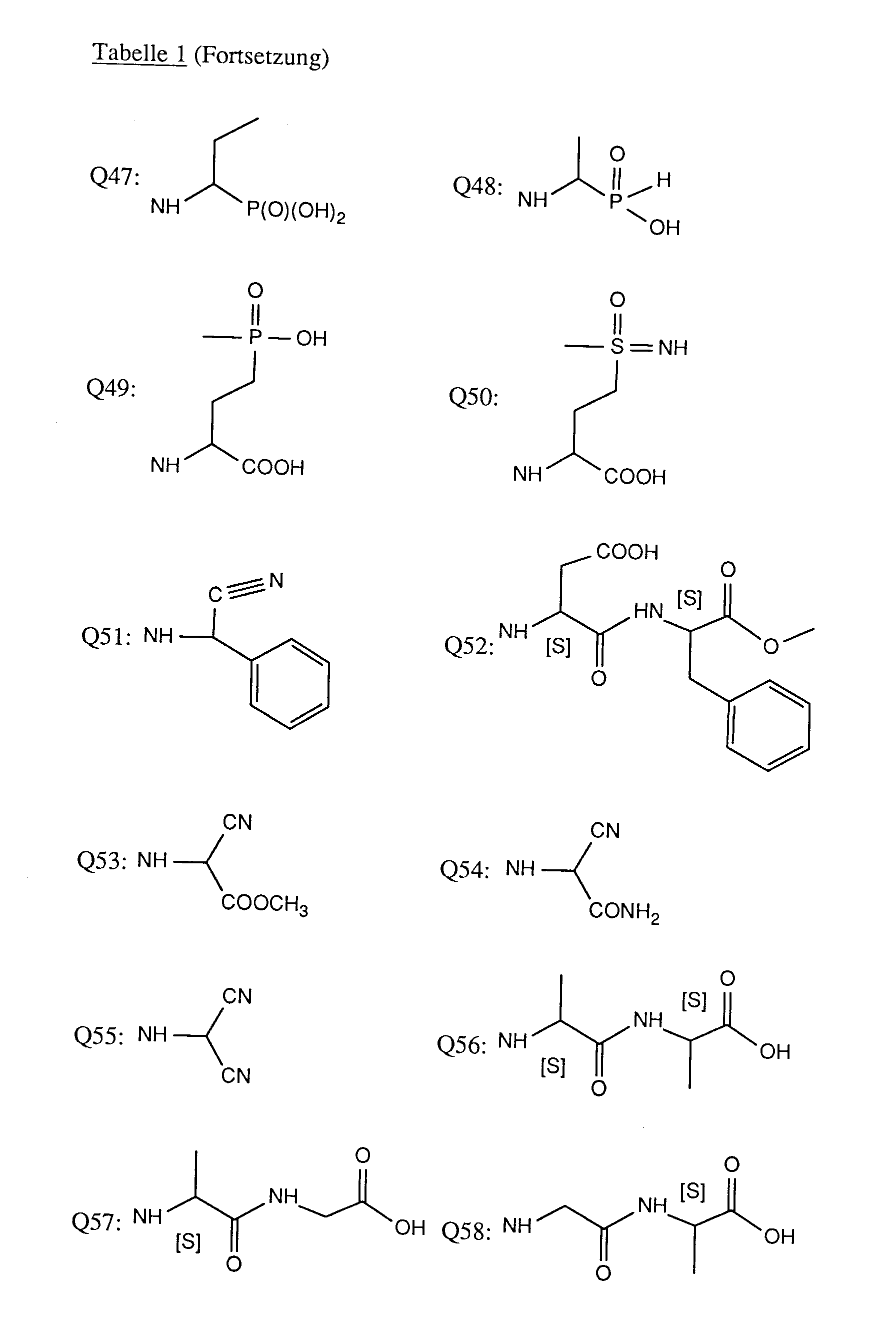 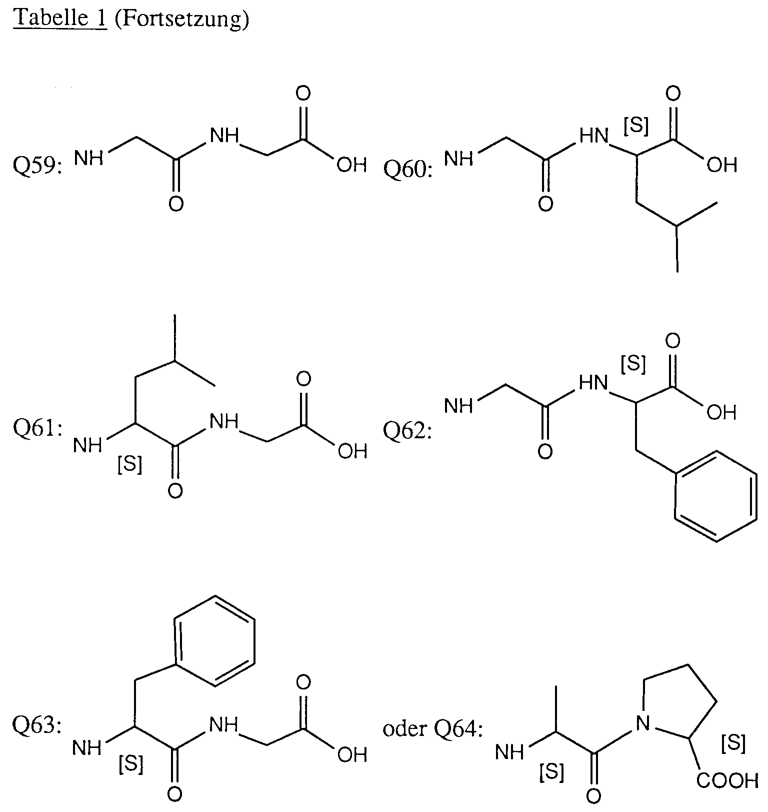
Als bevorzugte Einzelverbindungen aus dem Umfang der Formel I sind zu nennen:

N-(o-((4,6-Dimethoxy-2-pyrimidinyl)oxy)benzoyl)glycin-methylester (Beispiele 1 und 2);
- N-(o-((4,6-Dimethoxy-2-pyrimidinyl)oxy)benzoyl)-L-alaninethylester (Beispiel 20);
- rac-o-((4,6-Dimethoxy-2-pyrimidinyl)oxy)-N-(dihydro-2-oxo-3 (2H)-thienyl)benzamid (Beispiel 5):
- N-(o-((4,6-Dimethoxy-2-pyrimidinyl)oxy)benzoyl)-L-alaninmethylester (Beispiel 21 );
- N-(o-((4,6-Dimethoxy-2-pyrimidinyl)oxy)benzoyl)-L-valinmethylester (Beispiel 22);
- N-to-((4,6-Dimethoxy-2-pyrimidinyl)oxy)benzoyl)-L-leucinmethylester (Beispiel 23);
- rac-N-( 1 -Carbamoyl-1,2-dimethylpropyl)-o-((4,6-dimethoxy-2pyrimidinyl)thio)-benzamid (Beispiel 7);
- 1-(o-((4,6-Dimethoxy-2-pyrimidinyl)oxy)benzoyl)-L-prolintert-butylester (Beispiel 26);
- N-((3-((4,6-Dimethoxy-2-pyrimidinyl)oxy)-2-pyridyl)carbonyl)-L-alaninmethylester (Beispiel 27);
- N-((3-((4,6-Dimethoxy-2-pyrimidinyl)oxy)-2-pyridyl)carbonyl)glycinmethylester (Beispiel 9);
- o-((4,6-Dimethoxy-2-pyrimidinyl)oxy)-N-(<S>-1-(hydroxymethyl)-2-methylpropyl)-benzamid Beispiel 11); und
- N-(1-Cyano-1-methylethyl)-o-((4,6-dimethoxy-2-pyrimidinyl)oxy)benzamid (Beispiel 13).

Die neuen Verbindungen der Formel I können hergestellt werden, indem man eine Verbindung der Formel II 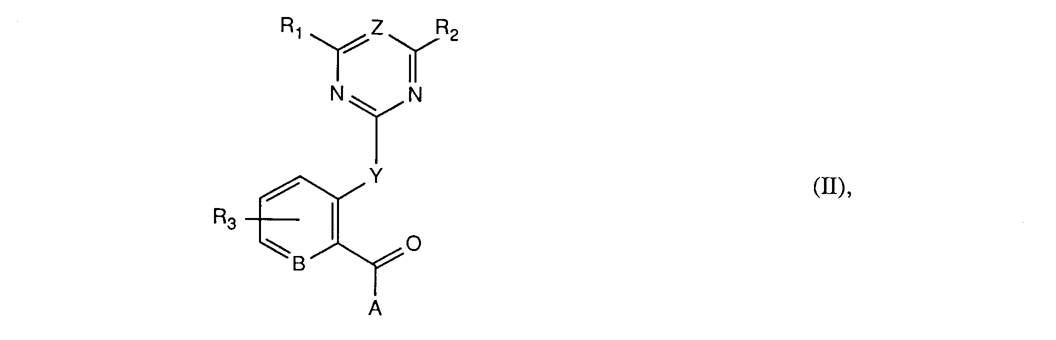
worin A eine Abgangsgruppe, vorzugsweise Chlor, oder Imidazolyl, bedeutet; oder ein Säureanhydrid der Formel II′ 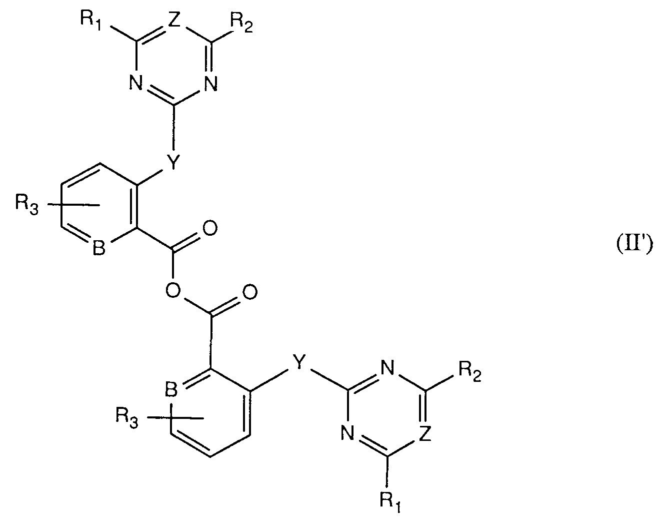
worin R₁, R₂, R₃, Y, Z und B die unter Formel I angegebene Bedeutung haben mit einem Amin der Formel III 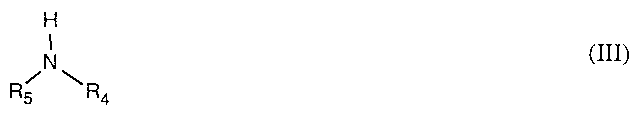
worin R₄ und R₅ die unter Formel I angegebene Bedeutung haben, umsetzt.

Die Umsetzung wird zweckmäßigerweise in Gegenwart einer Base durchführt. Geeignete Basen sind tertiäre Amine, beispielsweise Triethylamin, N,N-Dimethylanilin oder 2,5-Dimethylpyridin. Die Umsetzung wird vorzugsweise in Gegenwart eines Lösungsmittels durchgeführt. Geeignete Lösungsmittel sind:
- Kohlenwasserstoffe, beispielsweise Toluol;
- halogenierte Kohlenwasserstoffe, beispielsweise Dichlormethan, 1,2-Dichlorethan oder Chlorbenzol;
- Ether, beispielsweise Diethylether, Dimethoxyethan oder tert-Butylmethylether;
- aprotischen Lösungsmittel, beispielsweise Acetonitril,
- protischen Lösungsmittel, beispielsweise Alkohole, insbesondere Ethanol, oder Wasser, oder
- Zweiphasensysteme, wie beispielsweise ein Gemisch von Dichlormethan und Wasser, Toluol und Wasser, oder Diethylether und Wasser.

Weiter können die neuen Verbindungen der Formel I hergestellt werden, indem man eine Verbindung der Formel V 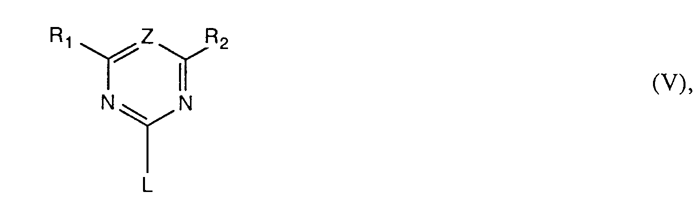
worin L eine Abgangsgruppe, insbesondere
- Halogen oder
- gegebenenfalls substituiertes Alkylsulfonyl, Benzylsulfonyl, Phenylsulfonyl, Alkylsulfonyloxy, Benzylsulfonyloxy, Phenylsulfonyloxy, oder 3-Alkylsulfonyl-1H- 1,2,4-triazol-1-yl, beispielsweise 3-Methylsulfonyl-1H 1,2,4-triazol-1-yl, und vorzugsweise Chlor, Methansulfonyl, Ethansulfonyl oder Benzylsulfonyl, bedeutet, und R₁ und R₂ die unter Formel I angegebene Bedeutung haben, mit einem Salicylamidderivat der Formel VII

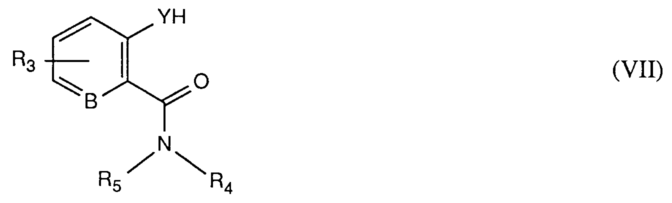

worin R₃, R₄, R₅, B und Y die unter Formel I in Anspruch 1 angegebene Bedeutung haben, umsetzt.

Zweckmäßigerweise erfolgt die Umsetzung in Gegenwart eines inerten Verdünnungsmittels, insbesondere eines organischen, zweckmäßigerweise aprotischen Lösungsmittels, und vorzugsweise
- eines aliphatischen oder cyclischen Ethers, beispielsweise Dimethoxyethan oder Tetrahydrofuran;
- eines aliphatischen Ketons, beispielsweise Aceton oder 2-Butanon;
- eines aliphatischen Nitrils, beispielsweise Acetonitril oder Propionitril;
- Dimethylformamid;
- N-Methylpyrrolidon; oder
- einer heteroaromatischen Verbindung, beispielsweise Pyridin oder Lutidin; einer Base, insbesondere
- eines Alkalimetallhydrids, beispielsweise Natriumhydrid oder Kaliumhydrid;
- eines Erdalkalimetallhydrids, beispielsweise Calciumhydrid;
- eines Alkalimetallhydrogencarbonates, beispielsweise Natrium-hydrogencarbonat oder Kaliumhydrogencarbonat;
- eines Alkalimetallcarbonates, beispielsweise Natriumcarbonat oder Kaliumcarbonat;
- eines Erdalkalimetallcarbonates, beispielsweise Calciumcarbonat oder Magnesium-carbonat;
- eines aliphatischen tertiären Amins, beispielsweise Triethylamin;
- eines vollsubstituierten Amidins, beispielsweise Diazabicycloundecen; oder
- eines basischen Heteroaromaten, beispielsweise Pyridin; eines reaktionsbeschleunigenden Zusatzes,insbesondere
- eines Kronenethers;
- eines Phasentransferkatalysators;
- einer Verbindung, welche die Abgangsgruppe L vorübergehend ersetzt, vorzugsweise Dimethylaminopyridin; oder
- einer Verbindung, welche im Falle, wo L Halogen bedeutet, die Abgangsgruppe aktiviert, vorzugsweise eines Silber- oder Kupfersalzes, beispielsweise Silbemitrat oder Kupfer-(I)-chlorid.

Die Umsetzung erfolgt in einem Temperaturbereich zwischen 0°C und 160°C, insbesondere zwischen 20°C und 100°C, vorzugsweise zwischen 20°C und dem Siedepunkt des Reaktionsgemisches.

Verbindungen der Formel II, worin A Chlor bedeutet, können beispielsweise hergestellt werden, indem man eine Säure der Formel IV 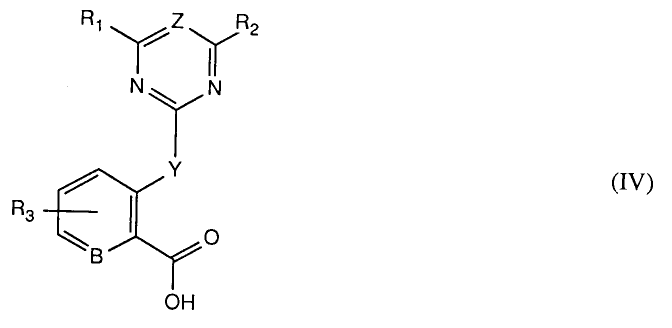
mit einem Chlorierungsmittel, wie Phosphoroxychlorid, Thionylchlorid, Oxalylchlorid oder Phosgen, vorzugsweise Phosphoroxychlorid, in Gegenwart einer Hilfsbase, wie beispielsweise Triethylamin, Dimethylanilin oder Pyridin, und gegebenenfalls in einem Lösungsmittel, wie einem Kohlenwasserstoff, beispielsweise Toluol; einem Chlorkohlenwasserstoff, beispielsweise Methylenchlorid; einem Ether, beispielsweise Tetrahydrofuran, bei einer Temperatur von -20°C bis zur Rückflußtemperatur des Rückflußgemisches, vorzugsweise von -5°C bis Raumtemperatur, umsetzt.

Es ist jedoch nicht notwendig das Zwischenprodukt der Formel II, d.h. im vorliegenden Falle das Säurechlorid, zu isolieren. Zweckmäßigerweise wird dieses "in situ" direkt mit dem Amin der Formel III umgesetzt, gegebenenfalls in Gegenwart einer zusätzlichen Base, wie beispielsweise Triethylamin. Diese Umsetzung erfolgt ebenfalls in einem Temperaturbereich von etwa -20°C bis zur Rückflußtemperatur des verwendeten Lösungsmittels, vorzugsweise zwischen -5°C und Raumtemperatur.

Anderseits ist es vorteilhaft, von einem Säureanhydrid der Formel II′ auszugehen. Diese Säureanhydride sind neue Verbindungen und bilden daher ebenfalls einen Gegenstand der vorliegenden Erfindung. Die neuen Pyrimidinyl- und Triazinyl-salicylanhydride entsprechen der Formel II′ 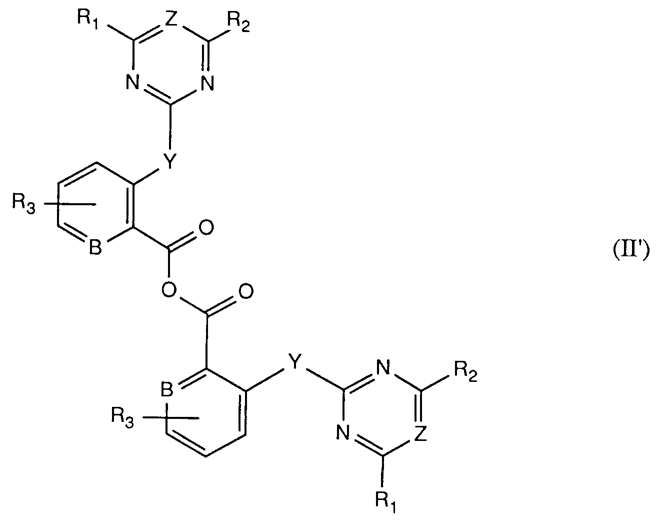
worin
B für Stickstoff, Methin, oder mit Fluor, Chlor, Methyl, Methoxy, 2-Propenyloxy, 2-Propinyloxy, Difluormethoxy oder Benzyloxy substituiertes Methin;
Y für Sauerstoff oder Schwefel;
Z für Methin oder Stickstoff;
R₁ für Chlor, Methyl, Methoxy, Ethoxy, Difluormethoxy, Methylamino, Ethylamino oder Dimethylamino;
R₂ für Methyl, Methoxy oder Difluormethoxy;
R₃ für Wasserstoff, Fluor, Chlor, Methyl oder Methoxy bedeuten.

Von den Verbindungen der Formel II′ sind diejenigen bevorzugt, in denen
a) B Methin oder Stickstoff bedeutet;
b) Y Sauerstoff bedeutet;
c) Z Methin bedeutet;
d) R₁ und R₂ Methoxy bedeuten; und
f) R₃ Wasserstoff bedeutet.

Die Verbindungen der Formel II′ können hergestellt werden, indem man eine Säure der Formel IV 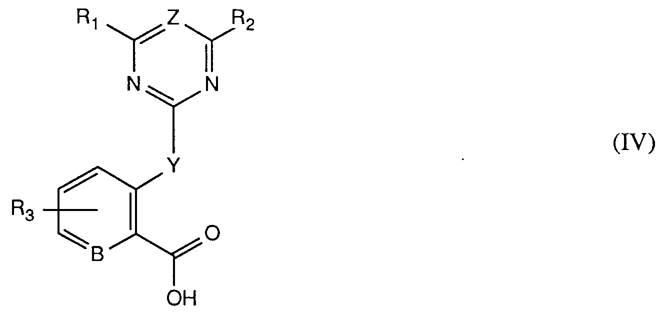
worin R₁, R₂, R₃, B und Y die unter Formel II′ angegebene Bedeutung haben, mit Phosphoroxychlorid und Triethylamin behandelt. Vorzugsweise werden dabei pro Äquivalent der Säure der Formel IV 0,50 bis 0,55 Äquivalente Phosphoroxychlorid und 2,0 bis 3,0 Äquivalente Triethylamin einsetzt.

Säuren der Formel IV sind entweder vorbekannt (beispielsweise aus den EP-Al O 223 406, 0 249 707, 0 249 708, 0 287 079, 0 315 889, 0 336 494 und 0 346 789), oder sie können nach den bekannten Verfahren, beispielsweise durch Kondensation einer Verbindung der Formel V, worin die Substituenten die oben angegebene Bedeutung haben, wobei L jedoch vorzugsweise Chlor, Methansulfonyl, Ethansulfonyl oder Benzylsulfonyl bedeutet, mit einem Salicylsäurederivat der Formel VI 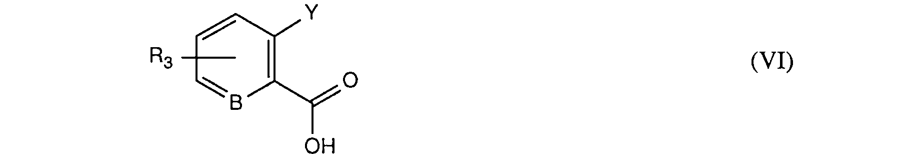
worin die Substituenten die oben angegebene Bedeutung haben, in Gegenwart einer geeigneten Base, wie Kaliumcarbonat, Kaliumhydroxid oder Natriumhydrid, und in Gegenwart eines geeigneten Lösungsmittels, wie Aceton, Methylethylketon, Acetonitril, Dimethylformamid oder Dimethylsulfoxid, zweckmäßigerweise in einem Temperaturbereich von 0°C bis zur Rückflußtemperatur, vorzugsweise aber zwischen 10°C und 60°C, hergestellt werden.

Verbindungen der Formeln III, V, VI und VII sind entweder vorbekannt, oder sie können nach den an sich aus der Literatur bekannten Verfahren hergestellt werden.

Die Verbindungen der Formel I (im folgenden auch als "Wirkstoffe" bezeichnet) besitzen herbizide Eigenschaften und eignen sich zur Bekämpfung von Unkräutem, einschließlich Ungräsern, so u.a. von Agropyron repens, Alopecurus myosuroides, Avena fatua, Bromus inermis, Echinochloa crus-galli, Poa annua, Sorghum halepense, Abutilon theophrasti, Amaranthus retroflexus, Cassia obtusifolia, Chenopodium album, Galium aparine, Matricaria chamomilla, Sinapis arvensis und Stellaria media, in verschiedenen Nutzpflanzenkulturen, so u.a. Kulturen von Reis, insbesondere Wasserreis, Weizen, Mais, Soja, Raps, Sonnenblumen und Baumwolle. Zudem sind die Verbindungen sowohl Vorauflauf- als auch Nachauflauf-Herbizide. Bei einigen Vertretern der Verbindundungen der Formel I hat sich eine gute Selektivität gezeigt, beispielsweise bei der Bekämpfung von Unkräutern in Soja- und Baumwolle-Kulturen.

Weiter besitzen die Verbindungen der Formel I pflanzenwachstumsregulierende Eigenschaften und eignen sich als Wirkstoffe zur positiven Beeinflussung des Wachstums von Nutzpflanzen. Dieser Effekt kann sowohl bei Kulturpflanzen eine erwünschte Wachstumshemmung herbeiführen als auch Unkräuter in ihrer Keimung genügend hemmen, um sie als Konkurrenten der Kulturpflanzen auszuschalten. Im Hinblick auf ökologische Zusammenhänge ist dies von Vorteil und demzufolge äußerst wünschenswert. Insbesondere sind dabei der Schutz der Erdoberfläche vor Austrocknung und/oder Erosion sowie die Verminderung des Unkraut-Samenvorrates im Boden (bei gleichzeitiger Verhinderung der Blüte und der eneuerten Versamung) zu nennen. Daher ist diese Wirkung unter Umständen der Vorzug zu geben vor der völligen, aber eventuell zeitlich begrenzten Verhinderung der Unkrautbildung.

In der Praxis genügt üblicherweise eine Konzentration von 1 g bis 3 kg der Verbindung der Formel I pro ha, vorzugsweise von 10 g bis 1 kg/ha, um den gewünschten herbiziden Effekt zu erzielen. Um den gewünschten herbiziden Effekt bei optimaler Nutzpflanzen-Verträglichkeit zu erzielen, ist der Bereich von 10 bis 100 g/ha in der Vorauflaufbehandlung und von 100 bis 1000 g/ha in der Nachauflaufbehandlung besonders günstig.

Die erfindungsgemäßen Unkrautbekämpfungsmittel und Pflanzenwachstumsregulierende Mittel enthalten eine wirksame Menge mindestens einer Verbindung der Formel I sowie in aller Regel außerdem Formulierungshilfsstoffe. Zweckmäßigerweise enthalten sie zumindest je einen Formulierungshilfsstoff aus jeder der folgenden Gruppen:
- feste Trägerstoffe;
- Lösungs- bzw. Dispersionsmittel;
- Tenside (Netzmittel und Emulgatoren);
- Dispergiermittel (ohne Tensidwirkung); und
- Stabilisatoren.

Die pestiziden Zubereitungen enthalten in der Regel neben den Wirkstoffen der Formel I 1 bis 99 % eines Formulierungshilfsstoffs aus der Gruppe
- feste Trägerstoffe;
- Lösungs- bzw. Dispersionsmittel:
- Dispergiermittel (ohne Tensidwirkung); und
- Stabilisatoren; und 0 bis 25 %, insbesondere 0,1 bis 25 % eines Tensides (Netzmittel und Emulgatoren).

Unter Verwendung solcher und anderer Hilfsstoffe können die Verbindungen der Formel I, d.h. die herbiziden Wirkstoffe, in die üblichen Formulierungen, wie Stäube, Pulver, Granulate, Lösungen, Emulsionen, Suspensionen, emulgierbare Konzentrate, Pasten und dgl., übergeführt werden.

Die Verbindungen der Formel I sind im allgemeinen wasserunlöslich und können nach für wasserunlösliche Verbindungen üblichen Methoden unter Verwendung der bekannten Formulierungshilfsstoffe konfektioniert werden. Die Herstellung der Mittel kann in an sich bekannter Weise duchgeführt werden, beispielsweise durch Vermischen des jeweiligen Wirkstoffes mit festen Trägerstoffen, durch Auflösen oder Suspendieren in geeigneten Lösungs- bzw. Dispersionsmitteln, gegebenenfalls unter Verwendung von Tensiden als Netzmitteln oder Emulgatoren und/oder Dispergiermitteln, oder durch Verdünnen bereits vorbereiteter emulgierbarer Konzentrate mit Lösungs- bzw. Dispersionsmitteln.

Als feste Trägerstoffe kommen im wesentlichen in Frage:
- natürliche Mineralstoffe, wie Kreide, Dolomit, Kalkstein, Tonerden und Kieselsäure und deren Salze, beispielsweise Kieselgur, Kaolin, Bentonit, Talkum, Attapulgit oder Montmorillonit;
- synthetische Mineralstoffe, wie hochdisperse Kieselsäure, Aluminiumoxid oder Silikate;
- organische Stoffe, wie Cellulose, Stärke, Harnstoff oder Kunstharze; und
- Düngemittel, wie Phosphate oder Nitrate.

Solche Trägerstoffe können beispielsweise als Pulver oder als Granulate vorliegen.

Als Lösungs- bzw. Dispersionsmittel kommen im wesentlichen in Frage:
- Aromate, wie Benzol, Toluol, Xylol und Alkylnaphthaline;
- chlorierte Aromaten und chlorierte aliphatische Kohlenwasserstoffe, wie Chlorbenzol, Chlorethylene oder Methylenchlorid;
- aliphatische Kohlenwasserstoffe, wie Cyclohexan oder Paraffine, beispielsweise Erdölfraktionen;
- Alkohole, wie Butanol oder Glykol, und deren Ether und Ester;
- Ketone, wie Aceton. Methylethylketon. Methylisobutylketon oder Cyclohexanon; und
- stark polare Lösungs- bzw. Dispersionsmittel, wie Dimethylformamid, N-Methyl-pyrrolidon oder Dimethylsulfoxid (wobei solche Lösungsmittel vorzugsweise Flammpunkte von mindestens 30°C und Siedepunkte von mindestens 50°C aufweisen), oder Wasser.

Weiter kommen als Lösungs- bzw. Dispersionsmittel auch sogenannte verflüssigte gasförmige Streckmittel oder Trägerstoffe, welche Produkte sind, die bei Raumtemperatur und unter Normaldruck gasförmig sind, in Frage. Beispiele solche Produkte sind insbesondere Aerosol-Treibgase, wie Halogenkohlenwasserstoffe, beispielsweise Dichlordifluormethan.

Liegt das erfindungsgemäße Unkrautbekämpfungsmittel in Form einer Druckgaspackung vor, so wird zweckmäßigerweise zusätzlich zum Treibgas ein Lösungsmittel eingesetzt.

Die Tenside (Netzmittel und Emulgatoren) können nichtionische Verbindungen sein, wie:
- Kondensationsprodukte von Fettsäuren, Fettalkoholen oder fettsubstituierten Phenolen mit Ethylenoxid;
- Fettsäureester und -ether von Zuckern oder mehrwertigen Alkoholen;
- die Produkte, welche aus Zuckern oder mehrwertigen Alkoholen durch Kondensation mit Ethylenoxid erhalten werden;
- Blockpolymere von Ethylenoxid und Propylenoxid; oder
- Alkyldimethylaminoxide.

Die Tenside können auch anionische Verbindungen sein, wie:
- Seifen;
- Fettsulfatester, beispielsweise Dodecylnatriumsulfat, Octadecylnatriumsulfat oder Cetylnatriumsulfat;
- Alkylsulfonate, Arylsulfonate oder fettaromatische Sulfonate, wie Alkylbenzolsulfonate, beispielsweise Calcium-dodecylbenzolsulfonat oder Butylnaphthalen-sulfonate; oder
- komplexere Fettsulfonate, beispielsweise die Amidkondensationsprodukte von Ölsäure und N-Methyltaurin, oder das Natriumsulfonat von Dioctylsuccinat.

Schließlich können die Tenside kationische Verbindungen sein, wie Alkyldimethyl-benzylammoniumchloride, Dialkyldimethylammoniumchloride, Alkylmethylammoniumchloride oder ethoxylierte Ammoniumchloride.

Als Dispergiermittel (ohne Tensidwirkung) kommen im wesentlichen in Frage: Lignin, Natrium- und Ammoniumsalze von Ligninsulfonsäuren, Natriumsalze von Maleinsäureanhydrid-Diisobutylen-Copolymeren, Natrium- und Ammoniumsalze von sulfonierten Polykondensationsprodukten aus Naphthalen und Formaldehyd, oder Sulfitablaugen.

Als Dispergiermittel, welche sich insbesondere als Verdickungs- bzw. Antiabsetzmittel eignen, können beispielsweise Methylcellulose, Carboxymethylcellulose, Hydroxyethylcellulose, Polyvinylalkohol, Alginate , Caseinate oder Blutalbumin eingesetzt werden.

Beispiele von geeigneten Stabilisatoren sind:
- säurebindende Mittel, beispielsweise Epichlorhydrin, Phenylglycidether oder Soyaepoxide;
- Antioxidantien, beispielsweise Gallussäureester oder Butylhydroxytoluol;
- UV-Absorber, beispielsweise substituierte Benzophenone, Diphenylacrylonitrilsäureester oder Zimtsäureester: oder
- Deaktivatoren, beispielsweise Salze der Ethylendiaminotetraessigsäure oder Polyglykole.

Die erfindungsgemäßen Unkrautbekämpfungsmittel und pflanzenwachstumsregulierenden Mittel können zusätzlich zu den Verbindungen der Formel I noch weitere Wirkstoffe, wie beispielsweise Insektizide, Akarizide, Nematizide, Molluskizide, Bakterizide, Fungizide, Herbizide, Pflanzenwachstumsregulatoren, Düngemittel und Spurenelement-Vermittler, enthalten. Solche Kombinationsmittel eignen sich zur Verbreiterung des Wirkungsspektrums.

Die erfindungsgemßen Unkrautbekämpfungsmittel enthalten im allgemeinen zwischen 0,001 und 95 Gewichtsprozent, vorzugsweise zwischen 0,5 und 75 Gewichtsprozent, einer oder mehrerer der Verbindungen der Formel I als Wirkstoff. Sie können beispielsweise in einer Form vorliegen, die sich für die Lagerung und den Transport eignet. In solchen Formulierungen, beispielsweise emulgierbaren Konzentraten, ist die Wirkstoffkonzentration normalerweise im höheren Bereich, vorzugsweise zwischen 1 und 50 Gewichtsprozent, insbesondere zwischen 5 und 30 Gewichtsprozent. Diese Formulierungen können dann, beispielsweise mit gleichen oder verschiedenen inerten Stoffen, bis zur Wirkstoffkonzentration verdünnt werden, die sich für den praktischen Gebrauch eignet, also vorzugsweise etwa 0,001 bis 10 Gewichtsprozent, insbesondere etwa 0,005 bis 5 Gewichtsprozent. Die Wirkstoffkonzentrationen können jedoch auch kleiner oder größer sein.

Wie erwähnt, kann die Herstellung der erfindungsgemäßen Unkrautbekämpfungsmittel in an sich bekannter Weise durchgeführt werden.

Zur Herstellung pulverförmiger Präparate kann der Wirkstoff, d.h. mindestens eine Verbindung der Formel I, mit einem festem Trägerstoff vermischt werden, beispielsweise durch Zusammenmahlen. Oder man kann den festen Trägerstoff mit einer Lösung oder Suspension des Wirkstoffes imprägnieren und dann das Lösungsbzw. Dispersionsmittel durch Abdunsten, Erhitzen oder Absaugen unter vermindertem Druck entfernen. Durch Zusatz von Tensiden bzw. Dispergiermitteln kann man solche pulverförmige Mittel mit Wasser leicht benetzbar machen, so daß sie in wäßrige Suspensionen, die sich beispielsweise als Spritzmittel eignen, übergeführt werden können.

Der Wirkstoff kann auch mit dem Tensid und einem festen Trägerstoff zur Bildung eines benetzbaren Pulvers, welches in Wasser dispergierbar ist, vermischt werden, oder er kann mit einem festen, vorgranulierten Trägerstoff zur Bildung eines granulatförmigen Produktes vermischt werden.

Gewünschtenfalls kann der Wirkstoff in einem mit Wasser nicht mischbaren Lösungsmittel, wie beispielsweise einem hochsiedenden Kohlenwasserstoff, gelöst werden. Dieses enthält zweckmäßigerweise gelösten Emulgator, so daß die Lösung bei ihrer Zugabe zu Wasser selbstemulgierend ist. Anderseits kann der Wirkstoff mit einem Emulgator vermischt und das Gemisch dann mit Wasser auf die gewünschte Konzentration verdünnt werden. Zudem kann der Wirkstoff in einem Lösungsmittel gelöst und danach mit einem Emulgator vermischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die erfindungsgemäße Verwendung der beschriebenen Unkrautbekämpfungsmittel kann nach üblichen Applikationsmethoden, wie Spritzen, Sprühen, Stäuben, Gießen oder Streuen. erfolgen.

Die folgenden Beispiele dienen der näheren Erläuterung der Erfindung.

### A. Herstellung von Verbindungen der Formel I

### Beispiel 1: N-(o-((4,6-Dimethoxy-2-pyrimidinyl)oxy)benzoyl)glycinmethylester

12,6 g o-((4,6-Dimethoxy-2-pyrimidinyl)oxy)benzoesäure werden in 500 ml Dichlormethan gelöst und mit 30 g Triethylamin versetzt. Bei -5°C tropft man 7,5 g Phosphoroxychlorid, gelöst in 25 ml Dichlormethan, zu und rührt 5 min nach. Unter intensivem Rühren wird dann eine Lösung von 8,9 g frisch hergestelltem Glycinsäuremethylester in 100 ml Wasser zugegeben. Man läßt unter Nachrühren innerhalb von 15 min Raumtemperatur erreichen. Man trennt dann die organische Phase ab, wäscht dreimal mit frischem Wasser, trocknet über Magnesiumsulfat und dampft ein. Der zurückbleibende Rückstand wird mittels Flashchromatographie gereinigt. Man erhält als nahezu farbloses Öl reinen
N-(o-((4,6-Dimethoxy-2-pyrimidinyl)oxy)benzoyl)glycinmethylester, ¹H-NMR(CDCl3): 3,61, s, COOCH₃, 3,81. s, 2malOCH₃, 4,16, d, J = 5 Hz, CH₂, 5,82, s, -CH=, 7,22 - 8,06, NH und 4 aromatische H-Atome.

### Beispiel 2: N-(o-((4,6-Dimethoxy-2-pyrimidinyl)oxy)benzoyl)glycinmethylester

5,35 g Bis-o-((4,6-Dimethoxy-2-Pyrimidinyl)oxy)benzoesäureanhydrid und 2 g Triethylamin werden in 100 ml Dichlormethan vorgelegt. Unter Rühren fügt man eine frisch zubereitete Lösung von 1,78 g Glycinsäuremethylester in 15 ml Wasser zu und läßt 1 Stunde bei Raumtemperatur nachrühren. Man trennt die organische Phase ab, wäscht mit NatriumhydrogencarbonatLösung und mit Wasser nach, trocknet und dampft ein. Man erählt so 3,5 g nahezu reines Produkt, das nach TLC mit dem in Beispiel 1 hergestellten N-(o-((4,6-Dimethoxy-2-pyrimidinyl)oxy)benzoyl)glycinmethylester identisch ist (TCL-Laufmittel: Essigester/Hexan 2: 1, Rf-Wert: 0,55).

### Beispiel 3: N-(o-((4,6-Dimethoxy-2-pyrimidinyl)oxy)benzoyl)-L-alanin

6,0 g N-(o-((4,6-Dimethoxy-2-pyrimidinyl)oxy)benzoyl)-L-alanin-tert-butylester (Beispiel 31 ) werden in 40 ml Trifluoressigsäure gelöst und die Lösung im verschlossenen Reaktionsgefäß 2 Tage bei Raumtemperatur gehalten. Die leichtflüchtigen Anteile werden dann am Rotationsverdampfer entfernt. Der zurückbleibende ölige Rückstand wird in Dichlormethan aufgenommen und zweimal mit 300 ml Wasser gewaschen. Man trocknet über Natriumsulfat und dampft ein. Das zurückbleibende Öl wird zur weiteren Reinigung mittels Gradient-Flashchromatographie gereinigt. Der gummiartige Rückstand von 4,8 g ist reines N-(o-((4,6-Dimethoxy-2-pyrimidinyl)oxy)benzoyl)-L-alanin: R_{f}: 0,19 (Essigsäureethylester/Methanol 3:1).

### Beispiel 3a: N-(o-((4,6-Dimethoxy-2-pyrimidinyl)oxy)benzoyl)-(L-Leu)-(L-Al a)-ethylester

3.7 g N-(o-((4,6-Dimethoxy-2-Pyrimidinyl)oxy)benzoyl)-L-Leucin werden in 250 ml Dichlormethan aufgenommen, der Reihe nach mit 3,3 g L-Alaninethylester-hydrochlorid und 7,5 g Triethylamin versetzt und unter Rühren auf -5°C gekühlt. Bei dieser Temperatur wird eine Lösung von 1,6 g Phosphoroxychlorid in 5 ml Dichlormethan zugetropft anschließend 20 min lang gerührt. Dann wird die organische Phase dreimal mit 250 ml Wasser gewaschen, über Natriumsulfat getrocknet und eingedampft. Das zurückbleibende Produkt wird mittels GradientFlashchromatographie gereinigt. Man erhält als glasartiges Produkt reinen N-(o-((4,6-Dimethoxy-2-pyrimidinyl)oxy)benzoyl)(L-Leu)-(L-Ala)-ethylester, R_{f}: 0,36 (Essigsäureethylester/Hexan 2:1).

### Beispiel 3b: N-(o-((4,6-Dimethoxy-2-pyrimidinyl)oxy)benzoyl)-(L-Phe)-(L-Ala)-ethylester

Auf gleiche Weise erhält man aus N-(o-((4,6-Dimethoxy-2pyrimidinyl)oxy)benzoyl)-L-phenylalanin und L-Alaninethylester-hydrochlorid als glasartiges Produkt den N-(o-((4,6-Dimethoxy-2-pyrimidinyl)oxy)benzoyl)-(L-phe)-(L-Ala)-ethylester, R_{f}: 0,48 (Essigsäureethylester/Hexan 2:1).

### B. Herstellung der Zwischenprodukte der Formel II

### Beispiel 3c: Bis-o-((4,6-Dimethoxy-2-pyrimidinyl)oxy)benzoesäureanhydrid

27,6 g Bis-o-((4,6-Dimethoxy-2-pyrimidinyl)oxy)benzoesäure werden in eine Lösung von 25 g Triethylamin in 500 ml Dichlormethan eingetragen. Die Lösung wird auf -5°C gekühlt und dann bei dieser Temperatur mit einer Lösung von 8 g Phosphoroxychlorid in 25 ml Dichlormethan behandelt. Nach lOminütigem Nachrühren wird die Lösung zweimal kurz mit je 500 ml Eiswasser gewaschen. Die organische Phase wird getrocknet und eingedampft. Durch Umkristallisation mit Essigester/Hexan (7:8) erhält man 24,2 g reines Bis-o-((4,6-Dimethoxy-2-pyrimidinyl)oxy)bezoesäureanhydrid; Smp.: 120- 122°C.

Analog den Beispielen 1 bis 3 werden die in den Tabellen 2 bis 5 aufgeführten Verbindungen der Formeln IB, IC, ID und IE hergestellt.

Wenn nicht anders angegeben, wurden die R_{f}-Werte in Essigsäureethylester/Hexan ermittelt.

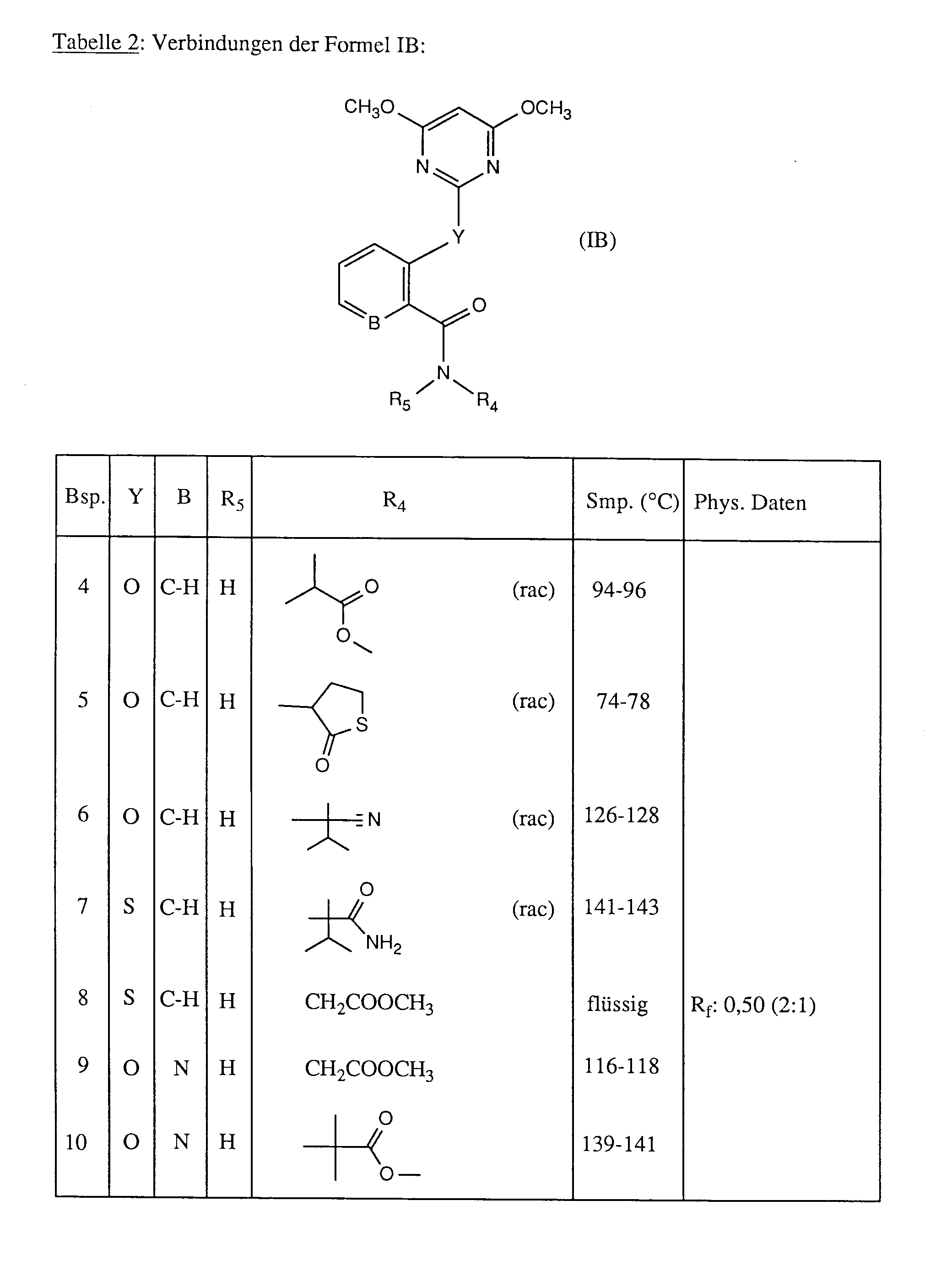

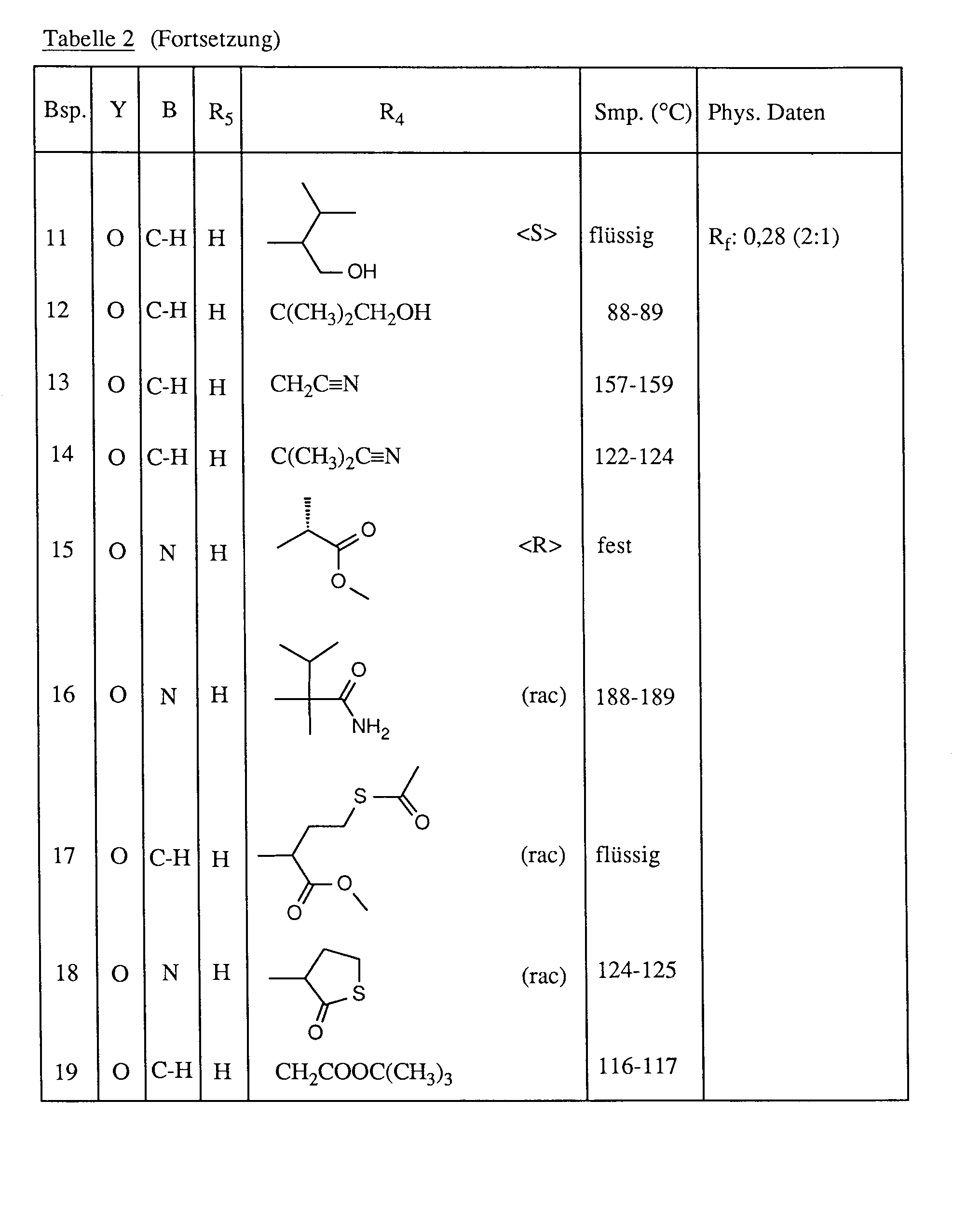

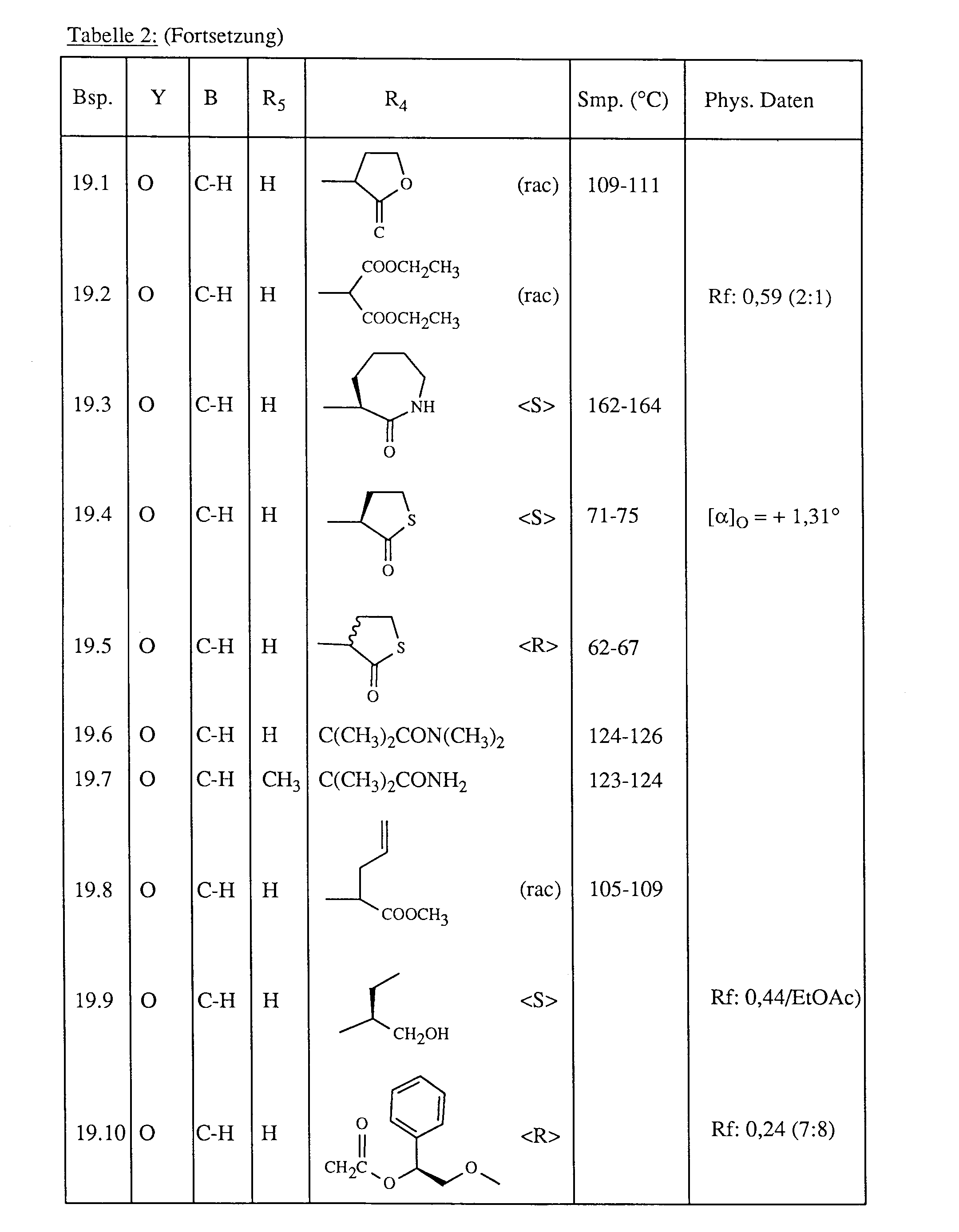

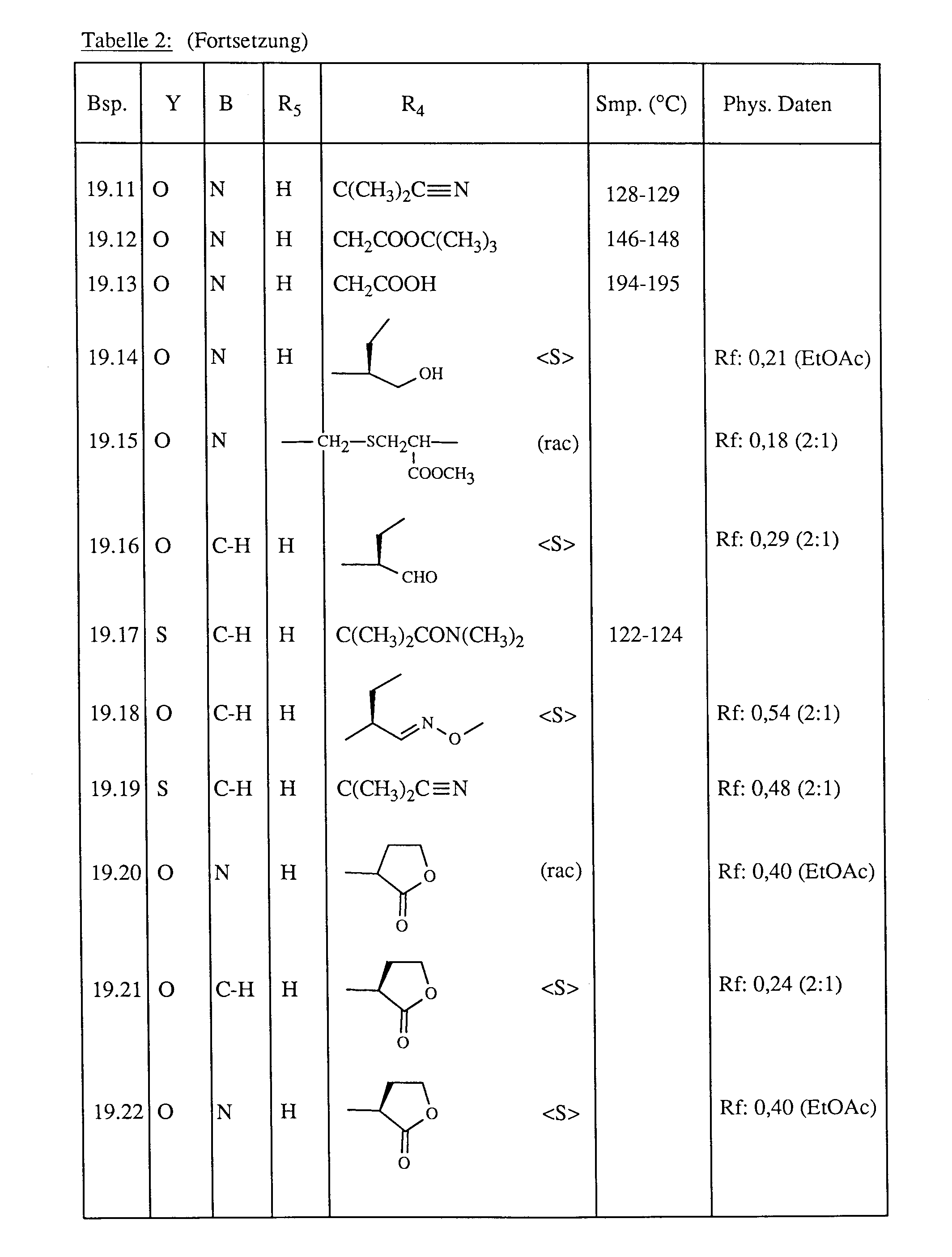

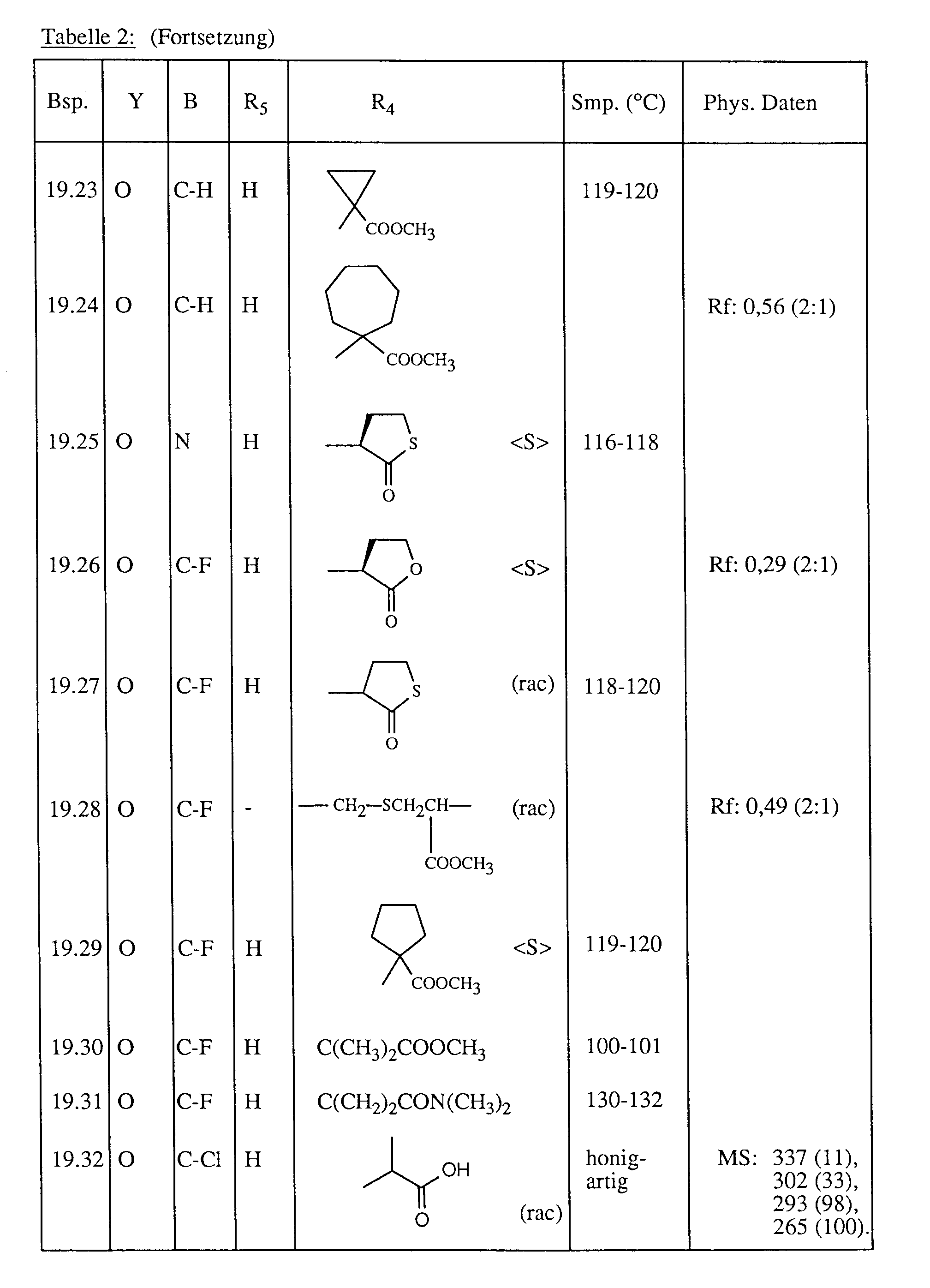

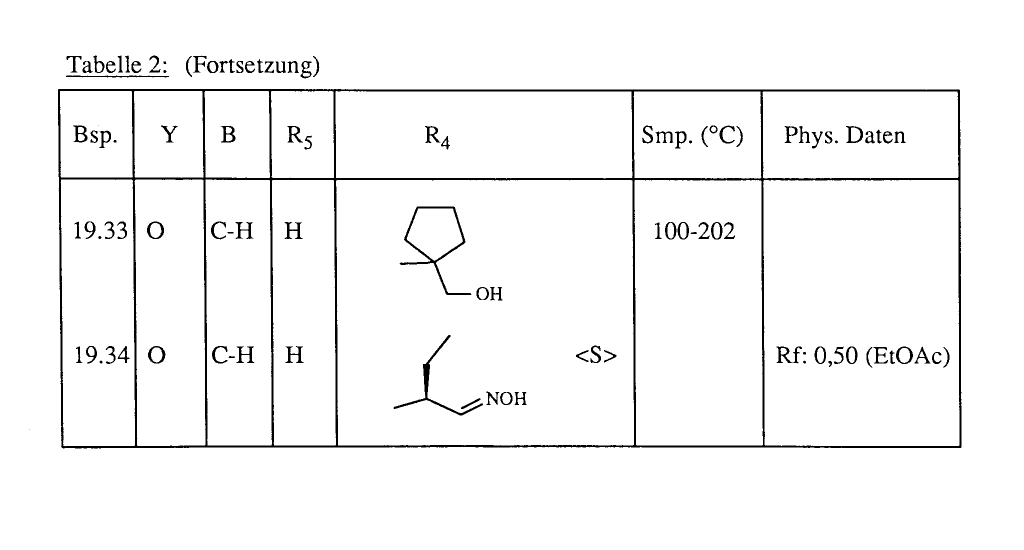

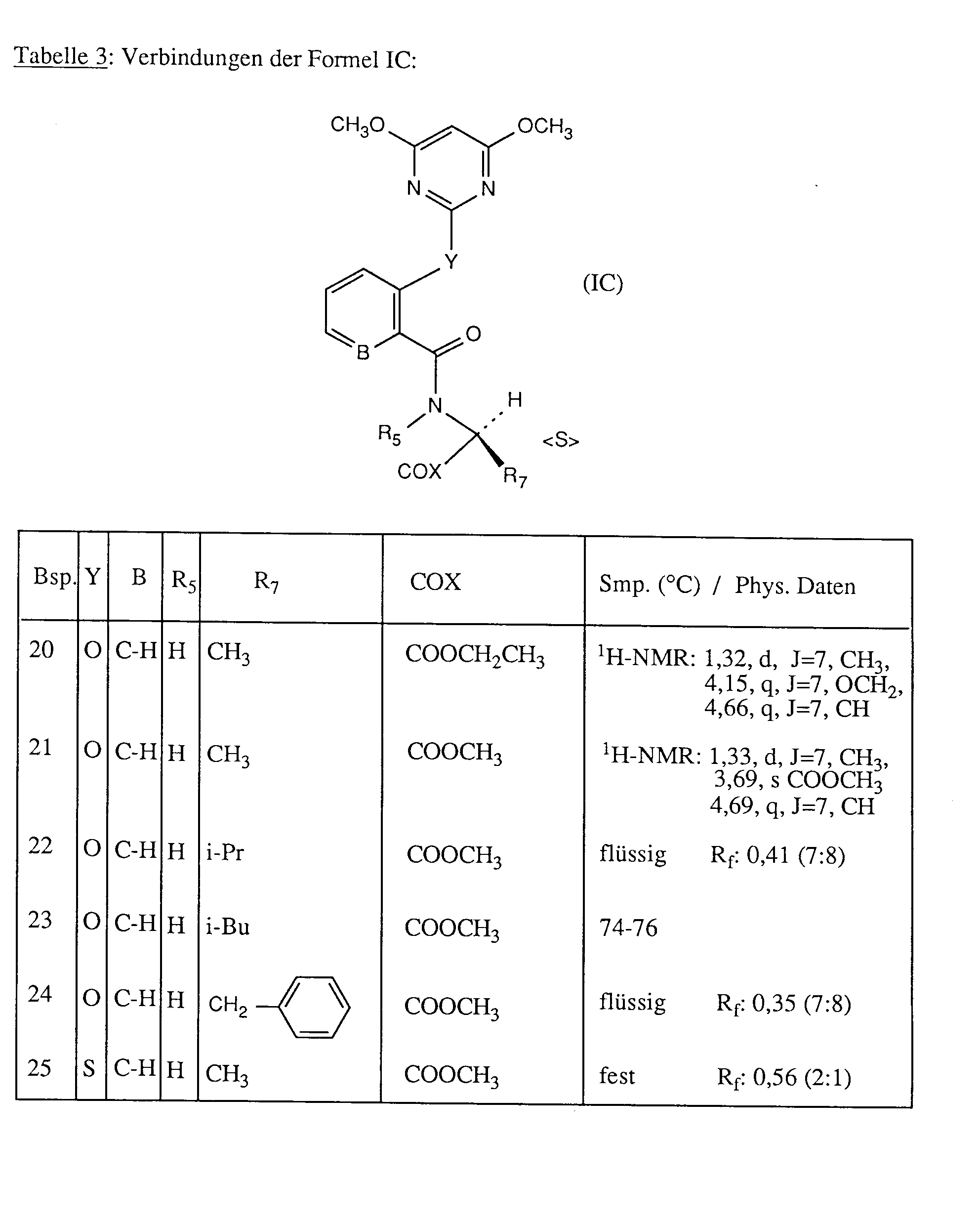

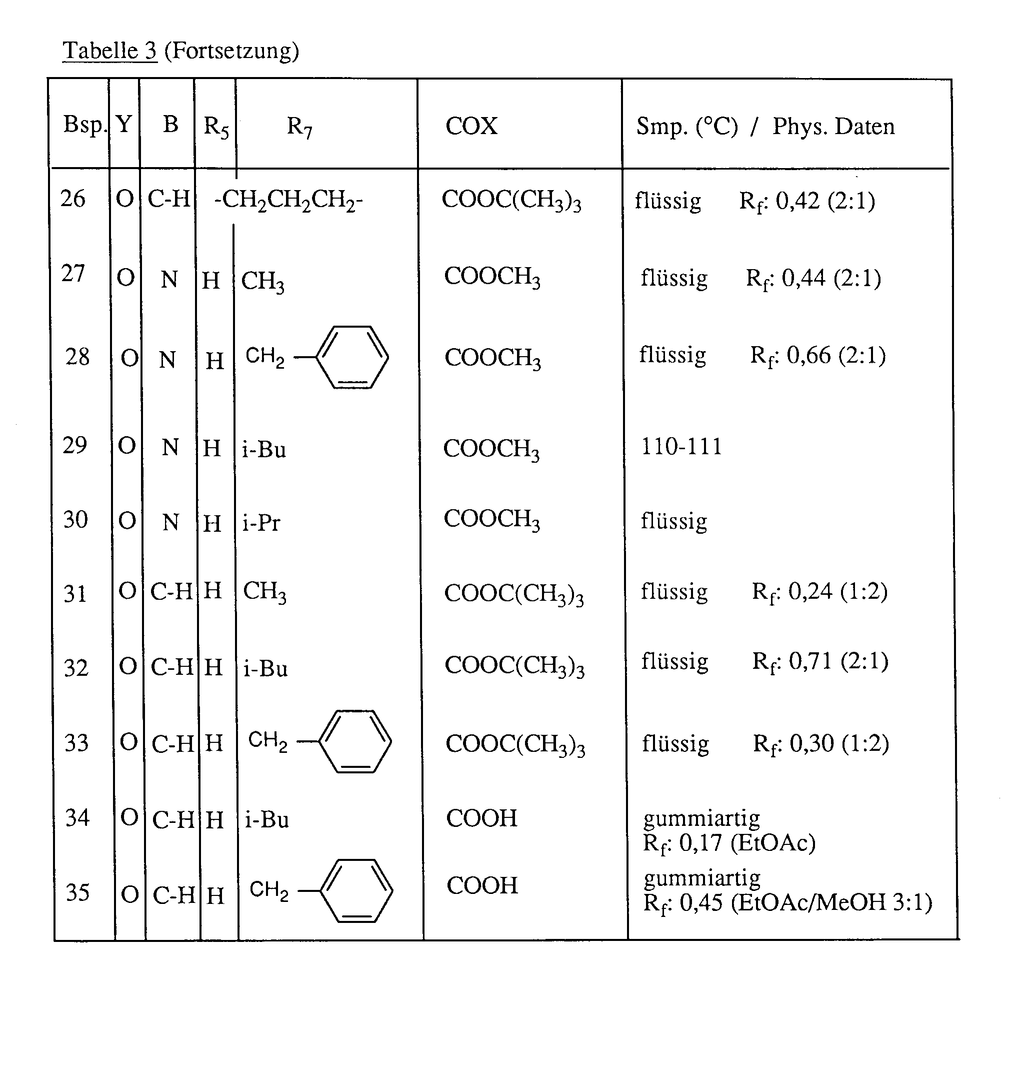

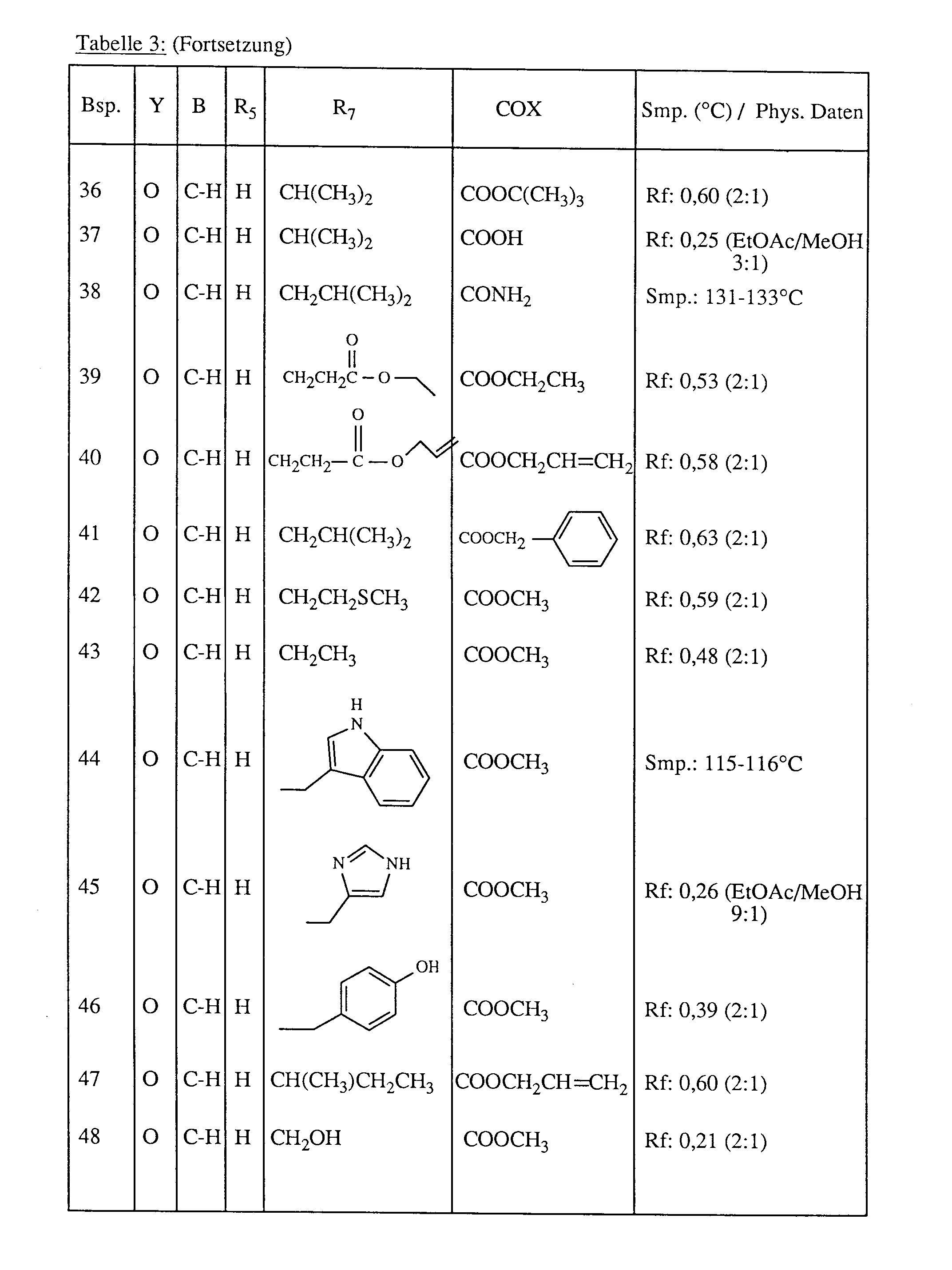

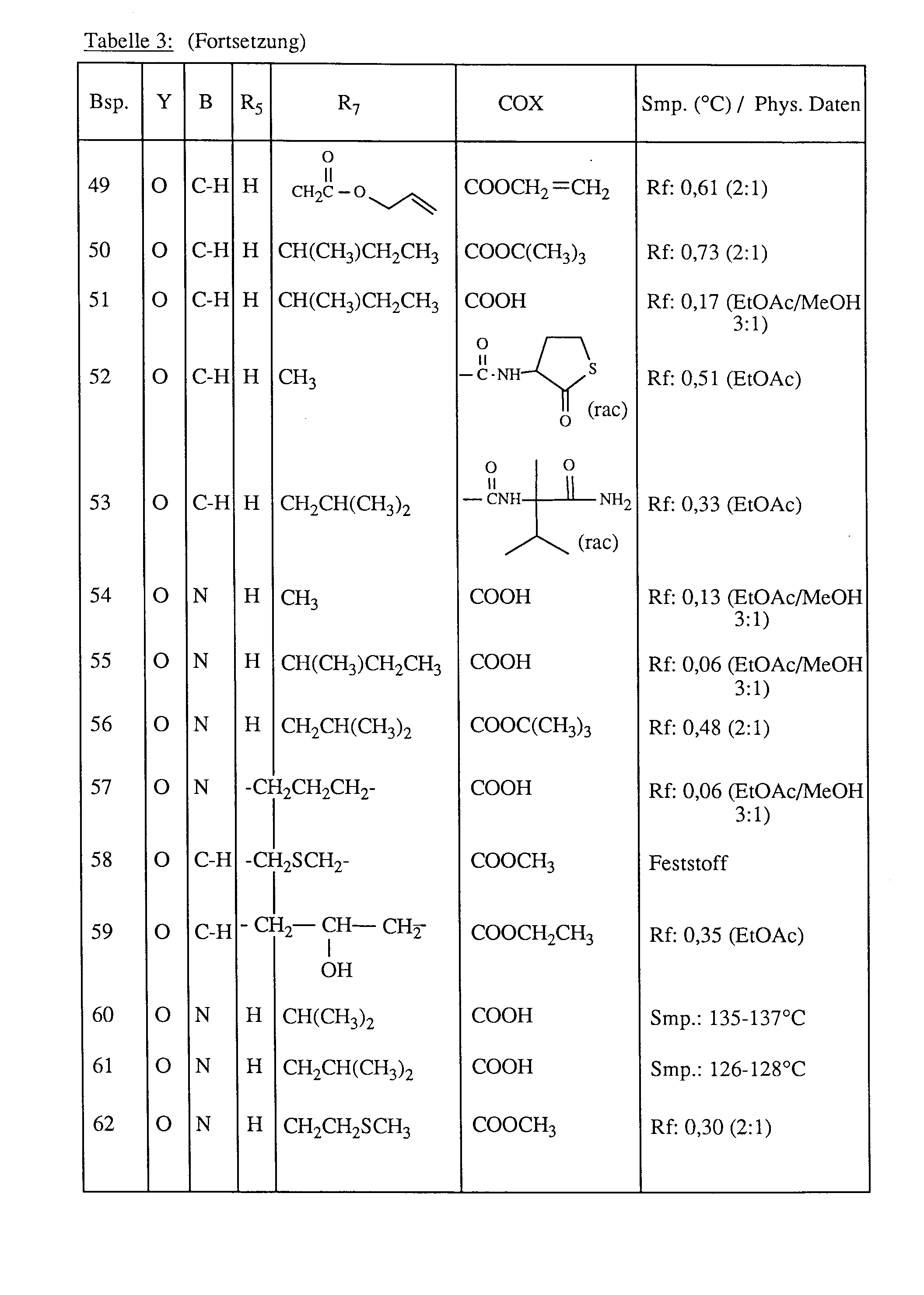

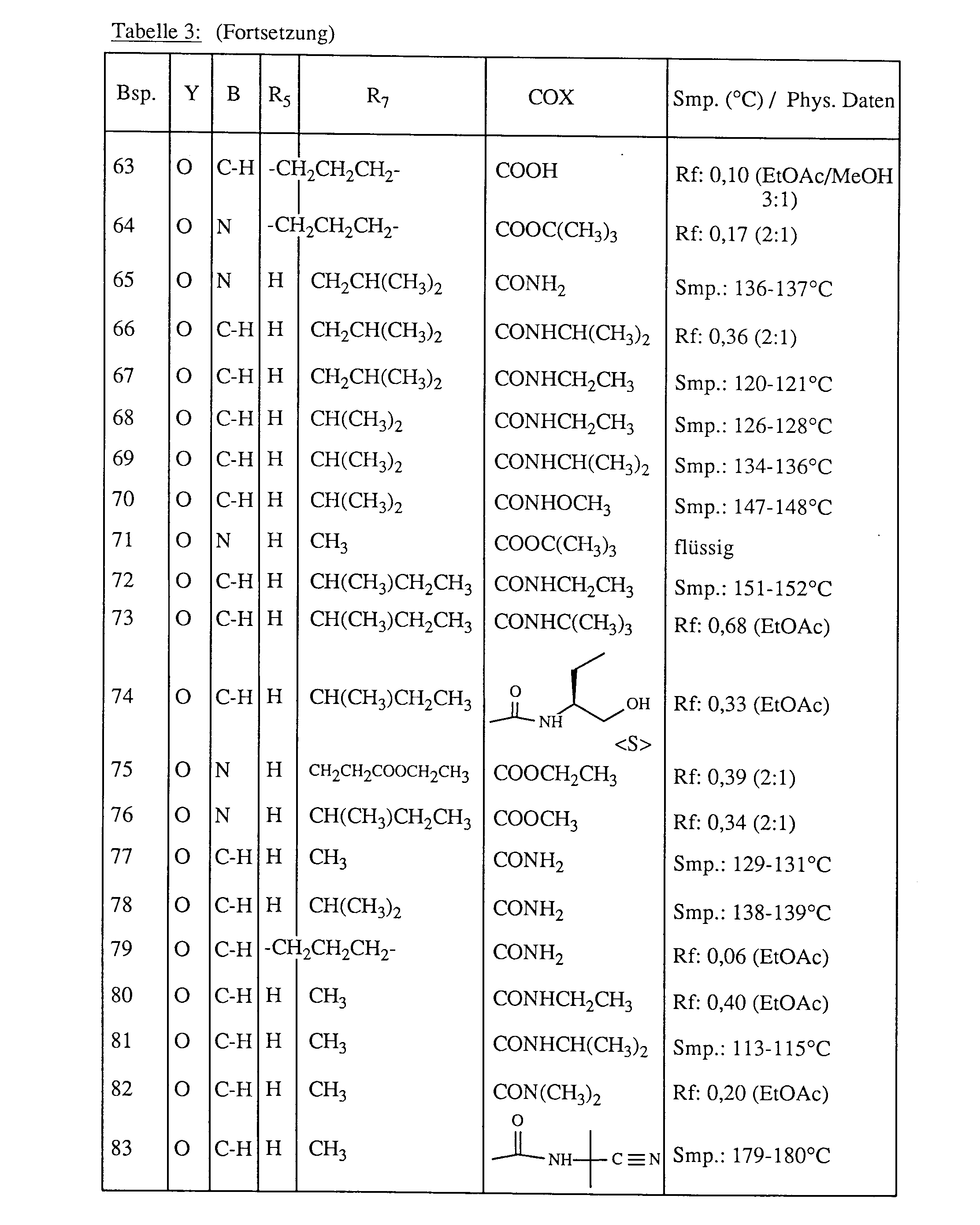

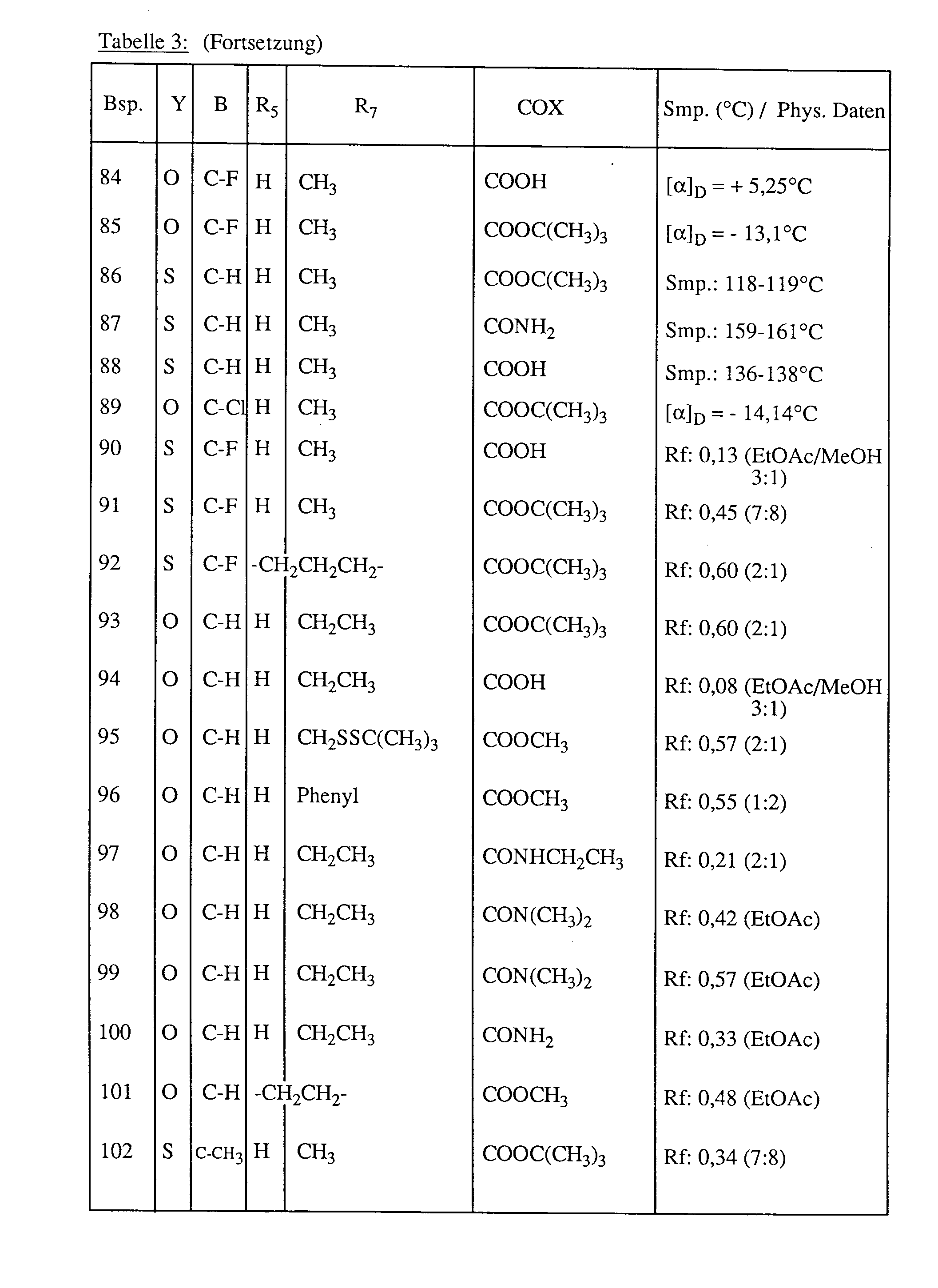

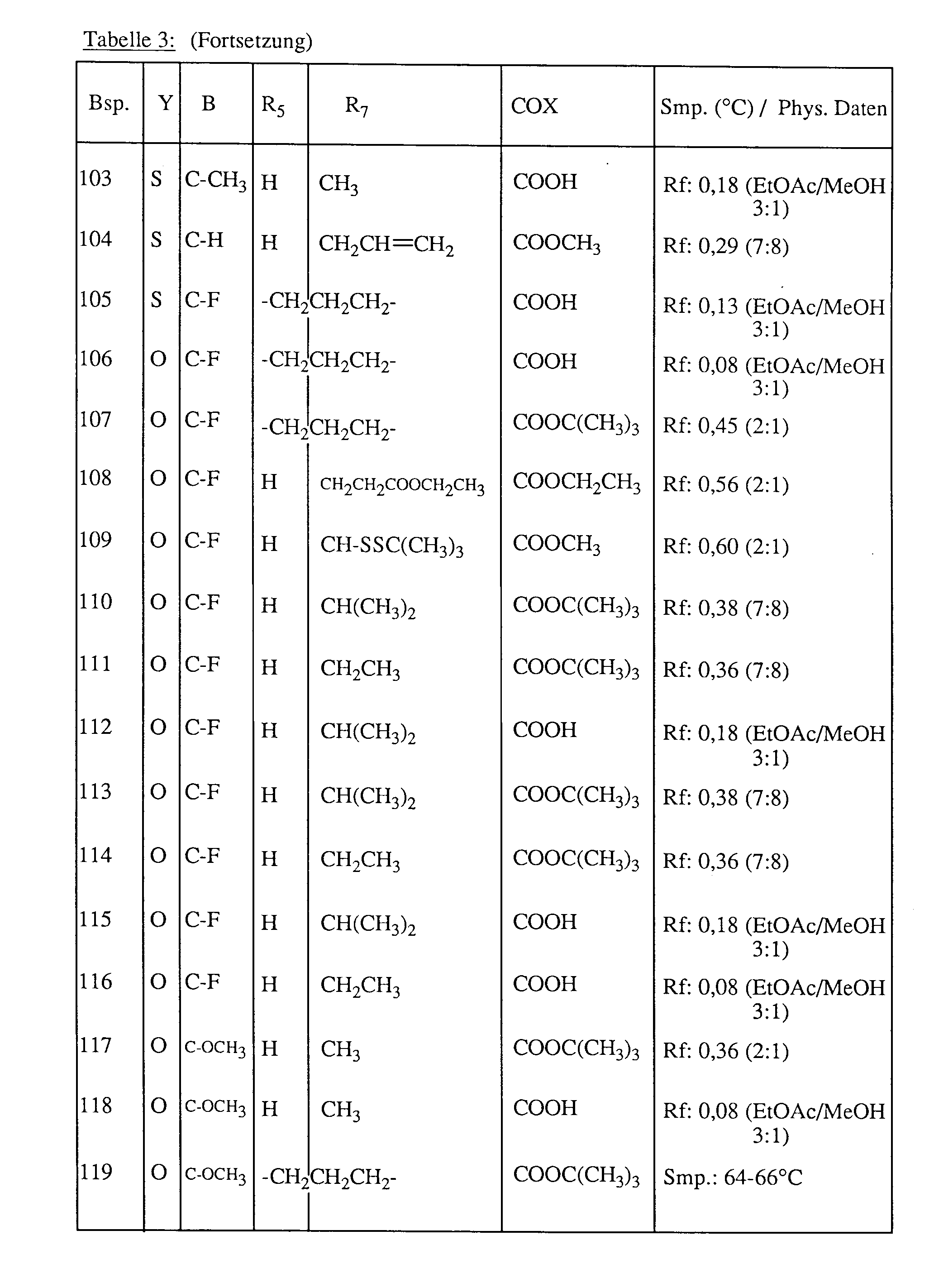

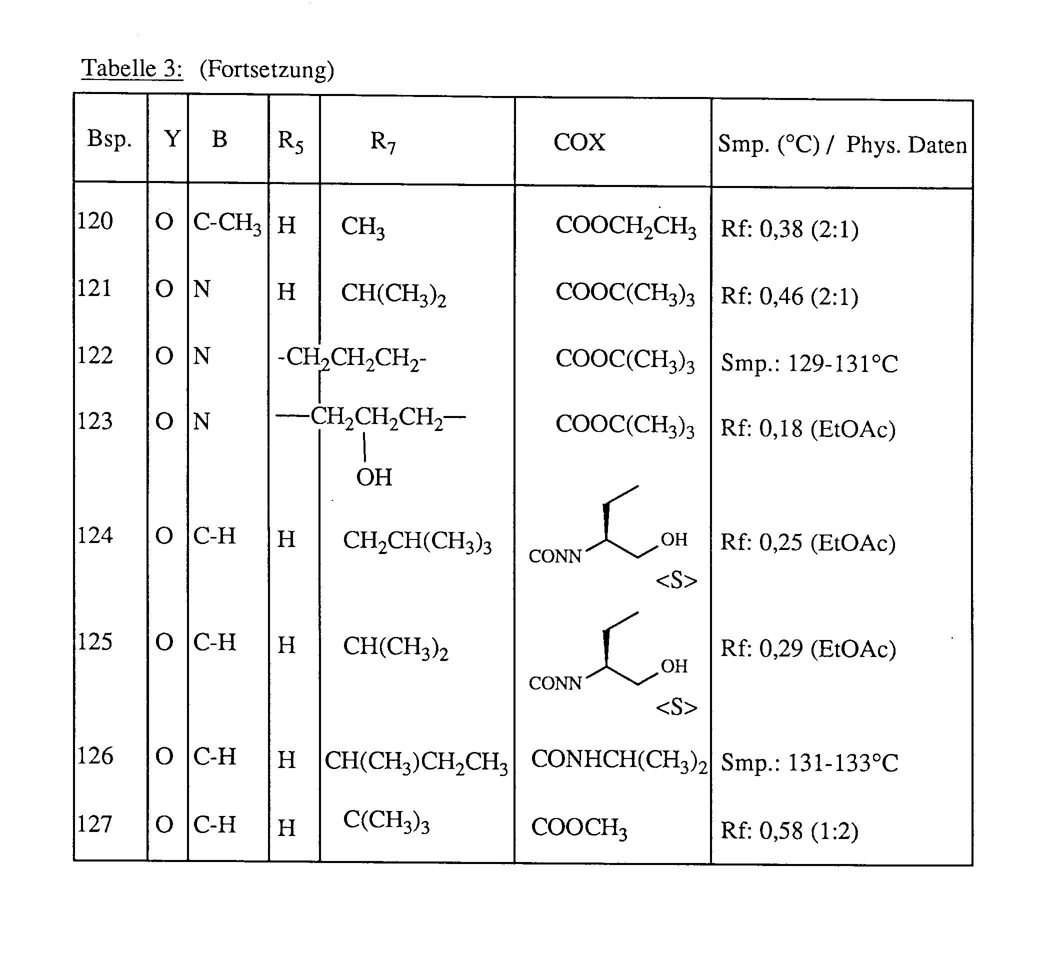

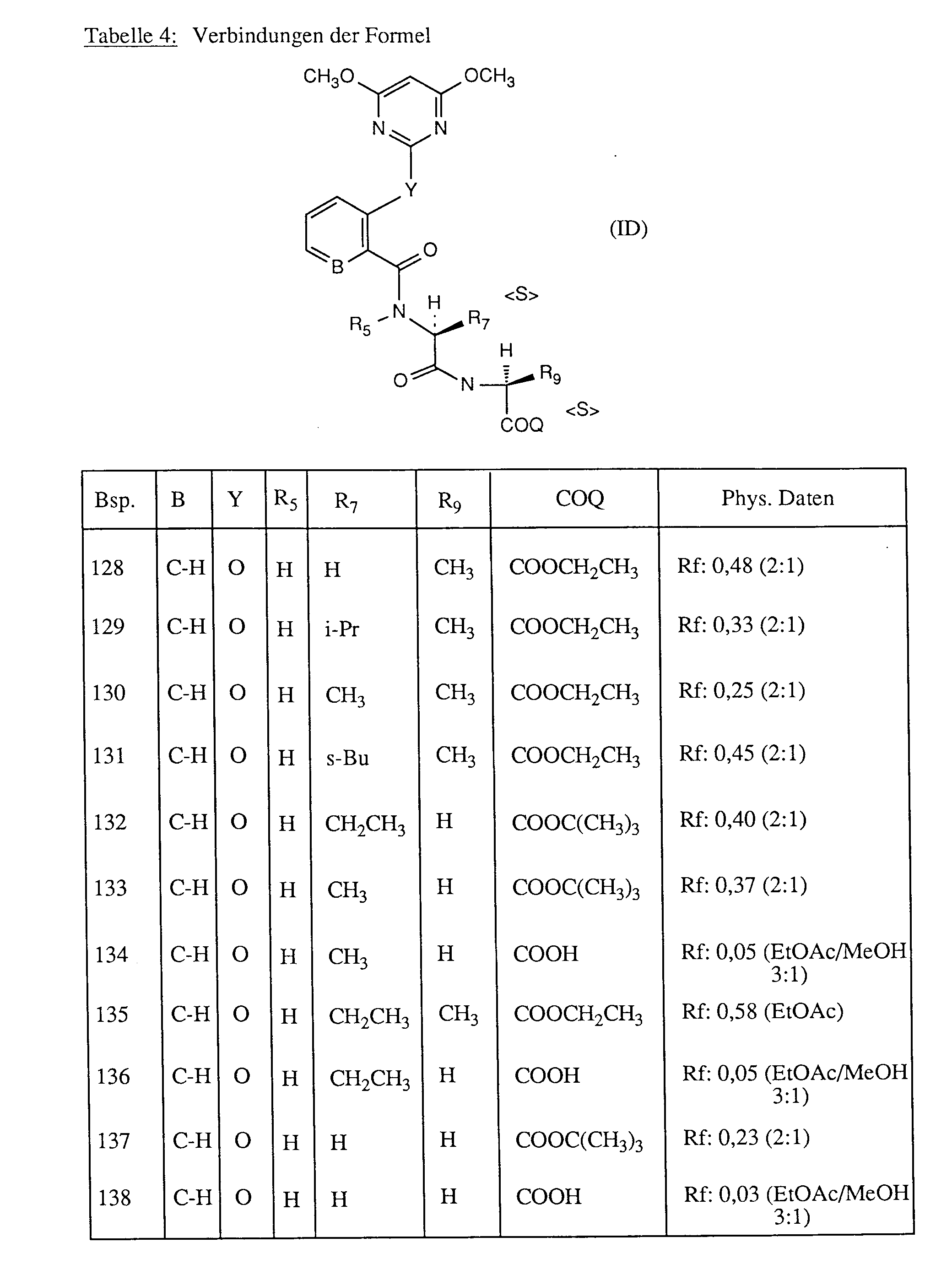

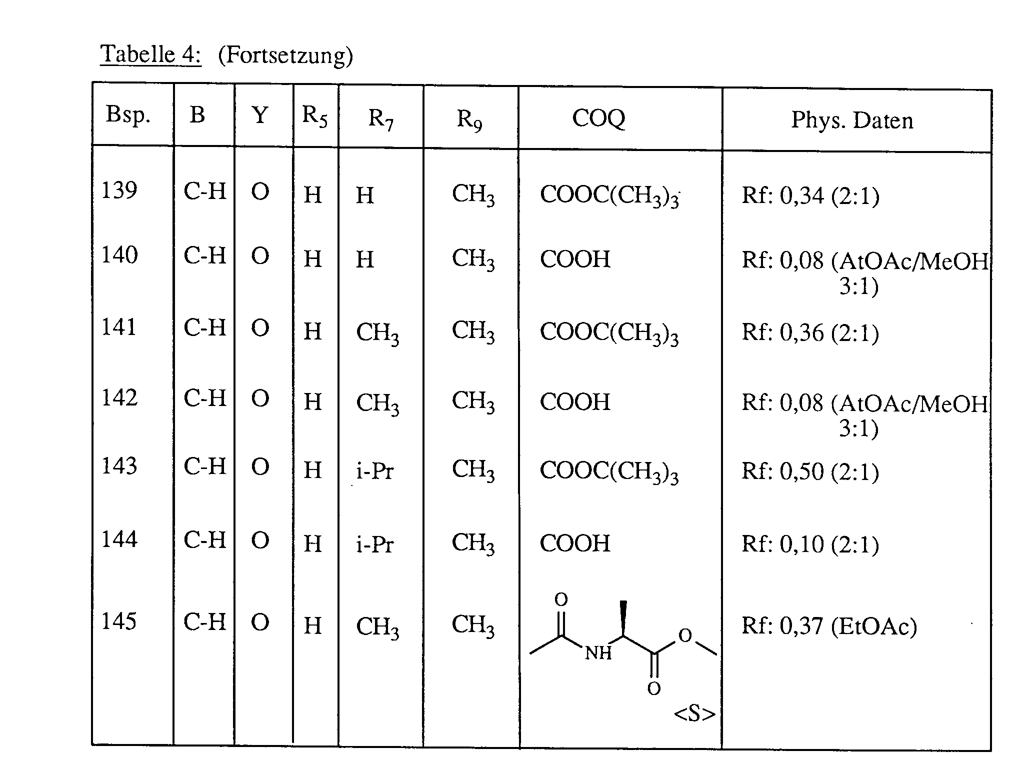

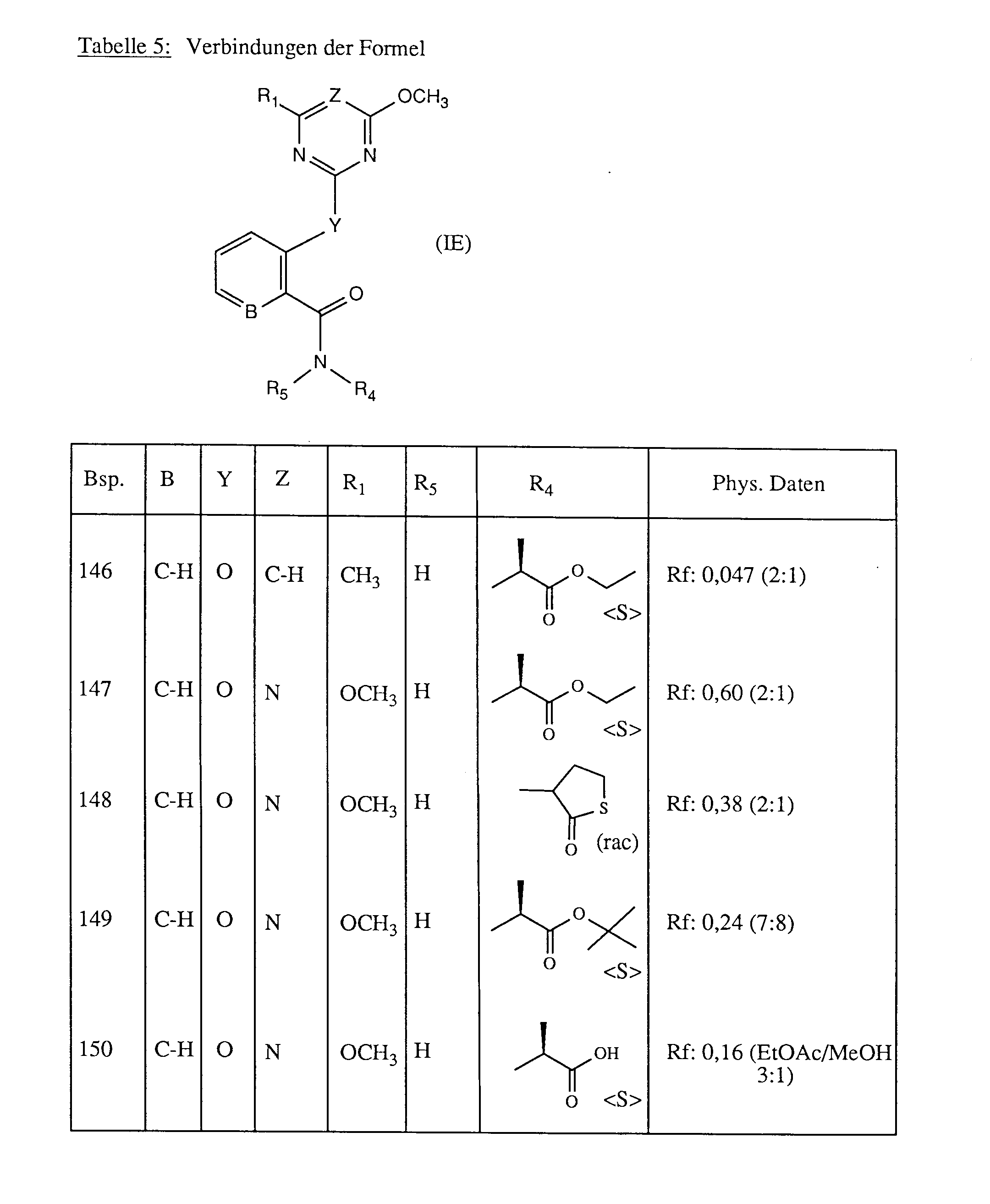

### C. Formulierungsbeispiele

Beispiel F1: Zur Herstellung eines 25%igen Spritzpulvers werden die nachstehend aufgeführten Bestandteile miteinander vermischt:

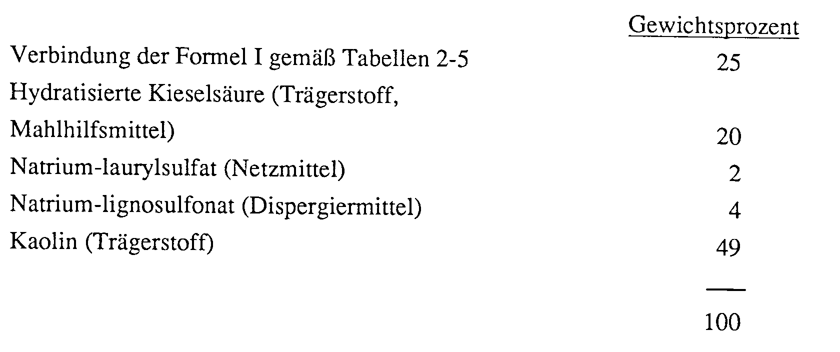

Es wird zuerst der flüssige bzw. geschmolzene Wirkstoff in einer Mahleinrichtung auf die vorgelegte Kieselsäure aufgetragen. Anschließend werden die weiteren Bestandteile zugemischt. Das Gemisch wird unter Verwendung einer Stiftmühle oder einer vergleichbaren Mahleinrichtung fein gemahlen.

Das resultierende Spritzpulver ergibt beim Einrühren in Wasser eine feine Suspension, die sich als gebrauchsfertige Spritzbrühe eignet.

Als Wirkstoff für diese Formulierung eignen sich inbesondere Verbindungen der Formel I, die flüssig sind oder einen niedrigen Schmelzpunkt, d.h. bis etwa +100°C, aufweisen.

Beispiel F2: Verbindungen der Formel I mit hohem Schmelzpunkt. d.h. ab etwa +100°C, können vorzugsweise als Wirkstoffe in konzentrierten Spritzpulvern verwendet werden, beispielsweise wie folgt:

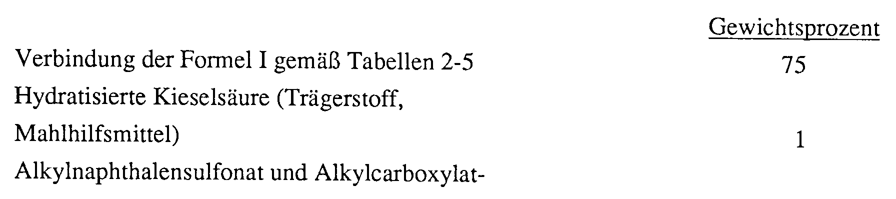

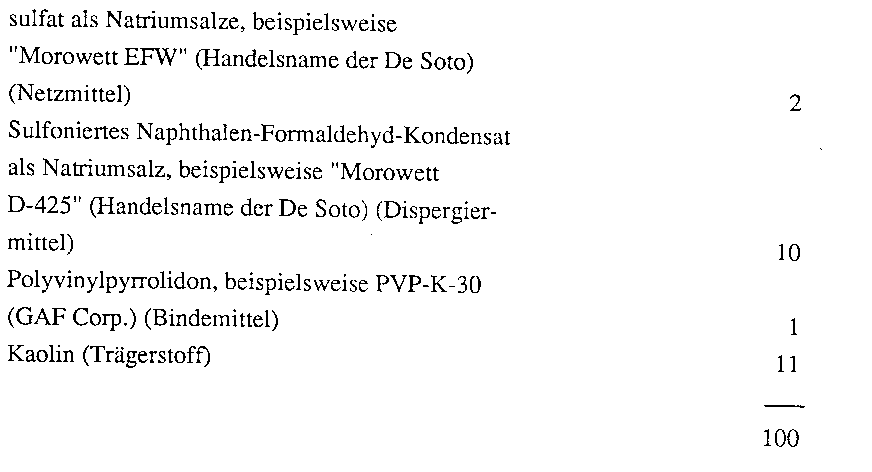

Die Bestandteile werden miteinander gemischt und unter Verwendung eine Stiftmühle oder einer vergleichbaren Mahleinrichtung, insbesondere einer Strahlmühle, fein gemahlen.

Beim Einrühren in Wasser ergibt das resultierende Spritzpulver eine feine Suspension beliebiger Konzentration, die sich als gebrauchsfertige Spritzbrühe eignet.

Beispiel F3: Ein Spritzpulver auf der Basis der Zusammensetzung des Beispiels F2 kann auch in ein dispergierbares Granulat übergeführt werden. Dazu wird das gemahlene Pulver in einer geeigneten Granuliervorrichtung (beispielsweise Granulierteller, Mischtrommel, Intensivmischer oder Wirbelschichtgranulator) mit einer wäßrigen Lösung des Bindemittels besprüht, bis sich Agglomerate gebildet haben. Danach wird das zugefügte Wasser in einem Trocknungsprozeß wieder entfernt. Die Granulate der gewünschten Größe werden ausgesiebt.

Das resultierende Granulat hat gegenüber dem Spritzpulver verschiedene Vorteile (keine Staubbildung bei der Applikation, leichtere Dosierbarkeit infolge besserer Fließeigenschaften). Die Applikation erfolgt nach Einrühren des Präparates in Wasser und nach vollständigem Zerfall der Granulate in die Primärpartikel genau gleich wie beim Spritzpulver.

Beispiel F4: Die Verbindungen der Formel I sind in den üblichen organischen Lösungsmitteln beschränkt löslich. Demzufolge sind emulgierbare Konzentrate von verhältnismäßig niedriger Konzentration möglich; beispielsweise:

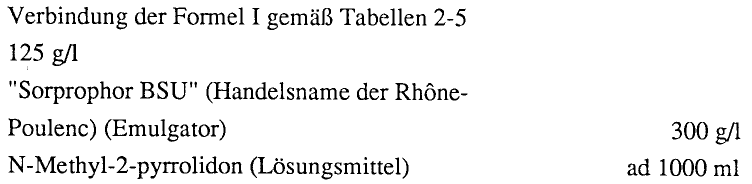

Der Wirkstoff und der Emulgator werden unter Rühren ins Lösungsmittel eingetragen. Das Gemisch wird gerührt, bis eine homogene Lösung entstanden ist.

Das resultierende emulgierbare Konzentrat läßt sich in Wasser emulgieren und ergibt dabei ein gebrauchsfertige Spritzbrühe der gewünschten Konzentration.

Beispiel F5: Verbindungen der Formel I mit einem Schmelzpunkt ab etwa +60°C können auch als sogenannte Suspensionskonzentrate ("Flowables") formuliert werden;
Beispielsweise:

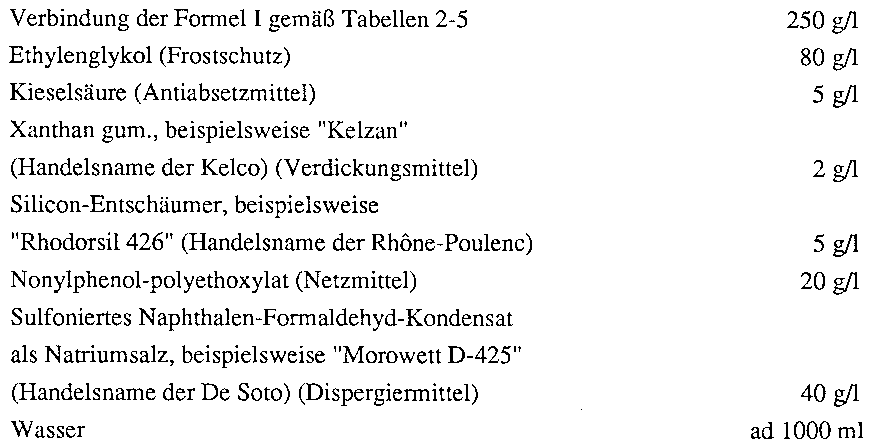

Die Formulierungshilfsstoffe werden in Wasser gelöst. In der Lösung wird der vorgemahlene Wirkstoff unter Rühren dispergiert. Die resultierende grobe Suspension wird nun einer Naßmahlung unterzogen (beispielsweise in einer Kolloid-Mühle oder einer Rührwerkkugelmühle). Gegebenenfalls sind nun noch weitere Zusätze in kleinen Mengen möglich, wie Antischaummittel, Antiabsetzmittel und Biozide.

Das resultierende "Flowable" läßt sich zur Anwendung mit Wasser beliebig verdünnen und ergibt dabei eine gebrauchsfertige Spritzbrühe der gewünschten Konzentration.

### Biologische Beispiele

### Beispiel B1: Pre-emergente Herbizid-Wirkung

Im Gewächshaus wird unmittelbar nach der Einsaat der Versuchspflanzen in Saatschalen die Erdobefläche mit einer wässrigen Spritzbrühe entsprechend einer Aufwandmenge von 3 kg Wirksubstanz/Hektar behandelt. Die Saatschalen werden im Gewächshaus bei 22-25°C und 50-70 % relativer Luftfeuchtigkeit gehalten.

Nach 3 Wochen wird die Herbizidwirkung mit einem dreistufigen (2 = 80-100% Schädigung, 1 = 30-79% Schädigung, 0 = 0-29% Schädigung) Boniturschema im Vergleich in einer unbehandelten Kontrollgruppe bewertet.

In diesem Versuch zeigen die Verbindungen der Formel I gemäß den Tabellen 2-5 starke Herbizidwirkung.

Beispiele für die gute herbizide Wirksamkeit der Verbindungen der Formel I gibt die Tabelle B1:

Tabelle B1: Preemergente Herbizid-Wirkung:

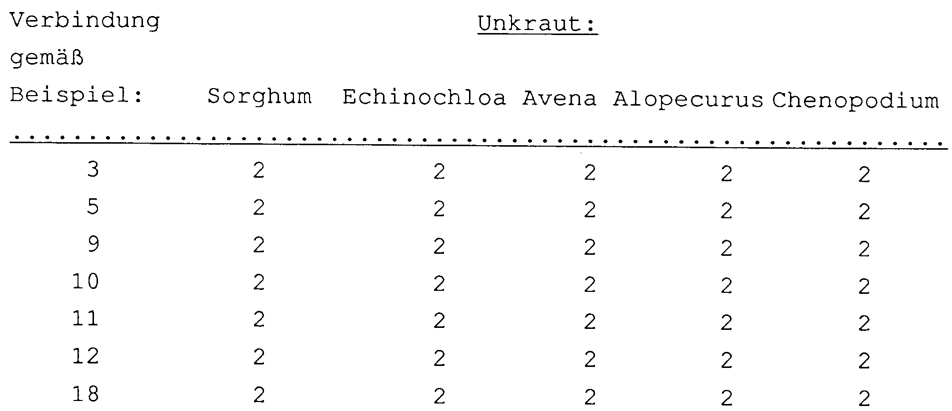

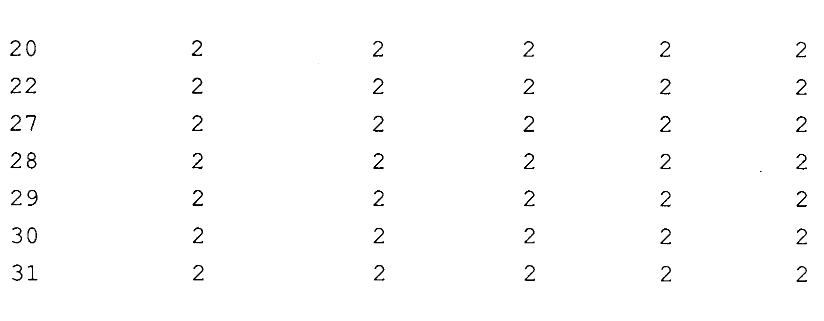

### Beispiel B2: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 3 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch ausgewertet. Auch in diesem Versuch zeigen die Verbindungen der Formel I gemäß den Tabellen 2-5 gute Herbizidwirkung.

### Beispiel B3: Herbizidwirkung für Wasserreis (paddy)

Das Wasserunkraut Echinochloa crus galli wird in Plastikbechern (60 cm² Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat durch eine Spritzung der Prüfsubstanzen auf die Gefäße. Die verwendete Dosis entspricht einer Wirkstoffmenge von 3 kg AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit.

Die Auswertung der Versuche findet 3 Wochen nach Applikation statt. Die Verbindungen der Formel I gemäß den Tabellen 2-5 schädigen dabei die Unkräuter.

### Beispiel B4: Wuchshemmung bei tropischen Bodenbedecker-Leguminosen (cover crops)

Die Versuchspflanzen Centrosema pubescens und Psophocarpus palustris werden in 4 cm-Torftöpfen mit Landerde (45 %), Torf (45 %) und Zonolit (10 %) durch Stecklinge vermehrt. Die Anzucht erfolgt im Gewächshaus bei einer Tagestemperatur von 27°C und einer Nachttemperatur von 23°C. Die Beleuchtungsdauer ist mindestens 14 Stunden/Tag bei einer Intensität von mindestens 7000 Lux.

Ca. 50 Tage nach Ansatz der Stecklinge erfolgt das Vertopfen in 13 cm-Töpfe, 4-5 Pflanzen/Topf. Nach weiteren 60 Tagen werden die Pflanzen auf ca. 15 cm Höhe zurückgeschnitten und appliziert. Dabei werden sie mit 0,1 bis 300 g Wirkstoff/ha (in der Regel 25%-ig formuliert) in wässriger Spritzbrühe besprüht. Die Wasseraufwandmenge beträgt ca. 200 l/ha.

4 Wochen nach der Applikation wird der Neuzuwachs im Gewicht ermittelt und in % zum Durchschnitt der unbehandelten Kontrollen dargestellt. Die nekrotischen Schäden sind in % zur gesamten Blattfläche aufgeführt.

Der Neuzuwachs der behandelten Pflanzen erweist sich als deutlich geringer als der der unbehandelten Kontrollen.

### Beispiel B5: Wuchsregulierung an Sojabohnen

Versuchspflanzen der Sorte Williams werden in 11 cm-Tontöpfen mit Landerde (45 %), Torf (45 %) und Zonolit (10 %) angesät und in der Klimakammer bei einer Tagestemperatur von 24°C und einer Nachttemperatur von 19°C angezogen. Die Beleuchtungsdauer ist 16 Stunden pro Tage bei einer Intensität von ca. 350 Mikro-Einstein.

Ca. 24 Tage nach der Saat erfolgt das Umtopfen in 18 cm-Töpfe, 2 Pflanzen/Topf. Nach weiteren 12 Tagen und im Stadium von 5-6 Trifolia-Blätter findet die Applikation mit 0,1 bis 300 g Wirkstoff/ha statt, in der Regel 25%-ig formuliert und in wässriger Spritzbrühe. Die Wasseraufwandmenge beträgt ca. 200 l/ha.

Ca. 4 Wochen nach der Applikation findet die Auswertung statt. Hierbei wird die Höhe des Neuzuwachses gemessen und in % zum Durchschnitt der unbehandelten Kontrollen dargestellt. Die nekrotischen Schäden sind in % zur gesamten Blattfläche aufgeführt.

Die behandelten Pflanzen zeigen einen deutlich geringeren Neuzuwachs als die unbehandelten Kontrollen.

### Beispiel B6: Wuchshemmunp bei Getreide

Versuchspflanzen (Sommergerste der Sorte Iban) werden in 15 cm-Kunststofftöpfen mit steriler Landerde angesät und in der Klimakammer bei einer Tagestemperatur von 10- 15°C und einer Nachttemperatur von 5-10°C angezogen. Die Beleuchtungsdauer ist 13.5 Stunden pro Tag bei einer Intensität von ca. 25000 Lux.

Ca. 34 Tage nach der Saat und dem Ausdünnen auf 4 Pflanzen/Topf erfolgt die Applikation mit 0,1 bis 300 g Wirkstoff/ha, in der Regel 25%-ig formuliert und in wässriger Spritzbrühe. Die Wasseraufwandmenge ist ca. 500 l/ha. Nach der Applikation werden die Pflanzen im Gewächshaus bei einer Tagestemperatur von mindestens 10°C aufgestellt. Die Beleuchtungsdauer ist mind. 13.5 Stunden/Tag.

Ca. 28 Tage nach der Behandlung findet die Auswertung statt. Hierbei wird die Höhe des Neuzuwachses in % zum Durchschnitt der unbehandelten Kontrollen dargestellt. Die nekrotischen Schäden sind in % zur gesamten Blatfläche aufgeführt.

Die behandelten Pflanzen weisen im Vergleich zu unbehandelten Kontrollen eine Verringerung des Neuzuwachsese auf.

### Beispiel B7: Wuchshemmung bei Gräsern

Eine Mischung von Gräsern (z.B. Poa, Festuca, Lolium, Bromus, Cynosurus) und Klee (Trifolium pratense/repens) wird in 15 cm-Kunststofftöpfen mit steriler Landerde angesät und im Gewächshaus bei einer Tagestemperatur von 21 °C und einer Nachttemperatur von 17°C angezogen. Die Beleuchtungsdauer ist 13.5 Stunden/Tag bei einer Lichtintensität von mind. 7000 Lux. Nach dem Auflauf werden die Pflanzen wöchentlich auf ca. 6 cm Höhe zurückgeschnitten. Ca. 42 Tage nach der Saat und 1 Tag nach dem letzten Schnitt erfolgt die Applikation mit 0,1 bis 300 g Wirkstoff/ha, in der Regel 25%-ig formuliert und in wässriger Spritzbrühe. Die Wasseraufwand- menge ist ca. 500 l/ha.

Ca. 3 Wochen nach der Behandlung findet die Auswertung statt. Hierbei wird die Höhe des Neuzuwachses gemessen und in % zum Durchschnitt der unbehandelten Kontrollen dargestellt. Die nekrotischen Schäden sind in % zur gesamten Blattfläche aufgeführt.

Die geprüften Verbindungen der Formel I gemäß den Tabellen 2-5 bewirken eine Reduzierung des Neuzuwachses im Vergleich zur unbehandelten Kontrolle.

## Patentansprüche

1. Pyrimidinyl- und Triazinyl- salicylamide der Formel I 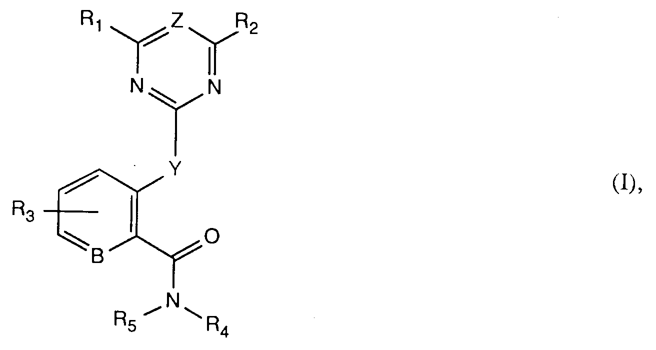 worin
B für Stickstoff, Methin, oder mit Fluor, Chlor, Methyl, Methoxy, 2-Propenyloxy, 2-Propinyloxy, Difluormethoxy, Phenoxy, Methylthio, Phenylthio, Phenyl oder Benzyloxy substituiertes Methin;
Y für Sauerstoff oder Schwefel;
Z für Methin oder Stickstoff;
R₁ für Chlor, Methyl, Methoxy, Ethoxy, Difluormethoxy, Methylamino, Ethylamino oder Dimethylamino;
R₂ für Methyl, Methoxy oder Difluormethoxy;
R₃ für Wasserstoff, Fluor, Chlor, Methyl oder Methoxy;
R₄ für eine der Gruppen (a), (b) oder (c) 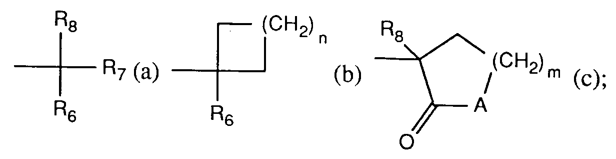 R₅ für Wasserstoff, Methyl oder zusammen mit R₇ -(CH₂)ₚ-, -CH₂SCH₂ oder -CH₂CHOHCH₂-;
R₆ für Hydroxymethyl, Formyl, Cyano, Hydroxyimino, C₁₋₄-Alkoxyimino, Phosphono, Phosphino, Methylphosphino oder eine Gruppe COX;
R₇ für Wasserstoff; C₁₋₄-Alkyl oder durch Hydroxy, C₁₋₄-Alkoxy, Mercapto, Acylmercapto, C₁₋₄-Alkylthio, Vinyl, Phenyl, 4-Hydroxyphenyl, 4-Imidazolyl, 3-Indolyl, Hydroxycarbonyl, C₁₋₄-Alkoxycarbonyl, 2-Propenyloxycarbonyl, Cyano, Carbamoyl, Methylphosphino oder Methylsulfoximino substituiertes C₁₋₄-Alkyl; Trifluormethyl; Ethinyl; Vinyl oder durch Chlor oder Methoxy substituiertes Vinyl; Phenyl oder durch Fluor, Chlor, Methyl, Trifluormethyl oder Methoxy substituiertes Phenyl; oder Cyano oder C₁₋₄-Alkoxycarbonyl;
R₈ für Wasserstoff oder Methyl;
A für Sauerstoff, Schwefel oder -NH-;
m für 1, 2 oder 3;
n für 0, 1, 2 oder 3;
p für 2 oder 3;
X für Hydroxy, C₁₋₄-Alkoxy, C₃₋₄-Alkenyloxy, Mercapto, C₁₋₄-Alkylthio, Amino, C₁₋₄-Alkylamino, C₂₋₄Dialkylamino, C₁₋₄-Alkoxyamino, oder durch Phenyl, Benzyloxy oder C₁₋₂-Alkoxy substituiertes C₁₋₄-Alkoxy, C₃₋₄-Alkenyloxy, Mercapto, C₁₋₄-Alkylthio, Amino, C₁₋₄-Alkylamino, C₂₋₄Dialkylamino, C₁₋₄-Alkoxyamino, oder eine der Gruppen (d), (e) oder f) 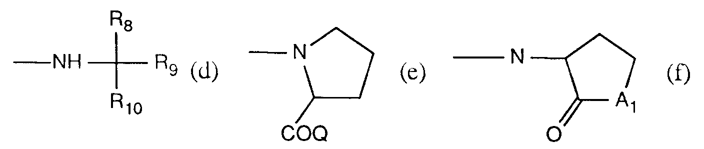 A₁ für Sauerstoff, Schwefel oder -NH-;
R₉ für Wasserstoff, C₁₋₄-Alkyl oder Benzyl;
R₁₀ für Hydroxymethyl, Cyano oder eine Gruppe COQ′;
Q für Hydroxy, C₁₋₄-Alkoxy, 2-Propenyloxy, Benzyloxy, Amino oder die Gruppe d); und
Q′ für Hydroxy, C₁₋₄-Alkoxy, 2-Propenyloxy, Benzyloxy, Amino oder die Gruppe d) stehen; sowie die Salze derjenigen Verbindungen der Formel I, welche eine freie Hydroxycarbonylgruppe aufweisen.

2. Verbindungen der Formel I nach Anspruch 1, dadurch gekennzeichnet, daß
B für Stickstoff, Methin, oder mit Fluor, Chlor, Methyl, Methoxy, 2-Propenyloxy, 2-Propinyloxy, Difluormethoxy oder Benzyloxy substituiertes Methin;
Y für Sauerstoff oder Schwefel;
Z für Methin oder Stickstoff;
R₁ für Chlor, Methyl, Methoxy, Ethoxy, Difluormethoxy, Methylamino, Ethylamino oder Dimethylamino;
R₂ für Methyl, Methoxy oder Difluormethoxy;
R₃ für Wasserstoff, Fluor, Chlor, Methyl oder Methoxy;
R₄ für eine der Gruppen (a), (b) oder (c) 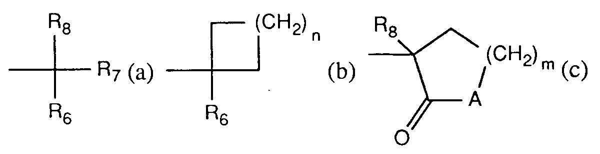 R₅ für Wasserstoff, Methyl oder zusammen mit R₇ -(CH₂)ₚ- oder -CH₂CHOHCH₂-;
R₆ für Hydroxymethyl, Cyano, Phosphono, Phosphino, Methylphosphino oder eine Gruppe COX;
R₇ für Wasserstoff; gegebenenfalls mit Hydroxy, C₁₋₄-Alkoxy, Mercapto, Acylmercapto, C₁₋₄-Alkylthio, Phenyl, 4-Hydroxyphenyl, 4-Imidazolyl, 3-Indolyl, Hydroxycarbonyl, C₁₋₄-Alkoxycarbonyl, 2-Propenyloxycarbonyl, Cyano, Carbamoyl, Methylphosphino oder Methylsulfoximino substituiertes C₁₋₄-Alkyl; Trifluormethyl; Ethinyl; Vinyl oder durch Chlor oder Methoxy substituiertes Vinyl; Phenyl oder durch Fluor, Chlor, Methyl, Trifluormethyl oder Methoxy substituiertes Phenyl;
R₈ für Wasserstoff oder Methyl;
A für Sauerstoff, Schwefel oder -NH-;
m für 1, 2 oder 3;
n für 0, 1, 2 oder 3;
p für 2 oder 3;
X für Hydroxy, C₁₋₄-Alkoxy, C₃₋₄-Alkenyloxy, Mercapto, C₁₋₄-Alkylthio, Amino, C₁₋₄-Alkylamino, C₂₋₄Dialkylamino, oder eine weitere der Gruppen (d) oder (e) 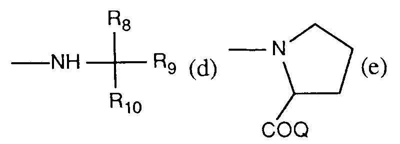 R₉ für Wasserstoff, C₁₋₄-Alkyl oder Benzyl;
R₁₀ für COQ′;
Q für Hydroxy, C₁₋₄-Alkoxy, 2-Propenyloxy, Benzyloxy oder Amino; Q′ für Hydroxy, C₁₋₄-Alkoxy, 2-Propenyloxy, Benzyloxy oder Amino; sowie die Salze von Verbindungen der Formel I, welche eine freie Hydroxycarbonylgruppe aufweisen.

3. Verbindungen der Formel I nach Anspruch 1, dadurch gekennzeichnet, daß B Methin oder Stickstoff bedeutet.

4. Verbindungen der formel I nach Anspruch 3, dadurch gekennzeichnet, daß das Methin durch Fluor oder Chlor substituiert ist.

5. Verbindungen der Formel I nach Anspruch 1, dadurch gekennzeichnet, daß Y Sauerstoff bedeutet.

6. Verbindungen der Formel I nach Anspruch 1, dadurch gekennzeichnet, daß Z Methin bedeutet.

7. Verbindungen der Formel I nach Anspruch 1, dadurch gekennzeichnet, daß R₁ und R₂ Methoxy bedeuten.

8. Verbindungen der Formel I nach Anspruch 1, dadurch gekennzeichnet, daß R₃ Wasserstoff bedeutet.

9. Verbindungen der Formel I nach Anspruch 1, dadurch gekennzeichnet, daß R₄ eine Gruppe (a) bedeutet.

10. Verbindungen der Formel I nach Anspruch 1, dadurch gekennzeichnet, daß R₅ Wasserstoff bedeutet.

11. Verbindungen der Formel I nach Anspruch 1, dadurch gekennzeichnet, daß R⁶ Cyano oder eine COX-Gruppe bedeutet.

12. Verbindungen der Formel I nach Anspruch 11, dadurch gekennzeichnet, daß X C₁₋₄-Alkoxy oder Amino bedeutet.

13. Verbindungen der Formel I nach Anspruch 1, dadurch gekennzeichnet, daß R₇ Wasserstoff, C₁₋₄-Alkyl, 2-Propenyl oder zusammen mit R₅ -CH₂CH₂CH₂- oder -CH₂SCH₂- bedeutet.

14. Verbindungen der Formel I nach Anspruch 1, dadurch gekennzeichnet, daß R₈ Wasserstoff bedeutet.

15. Verbindungen der Formel I nach Anspruch 1, dadurch gekennzeichnet, daß R₄ eine chirale Gruppe der formel IA 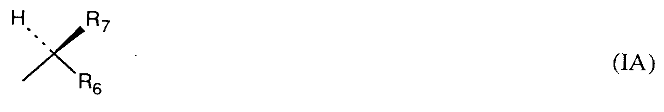 mit der Chiralität <S> bedeutet.

16. Verbindungen der Formel I nach Anspruch 1, dadurch gekennzeichnet, daß R₄ für die Gruppe (c) steht, worin A Sauerstoff oder Schwefel und m = 1 bedeutet.

17. Verbindungen der Formel I nach Anspruch 16, dadurch gekennzeichnet, daß R₄ eine Gruppe der Formel IF 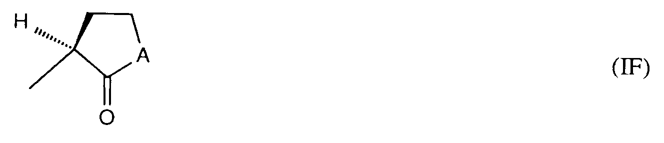 mit der Chiralität <S> bedeutet.

18. Verbindungen der Formel I nach Anspruch 1, dadurch gekennzeichnet, daß
R¹ für Methoxy, Ethoxy, oder Difluormethoxy;
R² für Methoxy;
R³ für Wasserstoff;
B für Stickstoff, Methin, oder durch Chlor oder Fluor substituiertes Methin;
Y für Sauerstoff oder Schwefel;
Z für Methin; und
NR⁴R⁵ eine der Gruppen 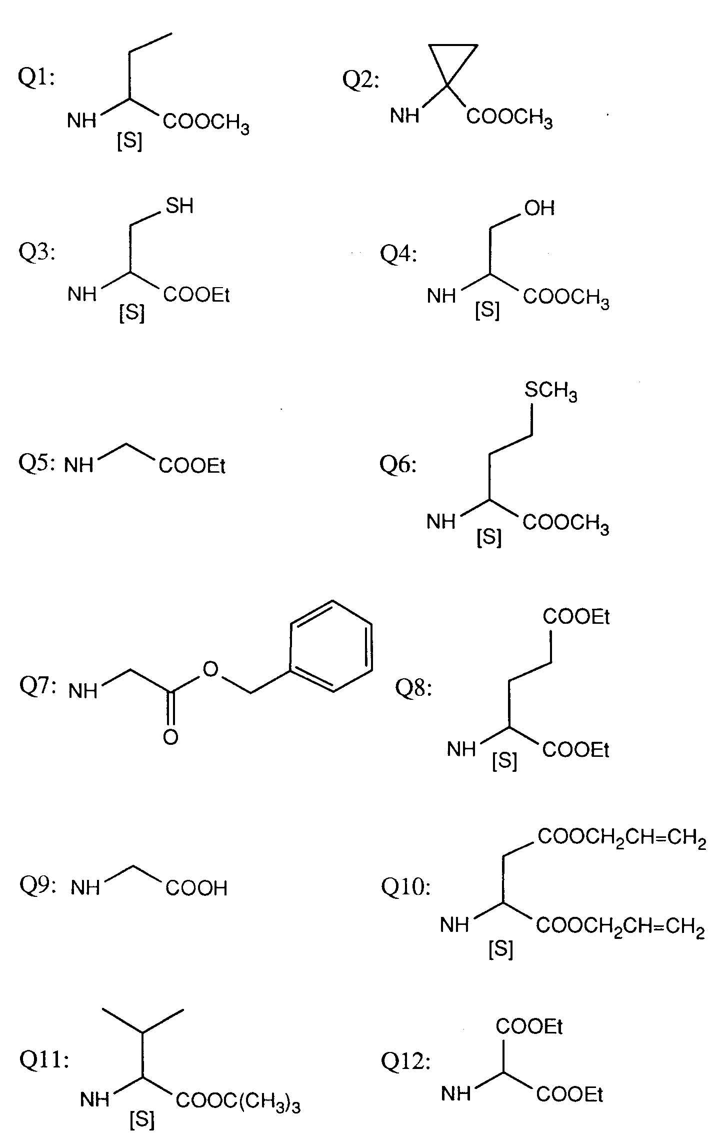 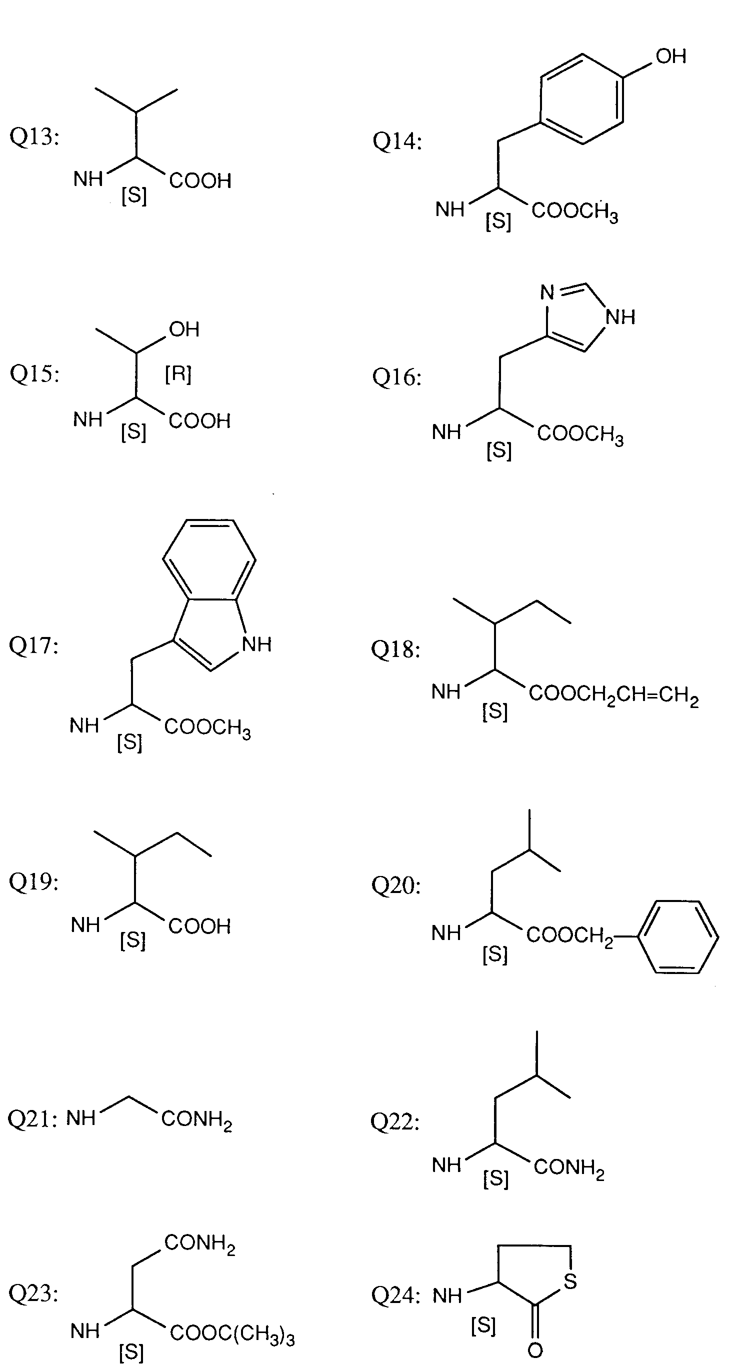 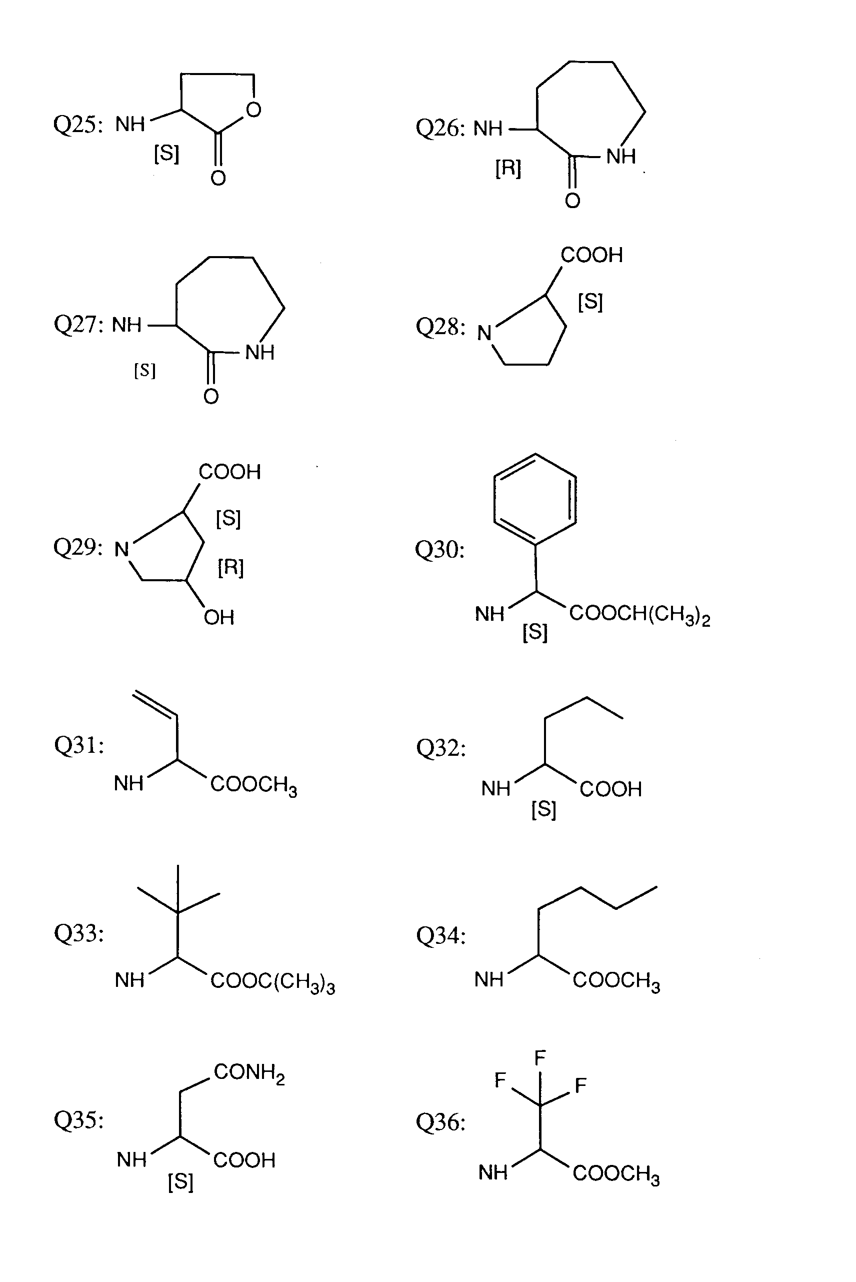 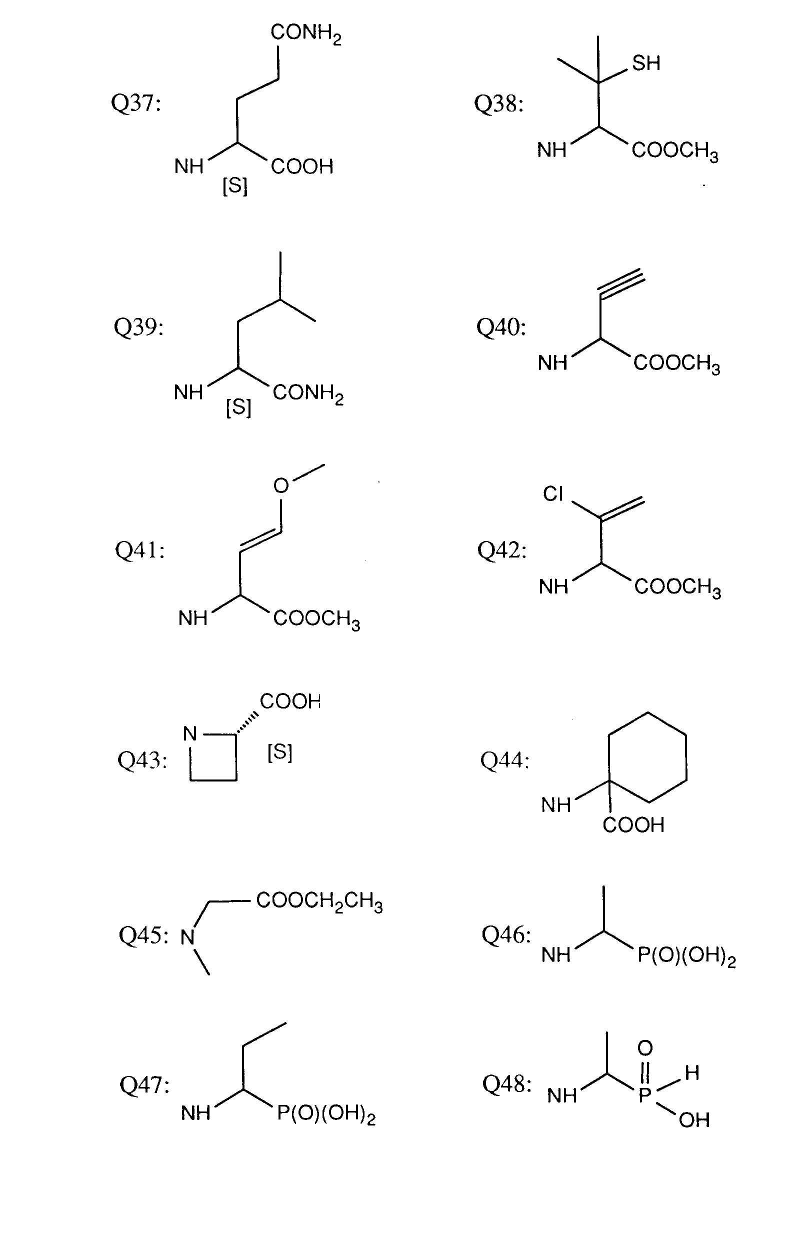 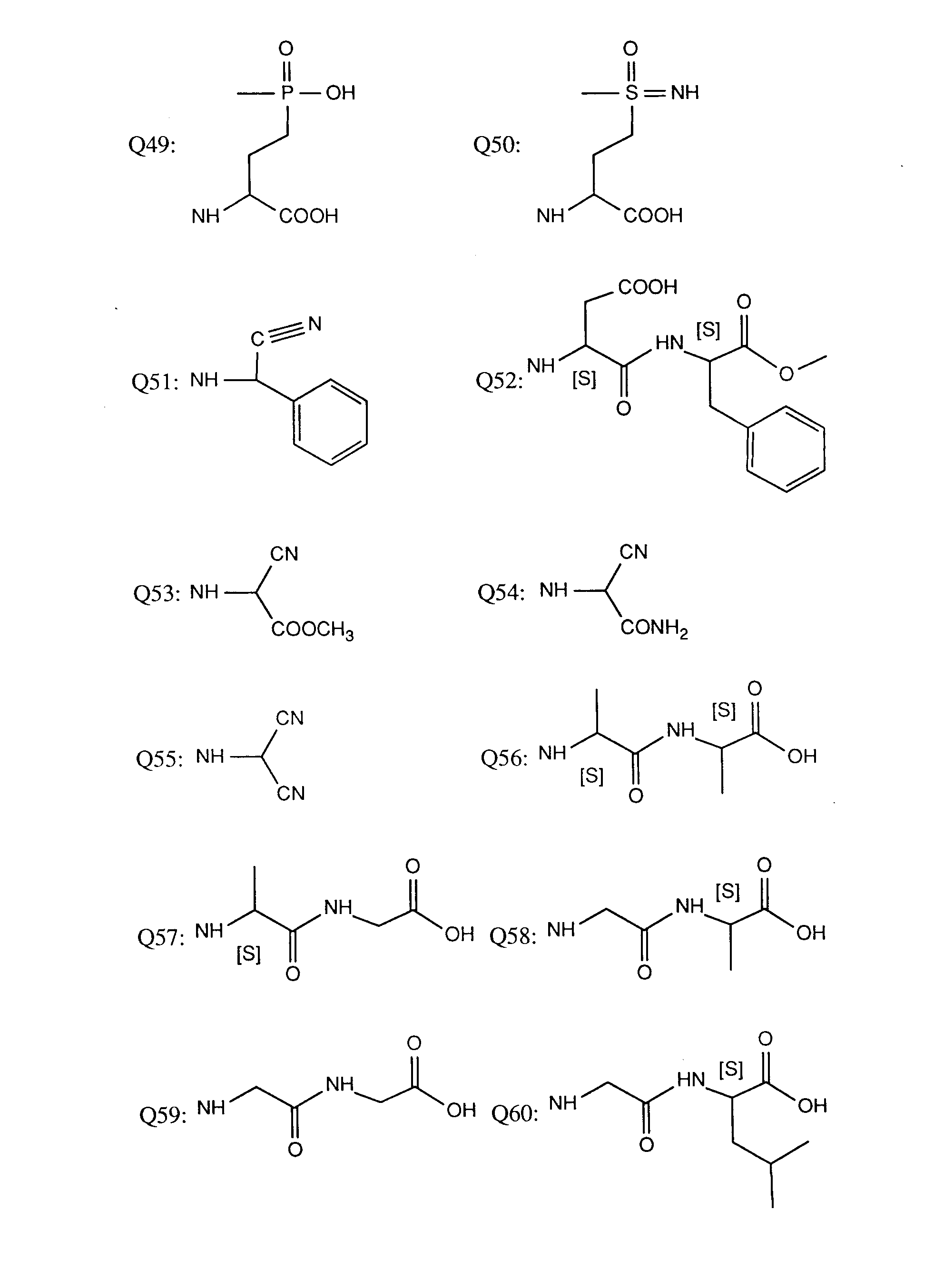 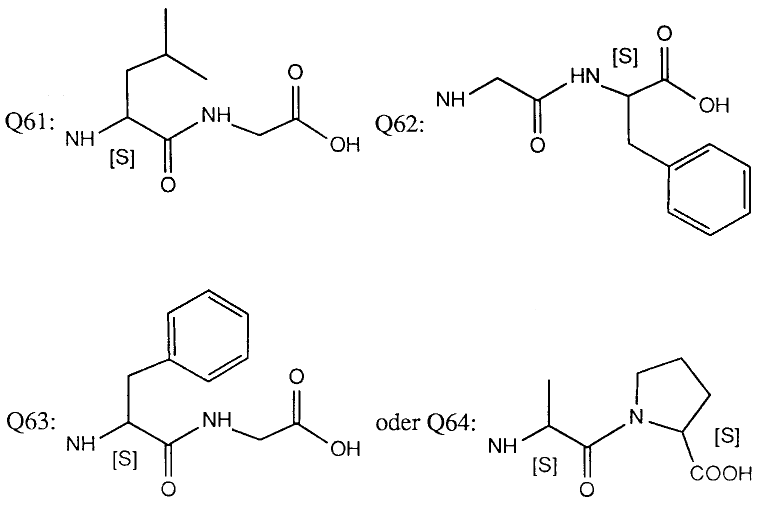 bedeuten.

19. Verbindungen der Formel I nach Anspruch 1, ausgewählt aus der Gruppe
N-(o-((4,6-Dimethoxy-2-pyrimidinyl)oxy)benzoyl)glycinmethylester;
N-(o-((4,6-Dimethoxy-2-pyrimidinyl)oxy)benzoyl)-L-alaninethylester;
rac-o-((4,6-Dimethoxy-2-pyrimidinyl)oxy)-N-(dihydro-2-oxo-3(2H)-thienyl)benzamid;
N-(o-((4,6-Dimethoxy-2-pyrimidinyl)oxy)benzoyl)-L-alanin-methylester;
N-(o-((4,6-Dimethoxy-2-pyrimidinyl)oxy)benzoyl)-L-valinmethylester;
N-(o-((4,6-Dimethoxy-2-pyrimidinyl)oxy)benzoyl)-L-leucinmethylester;
rac-N-(1-Carbamoyl-1,2-dimethylpropyl)-o-((4,6-dimethoxy-2-pyrilmidinyl)-thio)-benzamid;
1-(o-((4,6-Dimethoxy-2-pyrimidinyl)oxy)benzoyl)-L-prolin-tert-butylester;
N-((3-((4,6-Dimethoxy-2-pyrimidinyl)oxy)-2-pyridyl)-carbonyl)-L-alaninmethylester;
N-((3-((4,6-Dimethoxy-2-pyrimidinyl)oxy)-2-pyridyl)-carbonyl)-glycinmethylester;
o-((4,6-Dimethoxy-2-pyrimidinyl)oxy)-N-(<S>-1-(hydroxymethyl)2-methylpropyl)benzamid; und
N-(1-Cyano-1-methylethyl)-o-((4,6-dimethoxy-2-pyrimidinyl)oxy)benzamid.

20. Verfahren zur Herstellung von Verbindungen der Formel I gemäß Anspruch 1, dadurch gekennzeichnet, daß man eine Verbindung der Formel II 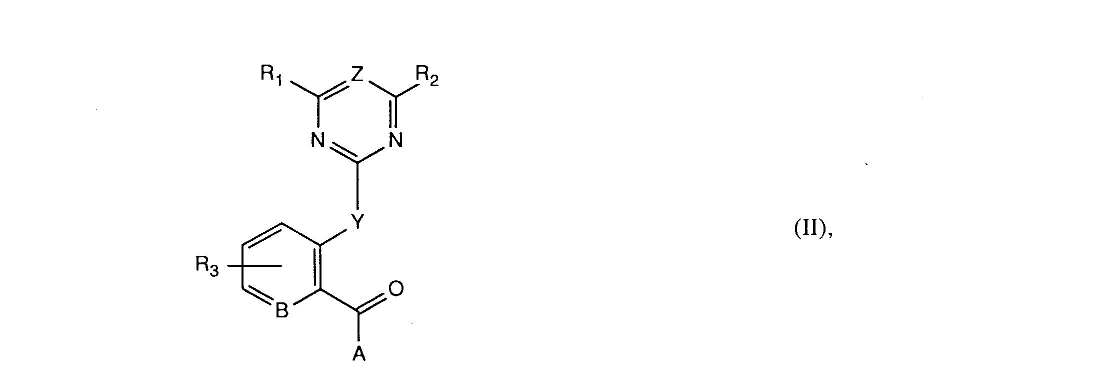 worin A eine Abgangsgruppe bedeutet;
oder ein Säureanhydrid der Formel II′ 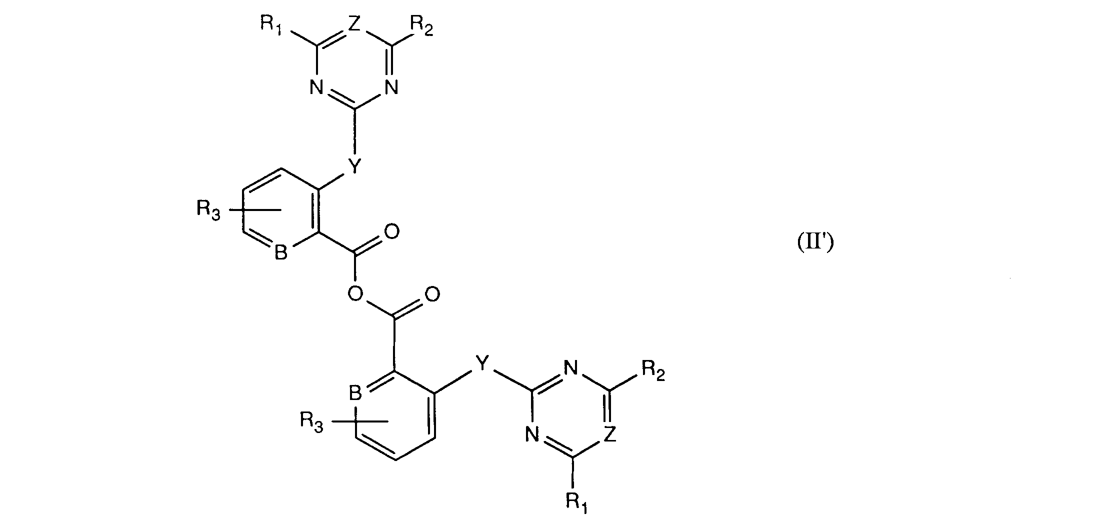 worin R₁, R₂, R₃, Y, Z und B die unter Formel I in Anspruch 1 angegebene Bedeutung haben
mit einem Amin der Formel III 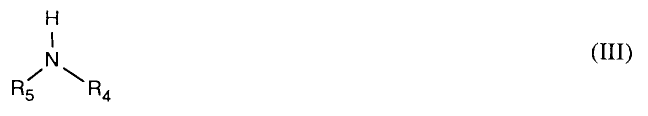 worin R₄ und R₅ die unter Formel I in Anspruch 1 angegebene Bedeutung haben, umsetzt.

21. Verfahren nach Anspruch 20, dadurch gekennzeichnet, daß man die Umsetzung in Gegenwart einer Base durchführt.

22. Verfahren nach Anspruch 20, dadurch gekennzeichnet, daß man die Umsetzung in Gegenwart eines Lösungsmittels durchführt.

23. Verfahren zur Herstellung von Verbindungen der Formel I gemäß Anspruch 1, dadurch gekennzeichnet, daß man eine Verbindung der Formel V 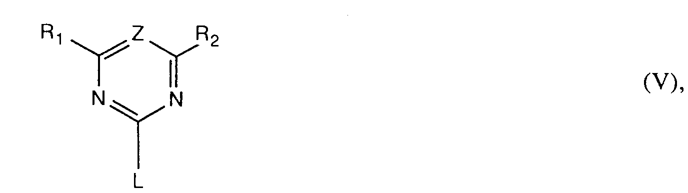 worin L eine Abgangsgruppe bedeutet,
mit einem Salicylamidderivat der Formel VII 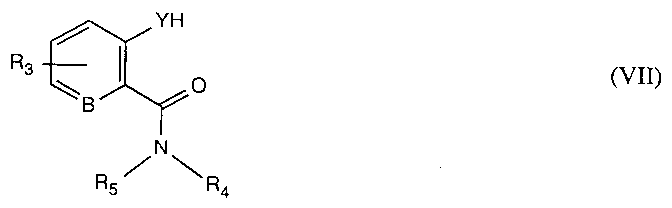 worin R₃, R₄, R₅, B und Y die unter Formel I in Anspruch 1 angegebene Bedeutung haben, umsetzt.

24. Verfahren nach Anspruch 23, dadurch gekennzeichnet, daß man die Umsetzung in Gegenwart eines inerten Verdünnungsmittels durchführt.

25. Verfahren nach Anspruch 23, dadurch gekennzeichnet, daß man die Umsetzung in Gegenwart einer Base durchführt.

26. Verfahren nach Anspruch 23, dadurch gekennzeichnet, daß man die Umsetzung in Gegenwart eines reaktionsbeschleunigenden Zusatzes durchführt.

27. Verfahren nach Anspruch 23, dadurch gekennzeichnet, daß man die Umsetzung bei einer Temperatur zwischen O°C und 160°C durchführt.

28. Pyrimidinyl- und Triazinyl-salicylanhydride der Formel II′ 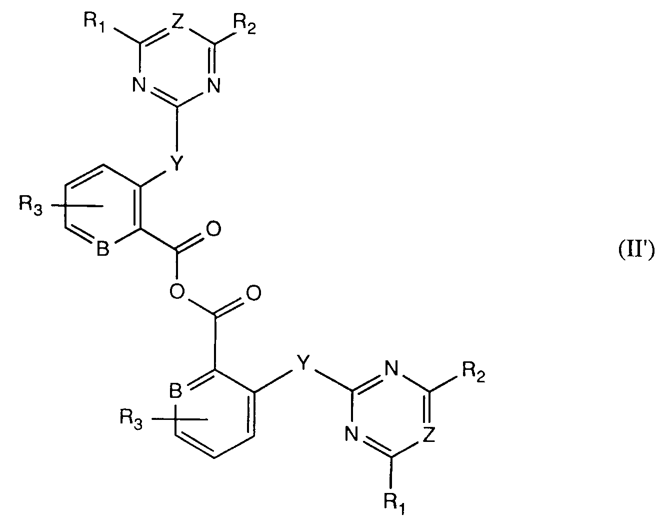 worin
B für Stickstoff, Methin, oder mit Fluor, Chlor, Methyl, Methoxy, 2-Propenyloxy, 2-Propinyloxy, Difluormethoxy oder Benzyloxy substituiertes Methin;
Y für Sauerstoff oder Schwefel;
Z für Methin oder Stickstoff;
R₁ für Chlor, Methyl, Methoxy, Ethoxy, Difluormethoxy, Methylamino, Ethylamino oder Dimethylamino;
R₂ für Methyl, Methoxy oder Difluormethoxy;
R₃ für Wasserstoff, Fluor, Chlor, Methyl oder Methoxy steht.

29. Verbindungen der Formel II′ gemäß Anspruch 28, dadurch gekennzeichnet, daß B Methin oder Stickstoff bedeutet.

30. Verbindungen der Formel II′gemäß Anspruch 28, dadurch gekennzeichnet, daß Y Sauerstoff bedeutet.

31. Verbindungen der Formel II′ gemäß Anspruch 28, dadurch gekennzeichnet, daß Z Methin bedeutet.

32. Verbindungen der Formel II′ gemäß Anspruch 28, dadurch gekennzeichnet, daß R₁ und R₂ Methoxy bedeuten.

33. Verbindungen der Formel II′ gemäß Anspruch 28, dadurch gekennzeichnet, daß R₃ Wasserstoff bedeutet.

34. Verfahren zur Herstellung von Verbindungen der Formel II′ gemäß Anspruch 28, dadurch gekennzeichnet, daß man eine Säure der Formel IV 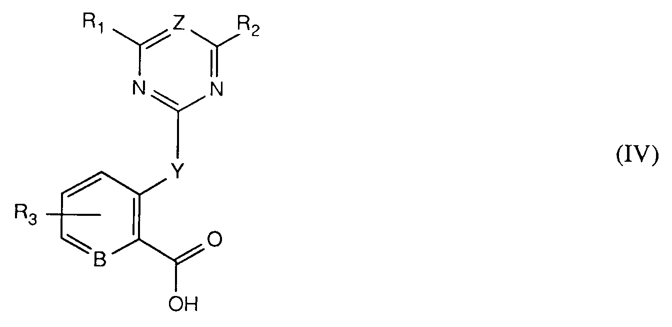 worin R₁, R₂, R₃, B und Y die unter Anspruch 28 angegebene Bedeutung haben, mit Phosphoroxychlorid und Triethylamin behandelt.

35. Verfahren nach Anspruch 34, dadurch gekennzeichnet, daß man pro Äquivalent der Säure der Formel IV 0,50 bis 0,55 Äquivalente Phosphoroxychlorid und 2,0 bis 3,0 Äquivalente Triethylamin einsetzt.

36. Ein herbizides und den Pflanzenwuchs hemmendes Mittel, gekennzeichnet durch ein Gehalt an einem oder mehreren Pyrimidinyl- und Triazinyl-salicylamide und -salicylanhydride der Formel I, gemäß Anspruch 1.

37. Mittel gemäß Anspruch 36, dadurch gekennzeichnet, daß es zwischen 0,001 % und 95 Gew.% Wirkstoff der Formel I gemäß Anspruch 1 enthält.

38. Verfahren zur Bekämpfung unerwünschten Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

39. Verfahren gemäß Anspruch 38, dadurch gekennzeichnet, daß man eine Wirkstoffmenge zwischen 0,001 und 3 kg pro Hektar appliziert.

40. Verfahren zur Regulierung des Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

41. Verfahren gemäß Anspruch 38, zur selektiven pre- oder post-emergenten Bekämpfung von Unkräutern in Nutzpflanzenkulturen.

42. Verwendung von Verbindungen der Formel I, gemäß Anspruch 1, als Wirkstoff in Unkrautbekämpfungsmitteln.

43. Verwendung von Verbindungen der Formel I, gemäß Anspruch 1, als Wirkstoff in pflanzenwachstumsregulierenden Mitteln.

## Patentansprüche (Patentansprüche für folgende(n) Vertragsstaat(en): ES)

1. Verfahren zur Herstellung von Pyrimidinyl- und Triazinyl-salicylamiden der Formel I 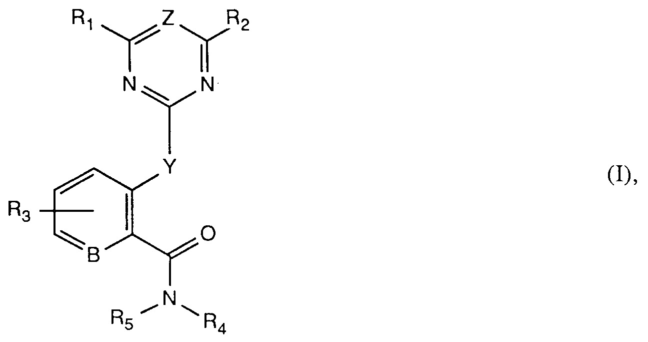 worin
B für Stickstoff, Methin, oder mit Fluor, Chlor, Methyl, Methoxy, 2-Propenyloxy, 2-Propinyloxy, Difluormethoxy, Phenoxy, Methylthio, Phenylthio, Phenyl oder Benzyloxy substituiertes Methin;
Y für Sauerstoff oder Schwefel;
Z für Methin oder Stickstoff;
R₁ für Chlor, Methyl, Methoxy, Ethoxy, Difluormethoxy, Methylamino, Ethylamino oder Dimethylamino;
R₂ für Methyl, Methoxy oder Difluormethoxy;
R₃ für Wasserstoff, Fluor, Chlor, Methyl oder Methoxy;
R₄ für eine der Gruppen (a), (b) oder (c) 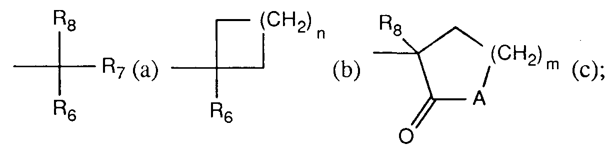 R₅ für Wasserstoff, Methyl oder zusammen mit R₇ -(CH₂)ₚ-, -CH₂SCH₂ oder -CH₂CHOHCH₂-;
R₆ für Hydroxymethyl, Formyl, Cyano, Hydroxyimino, C₁₋₄-Alkoxyimino, Phosphono, Phosphino, Methylphosphino oder eine Gruppe COX;
R₇ für Wasserstoff; C₁₋₄-Alkyl oder durch Hydroxy, C₁₋₄-Alkoxy, Mercapto, Acylmercapto, C₁₋₄-Alkylthio, Vinyl, Phenyl, 4-Hydroxyphenyl, 4-Imidazolyl, 3-Indolyl, Hydroxycarbonyl, C₁₋₄-Alkoxycarbonyl, 2-Propenyloxycarbonyl, Cyano, Carbamoyl, Methylphosphino oder Methylsulfoximino substituiertes C₁₋₄-Alkyl; Trifluormethyl; Ethinyl; Vinyl oder durch Chlor oder Methoxy substituiertes Vinyl; Phenyl oder durch Fluor, Chlor, Methyl, Trifluormethyl oder Methoxy substituiertes Phenyl; oder Cyano oder C₁₋₄-Alkoxycarbonyl;
R₈ für Wasserstoff oder Methyl;
A für Sauerstoff, Schwefel oder -NH-;
m für 1, 2 oder 3;
n für 0, 1, 2 oder 3;
p für 2 oder 3;
X für Hydroxy, C₁₋₄-Alkoxy, C₃₋₄-Alkenyloxy, Mercapto, C₁₋₄-Alkylthio, Amino, C₁₋₄-Alkylamino, C₂₋₄Dialkylamino, C₁₋₄-Alkoxyamino, oder durch Phenyl, Benzyloxy oder C₁₋₂-Alkoxy substituiertes C₁₋₄-Alkoxy, C₃₋₄-Alkenyloxy, Mercapto, C₁₋₄-Alkylthio, Amino, C₁₋₄-Alkylamino, C₂₋₄Dialkylamino, C₁₋₄-Alkoxyamino, oder eine der Gruppen (d), (e) oder f) 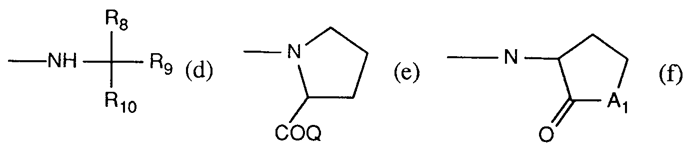 A₁ für Sauerstoff, Schwefel oder -NH-;
R₉ für Wasserstoff, C₁₋₄-Alkyl oder Benzyl;
R₁₀ für Hydroxymethyl, Cyano oder eine Gruppe COQ′;
Q für Hydroxy, C₁₋₄-Alkoxy, 2-Propenyloxy, Benzyloxy, Amino oder die Gruppe d); und
Q′ für Hydroxy, C₁₋₄-Alkoxy, 2-Propenyloxy, Benzyloxy, Amino oder die Gruppe d) stehen; sowie die Salze derjenigen Verbindungen der Formel I, welche eine freie Hydroxycarbonylgruppe aufweisen; dadurch gekennzeichnet, daß man eine Verbindung der Formel II 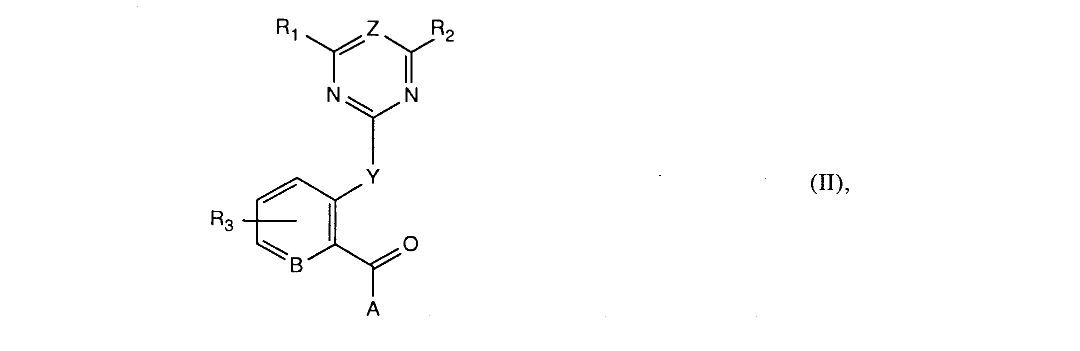 worin A eine Abgangsgruppe bedeutet;
oder ein Säureanhydrid der Formel II′ 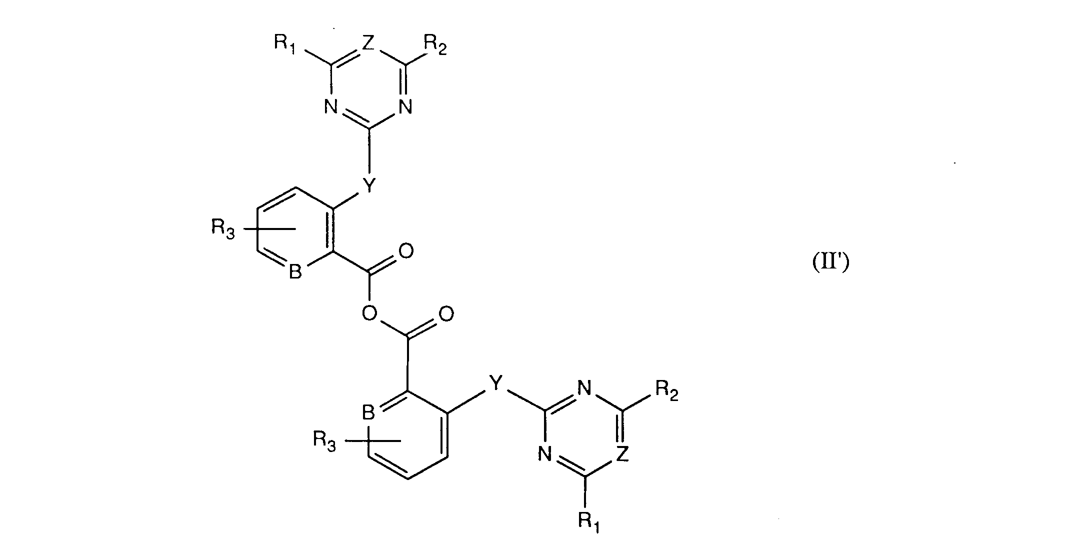 worin R₁, R₂, R₃, Y, Z und B die oben angegebene Bedeutung haben, mit einem Amin der Formel III 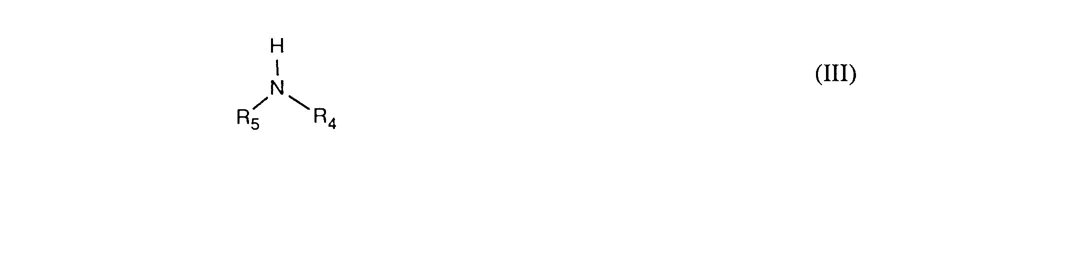 worin R₄ und R₅ die oben angegebene Bedeutung haben, umsetzt.

2. Verfahren nach Anspruch 1 zur Herstellung von Verbindungen der Formel I, worin
B für Stickstoff, Methin, oder mit Fluor, Chlor, Methyl, Methoxy, 2-Propenyloxy, 2-Propinyloxy, Difluormethoxy oder Benzyloxy substituiertes Methin;
Y für Sauerstoff oder Schwefel;
Z für Methin oder Stickstoff;
R₁ für Chlor, Methyl, Methoxy, Ethoxy, Difluormethoxy, Methylalmino, Ethylamino oder Dimethylamino;
R₂ für Methyl, Methoxy oder Difluormethoxy;
R₃ für Wasserstoff, Fluor, Chlor, Methyl oder Methoxy;
R₄ für eine der Gruppen (a), (b) oder (c) 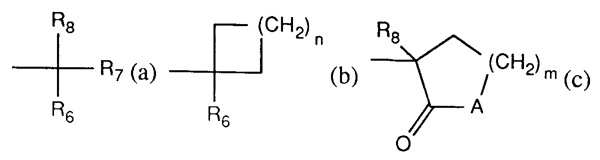 R₅ für Wasserstoff, Methyl oder zusammen mit R₇ -(CH₂)ₚ- oder -CH₂CHOHCH₂-;
R₆ für Hydroxymethyl, Cyano, Phosphono, Phosphino, Methylphosphino oder eine Gruppe COX;
R₇ für Wasserstoff; gegebenenfalls mit Hydroxy, C₁₋₄-Alkoxy, Mercapto, Acylmercapto, C₁₋₄-Alkylthio, Phenyl, 4-Hydroxyphenyl, 4-Imidazolyl, 3-Indolyl, Hydroxycarbonyl, C₁₋₄-Alkoxycarbonyl, 2-Propenyloxycarbonyl, Cyano, Carbamoyl, Methylphosphino oder Methylsulfoximino substituiertes C₁₋₄-Alkyl; Trifluormethyl; Ethinyl; Vinyl oder durch Chlor oder Methoxy substituiertes Vinyl; Phenyl oder durch Fluor, Chlor, Methyl, Trifluormethyl oder Methoxy substituiertes Phenyl;
R₈ für Wasserstoff oder Methyl;
A für Sauerstoff, Schwefel oder -NH-;
m für 1, 2 oder 3;
n für 0, 1, 2 oder 3;
p für 2 oder 3;
X für Hydroxy, C₁₋₄-Alkoxy, C₃₋₄-Alkenyloxy, Mercapto, C₁₋₄-Alkylthio, Amino, C₁₋₄-Alkylamino, C₂₋₄Dialkylamino, oder eine weitere der Gruppen (d) oder (e) 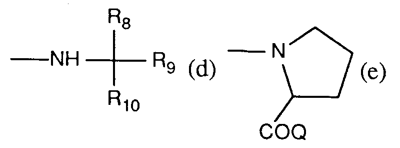 R₉ für Wasserstoff, C₁₋₄-Alkyl oder Benzyl;
R₁₀ für COQ′;
Q für Hydroxy, C₁₋₄-Alkoxy, 2-Propenyloxy, Benzyloxy oder Amino; Q′ für Hydroxy, C₁₋₄-Alkoxy, 2-Propenyloxy, Benzyloxy oder Amino; sowie die Salze von Verbindungen der Formel I, welche eine freie Hydroxycarbonylgruppe aufweisen.

3. Verfahren nach Anspruch 1 zur Herstellung von Verbindungen der Formel I, worin B Methin oder Stickstoff bedeutet.

4. Verfahren nach Anspruch 3 zur Herstellung von Verbindungen der Formel I, worin Methin durch Fluor oder Chlor substituiert ist.

5. Verfahren nach Anspruch 1 zur Herstellung von Verbindungen der Formel I, worin Y Sauerstoff bedeutet.

6. Verfahren nach Anspruch 1 zur Herstellung von Verbindungen der Formel I, worin Z Methin bedeutet.

7. Verfahren nach Anspruch 1 zur Herstellung von Verbindungen der Formel I, worin R₁ und R₂ Methoxy bedeuten.

8. Verfahren nach Anspruch 1 zur Herstellung von Verbindungen der Formel I, worin R₃ Wasserstoff bedeutet.

9. Verfahren nach Anspruch 1 zur Herstellung von Verbindungen der Formel I, worin R₄ eine Gruppe (a) bedeutet.

10. Verfahren nach Anspruch 1 zur Herstellung von Verbindungen der Formel I, worin R₅ Wasserstoff bedeutet.

11. Verfahren nach Anspruch 1 zur Herstellung von Verbindungen der Formel I, worin R₆ Cyano oder eine COX-Gruppe bedeutet.

12. Verfahren nach Anspruch 11 zur Herstellung von Verbindungen der Formel I, worin X C₁₋₄-Alkoxy oder Amino bedeutet.

13. Verfahren nach Anspruch 1 zur Herstellung von Verbindungen der Formel I, worin R₇ Wasserstoff, C₁₋₄-Alkyl, 2-Propenyl oder zusammen mit R₅ -CH₂CH₂CH₂- oder -CH₂SCH₂- bedeutet.

14. Verfahren nach Anspruch 1 zur Herstellung von Verbindungen der Formel I, worin R₈ Wasserstoff bedeutet.

15. Verfahren nach Anspruch 1 zur Herstellung von Verbindungen der Formel I, worin R₄ eine chirale Gruppe der Formel IA 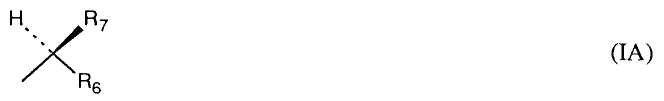 mit der Chiralität <S > bedeutet.

16. Verfahren nach Anspruch 1 zur Herstellung von Verbindungen der Formel I, worin R₄ für die Gruppe (c) steht, worin A Sauerstoff oder Schwefel und m = 1 bedeutet.

17. Verfahren nach Anspruch 16 zur Herstellung von Verbindungen der Formel I, worin R₄ eine Gruppe der Formel IF 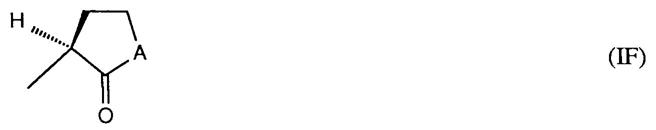 mit der Chiralität <S > bedeutet.

18. Verfahren nach Anspruch 1 zur Herstellung von Verbindungen der Formel I, worin
R¹ für Methoxy, Ethoxy, oder Difluormethoxy;
R² für Methoxy;
R³ für Wasserstoff;
B für Stickstoff, Methin, oder durch Chlor oder Fluor substituiertes Methin;
Y für Sauerstoff oder Schwefel;
Z für Methin; und
NR⁴R⁵ eine der Gruppen 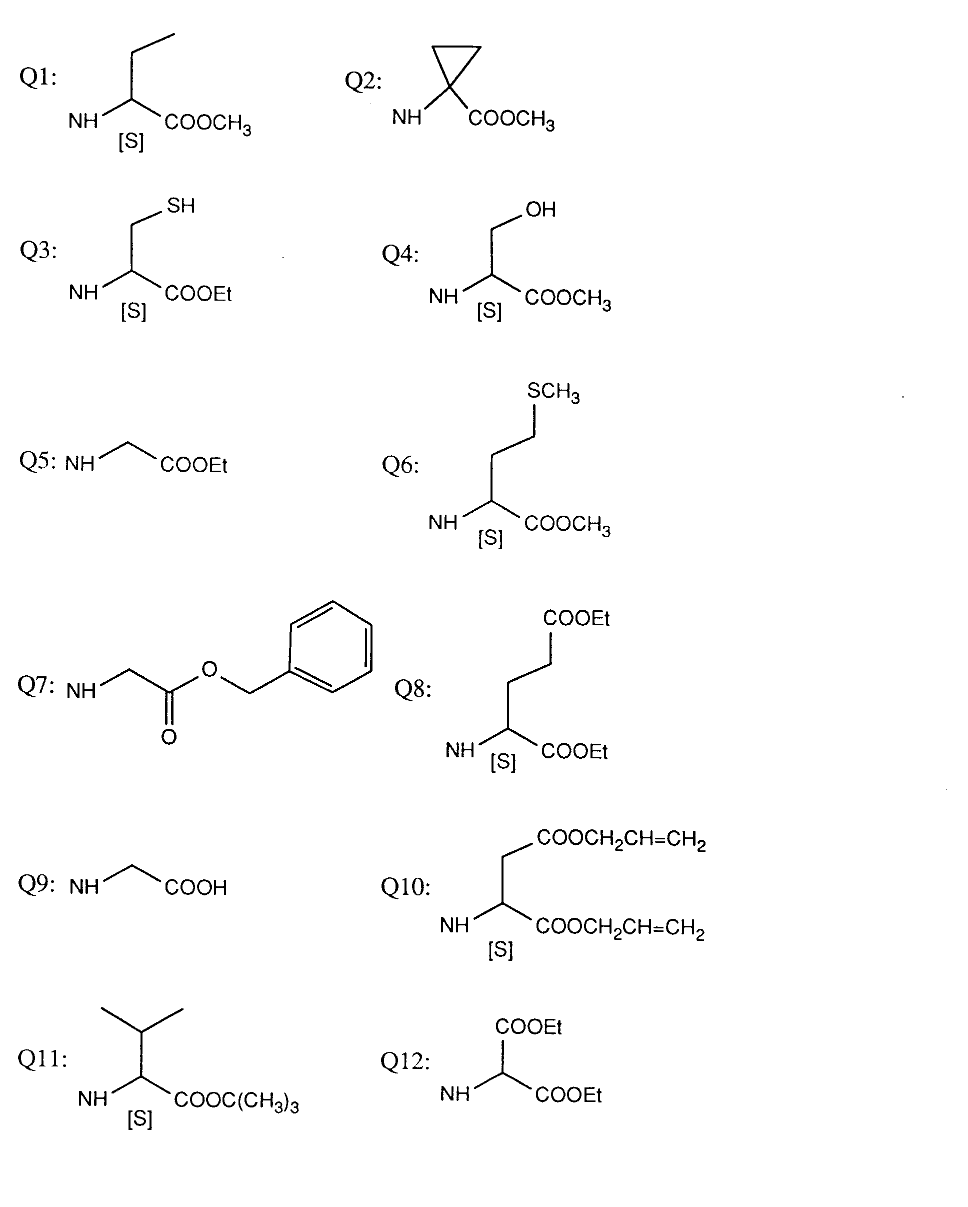 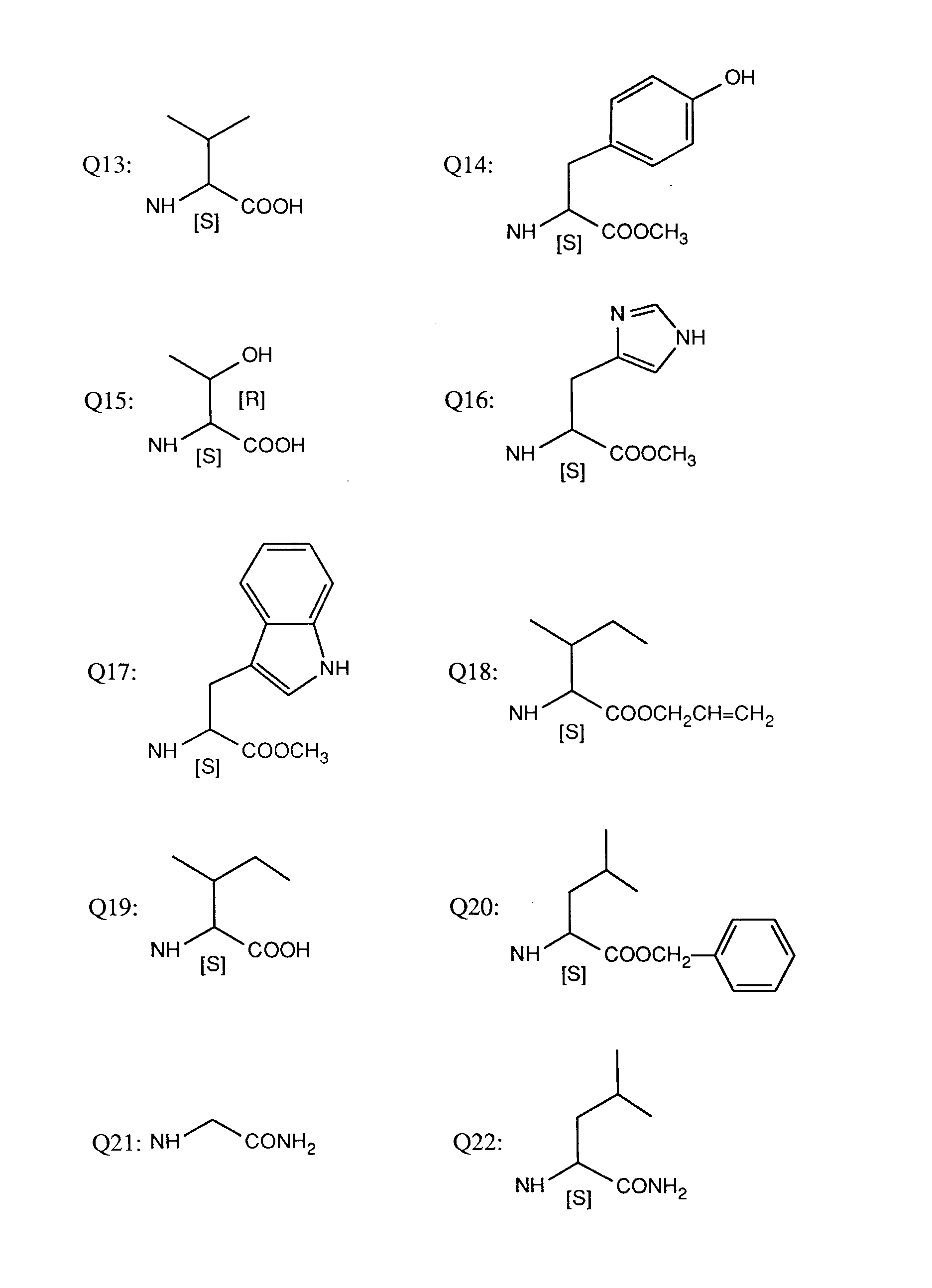 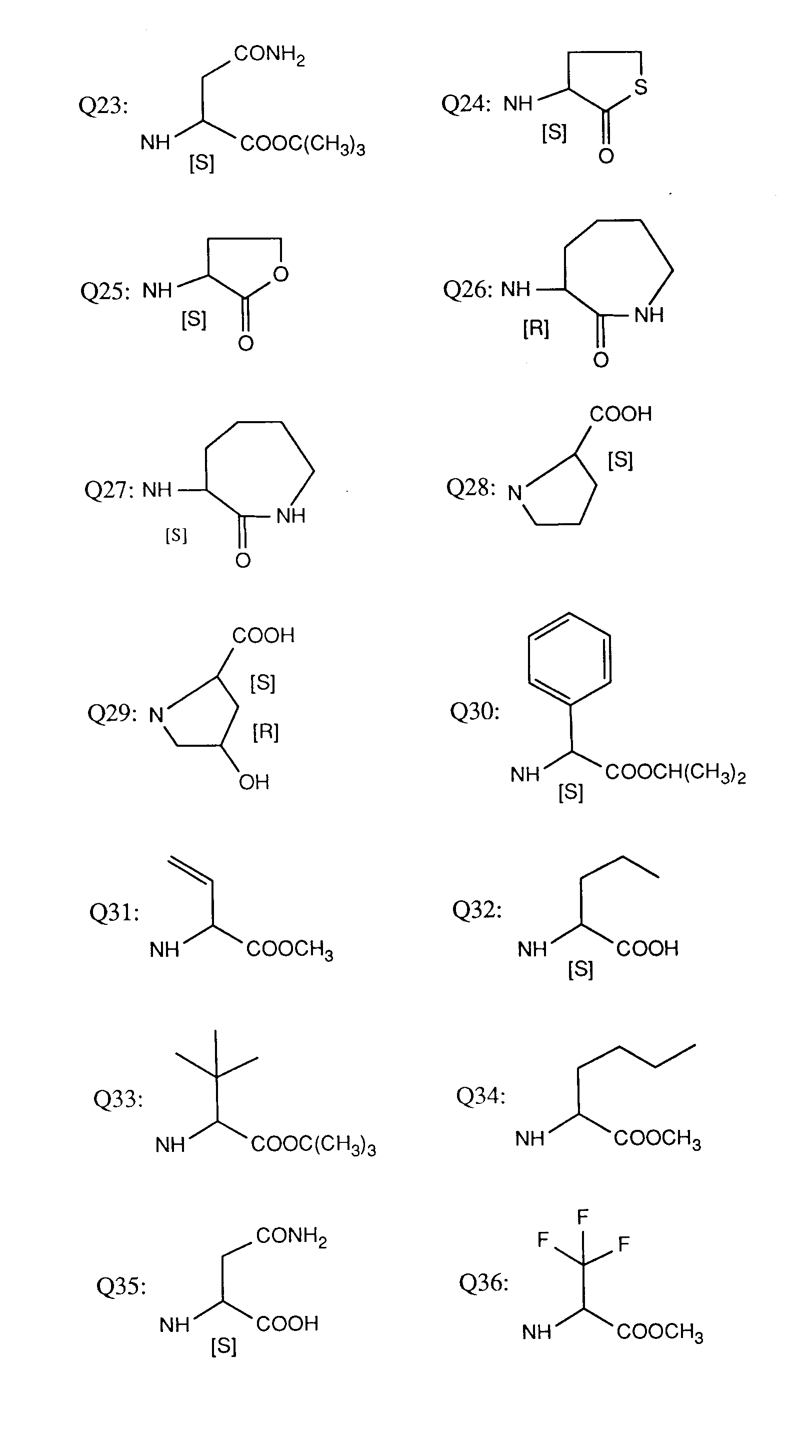 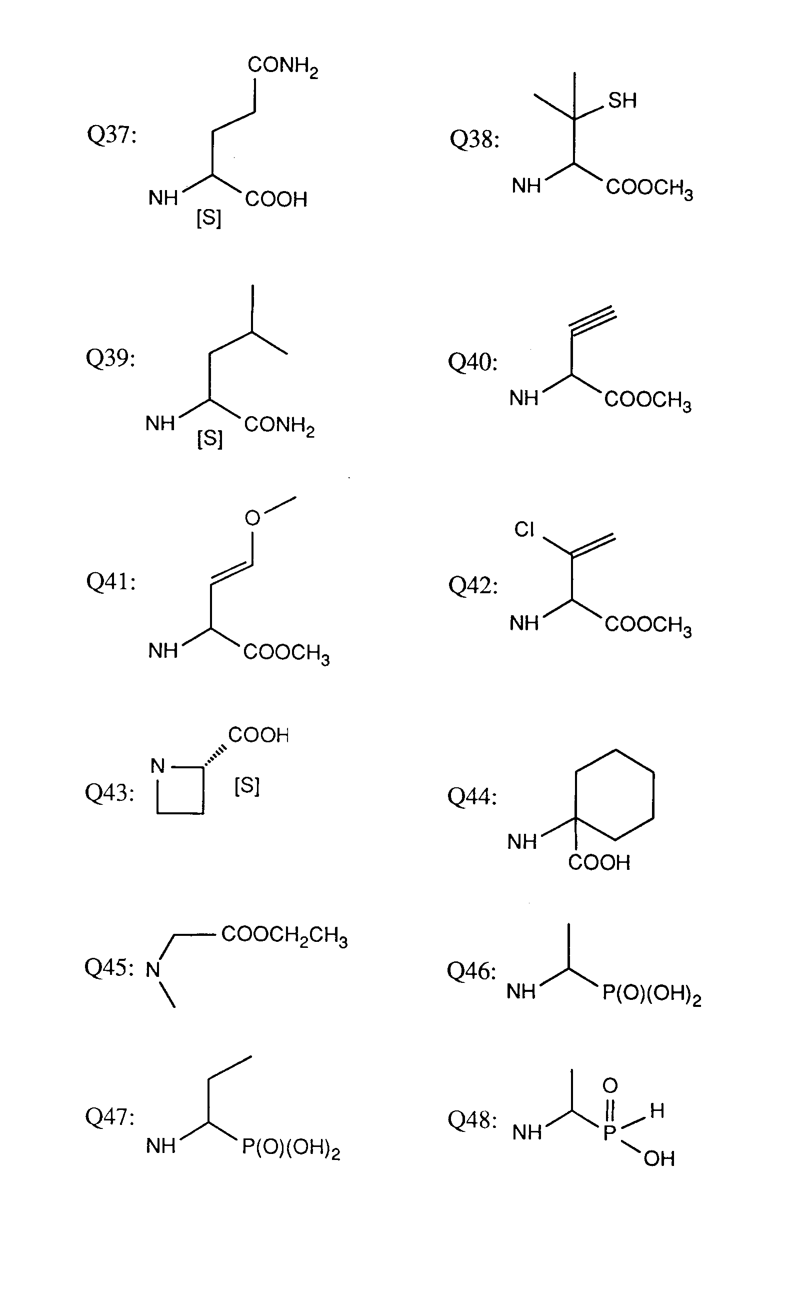 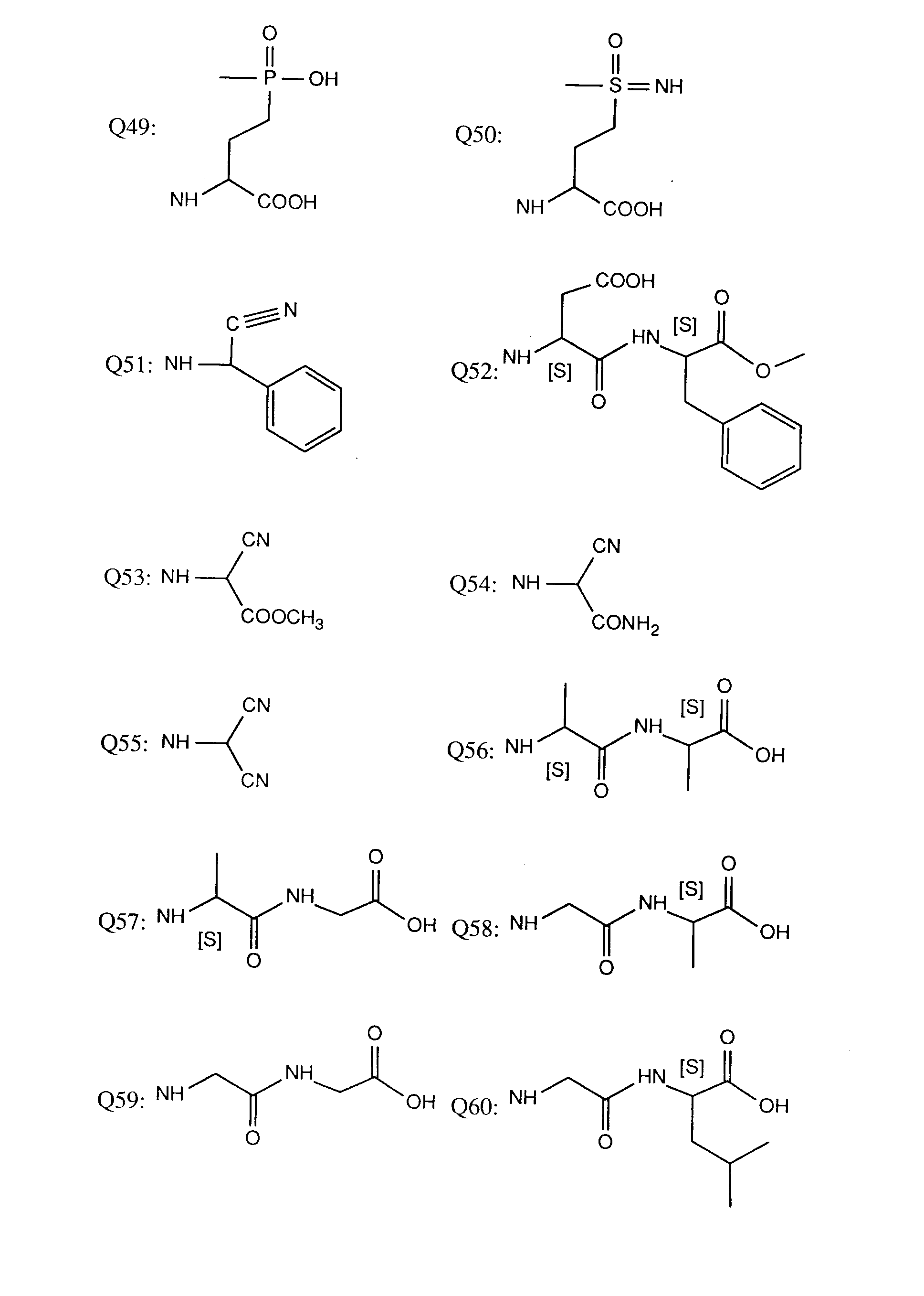 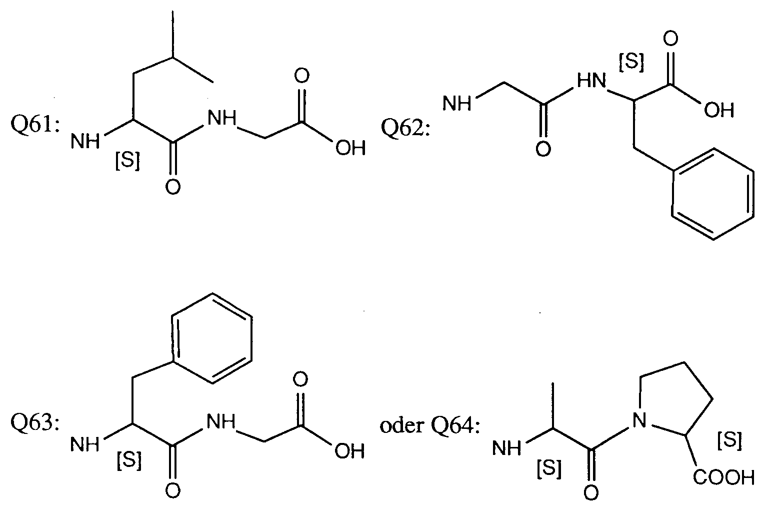 bedeuten.

19. Verfahren nach Ansprurh 1 zur Herstellung von Verbindungen der Formel I ausgewählt aus der Gruppe
N-(o-((4,6-Dimethoxy-2-pyrimidinyl)oxy)benzoyl)glycinmethyles ter;
N-(o-((4,6-Dimethoxy-2-pyrimidinyl)oxy)benzoyl)-L-alaninethylester;
rac-o-((4,6-Dimethoxy-2-pyrimidinyl)oxy)-N-(dihydro-2-oxo-3(2H)-thienyl)benzamid;
N-(o-((4,6-Dimethoxy-2-pyrimidinyl)oxy)benzoyl)-L-alanin-methylester;
N-(o-((4,6-Dimethoxy-2-pyrimidinyl)oxy)benzoyl)-L-valinmethylester;
N-(o-((4,6-Dimethoxy-2-pyrimidinyl)oxy)benzoyl)-L-leucinmethylester;
rac-N-(1-Carbamoyl-1,2-dimethylpropyl)-o-((4,6-dimethoxy-2-pyrimidinyl)-thio)-benzamid;
1-(o-((4,6-Dimethoxy-2-pyrimidinyl)oxy)benzoyl)-L-prolin-tert-butylester;
N-((3-((4,6-Dimethoxy-2-pyrimidinyl)oxy)-2-pyridyl)-carbonyl)-L-alaninmethylester;
N-((3-((4,6-Dimethoxy-2-pyrimidinyl)oxy)-2-pyridyl)-carbonyl)-glycinmethylester;
o-((4,6-Dimethoxy-2-pyrimidinyl)oxy)-N-(<S>-1-(hydroxymethyl)2-methylpropyl)-benzamid; und
N-(1-Cyano-1-methylethyl)-o-((4,6-dimethoxy-2-pyrimidinyl)oxy)benzamid.

20. Verfahren nach Anspruch 1, dadurch gekennzeichnet, daß man die Umsetzung in Gegenwart einer Base durchführt.

21. Verfahren nach Anspruch 1, dadurch gekennzeichnet, daß man die Umsetzung in Gegenwart eines Lösungsmittels durchführt.

22. Verfahren zur Herstellung von Verbindungen der Formel I gemäß Anspruch 1, dadurch gekennzeichnet, daß man eine Verbindung der Formel V 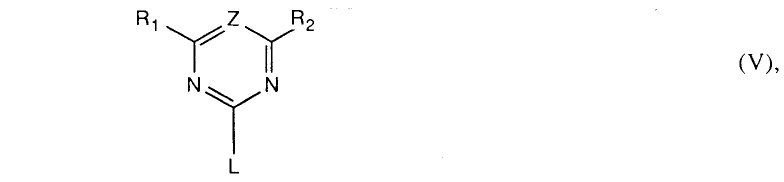 worin
R₁ und R₂ die unter Formel I in Anspruch 1 angegebene Bedeutung haben und L eine Abgangsgruppe bedeutet,
mit einem Salicylamidderivat der Formel VII 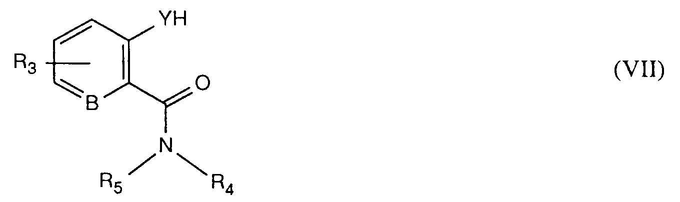 worin R₃, R₄, R₅, B und Y die unter Formel I in Anspruch 1 angegebene Bedeutung haben, umsetzt.

23. Verfahren nach Anspruch 22, dadurch gekennzeichnet, daß man die Umsetzung in Gegenwart eines inerten Verdünnungsmittels durchführt.

24. Verfahren nach Anspruch 22, dadurch gekennzeichnet, daß man die Umsetzung in Gegenwart einer Base durchführt.

25. Verfahren nach Anspruch 22, dadurch gekennzeichnet, daß man die Umsetzung in Gegenwart eines reaktionsbeschleunigenden Zusatzes durchführt.

26. Verfahren nach Anspruch 22, dadurch gekennzeichnet, daß man die Umsetzung bei einer Temperatur zwischen O°C und 160°C durchfürht.

27. Verfahren zur Herstellung von Pyrimidinyl- und Triazinyl-salicylanhydriden der Formel II′ 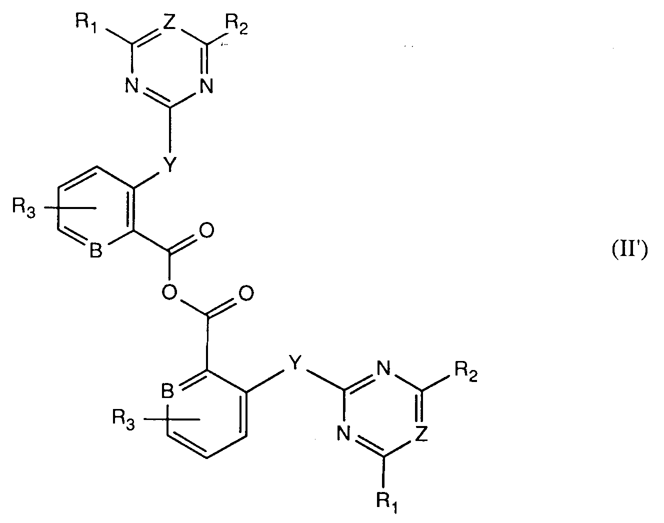 worin
B für Stickstoff, Methin, oder mit Fluor, Chlor, Methyl, Methoxy, 2-Propenyloxy, 2-Propinyloxy, Difluormethoxy oder Benzyloxy substituiertes Methin;
Y für Sauerstoff oder Schwefel;
Z für Methin oder Stickstoff;
R₁ für Chlor, Methyl, Methoxy, Ethoxy, Difluormethoxy, Methylamino, Ethylamino oder Dimethylamino;
R₂ für Methyl, Methoxy oder Difluormethoxy;
R₃ für Wasserstoff, Fluor, Chlor, Methyl oder Methoxy steht, dadurch gekennzeichnet, daß man eine Säure der Formel IV 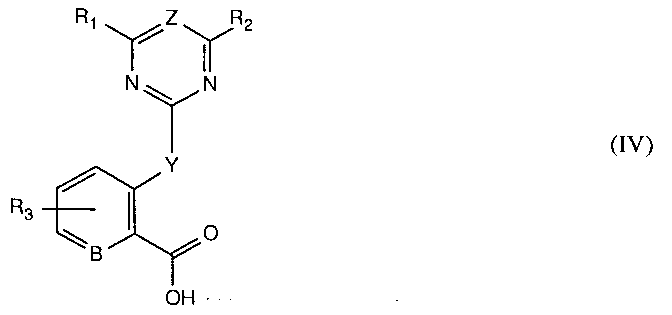 worin R₁, R₂, R₃, B und Y die oben angegebene Bedeutung haben, mit Phosphoroxychlorid und Triethylamin behandelt..

28. Verfahren nach Anspruch 27 zur Herstellung von Verbindungen der formel II′, worin B Methin oder Stickstoff bedeutet.

29. Verfahren nach Anspruch 27 zur Herstellung von Verbindungen der Formel II′, worin Y Sauerstoff bedeutet.

30. Verfahren nach Anspruch 27 zur Herstellung von Verbindungen der Formel II′, worin Z Methin bedeutet.

31. Verfahren nach Anspruch 27 zur Herstellung von Verbindungen der Formel II′, worin R₁ und R₂ Methoxy bedeuten.

32. Verfahren nach Anspruch 27 zur Herstellung von Verbindungen der Formel II′, worin R₃ Wasserstoff bedeutet.

33. Verfahren nach Anspruch 28, dadurch gekennzeichnet, daß man pro Äquivalent der Säure der Formel IV 0,50 bis 0,55 Äquivalente Phosphoroxychlorid und 2,0 bis 3,0 Äquivalente Triethylamin einsetzt.

34. Ein herbizides und den Pflanzenwuchs hemmendes Mittel, gekennzeichnet durch ein Gehalt an einem oder mehreren Pyrimidinyl- und Triazinyl-salicylamide und -salicylanhydride der Formel I, gemäß Anspruch 1.

35. Mittel gemäß Anspruch 36, dadurch gekennzeichnet, daß es zwischen 0,001 % und 95 Gew.% Wirkstoff der Formel I gemäß Anspruch 1 enthält.

36. Verfahren zur Bekämpfung unerwünschten Pflanzenwachstums, dadurch gekennzeichnet, daß man einen Wirkstoff der formel I, gemäß Anspruch 1, oder ein diesen Wirkstoff enthaltendes Mittel in einer wirksamen Menge auf die Pflanzen oder deren Lebensraum appliziert.

37. Verfahren gemäß Anspruch 36, dadurch gekennzeichnet, daß man eine Wirkstoffmenge zwischen 0,001 und 3 kg pro Hektar appliziert.

38. Verfahren zur Regulierung des Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

39. Verfahren gemäß Anspruch 36, zur selektiven pre- oder post-emergenten Bekämpfung von Unkräutern in Nutzpflanzenkulturen.

40. Verwendung von Verbindungen der Formel I, gemäß Anspruch 1, als Wirkstoff in Unkrautbekämpfungsmitteln.

41. Verwendung von Verbindungen der Formel I, gemäß Anspruch 1, als Wirkstoff in pflanzenwachstumsregulierenden Mitteln.
